# EUROPÄISCHE PATENTSCHRIFT

(11) **EP 2 178 828 B1**
(45) Veröffentlichungstag und Bekanntmachung des Hinweises auf die Patenterteilung: **26.06.2019**
(21) Anmeldenummer: 08786166.2
(22) Anmeldetag: 16.07.2008
(51) Int. Cl.: C07D 295/108, C07D 295/13, C08G 18/80, C08G 18/12, C09D 175/04, C08G 18/32, C08G 18/48, C09J 175/04, C07C 251/08, C08G 18/66

(54) **ALDIMINE UND ALDIMIN ENTHALTENDE ZUSAMMENSETZUNGEN**
ALDIMINES AND COMPOUNDS CONTAINING ALDIMINE
ALDIMINE ET ALDIMINE CONTENANT DES COMPOSITIONS

(30) Priorität: 16.07.2007 EP 07112503
(43) Veröffentlichungstag der Anmeldung: 28.04.2010
(73) Patentinhaber: Sika Technology AG, 6340 Baar (CH)
(72) Erfinder: BURCKHARDT, Urs, CH-8049 Zürich (CH)
(74) Vertreter: Sika Patent Attorneys
(86) Internationale Anmeldenummer: PCT/EP2008/059267
(87) Internationale Veröffentlichungsnummer: WO 2009/010522

(56) Entgegenhaltungen:
- EP-A- 1 384 709
- EP-A- 1 772 447
- DE-A1- 4 212 795
- ERIC C. BROWN ET AL: "Modular syntheses of multidentate ligands with variable N-donors: applications to tri- and tetracopper(I) complexes" DALTON TRANS., 8. Juni 2007 (2007-06-08), Seiten 3035-3042, XP002464027

## Beschreibung

### Technisches Gebiet

Die Erfindung betrifft das Gebiet der Aldimine.

### Stand der Technik

Aldimine sind Kondensationsprodukte aus primären Aminen und Aldehyden und stellen eine seit langem bekannte Stoffklasse dar. Bei Kontakt mit Wasser können Aldimine zu den entsprechenden Aminen und Aldehyden hydrolysieren. Aufgrund dieser Eigenart können sie als geschützte Form von Aminen, beziehungsweise von Aldehyden, verwendet werden. So werden Aldimine beispielsweise in der Polyurethanchemie eingesetzt, wo sie als durch Feuchtigkeit aktivierbare Vernetzer, sogenannte "blockierte Amine" oder "latente Härter", für ein- oder zweikomponentige Isocyanatgruppen aufweisende Zusammensetzungen dienen.

Die Vorteile der Verwendung von Aldiminen als latente Härter in Isocyanatgruppen aufweisenden Systemen liegen insbesondere darin, dass sich die Entstehung von unerwünschten Gasblasen vermeiden lässt, da die Aushärtungsreaktion über das blockierte Amin - im Gegensatz zur direkten Reaktion von Isocyanaten mit Feuchtigkeit - nicht unter Freisetzung von Kohlendioxid (CO₂) verläuft, und dass sich höhere Aushärtungsgeschwindigkeiten und/oder längere Offenzeiten erzielen lassen. Der Einsatz von Aldiminen als latente Härter in Isocyanatgruppen aufweisenden Zusammensetzungen kann aber auch Probleme verursachen. Bei einkomponentigen Zusammensetzungen kann die Lagerstabilität durch die Anwesenheit des Aldimins stark eingeschränkt sein. In Abhängigkeit der zur Herstellung des Aldimins verwendeten und bei der Aushärtungsreaktion wieder freigesetzten Aldehyde können die Zusammensetzungen zudem einen sehr starken Geruch aufweisen, welcher für viele Anwendungen nicht tolerierbar ist.

WO 2004/013088 A1 beschreibt geruchsfreie Polyaldimine, welche aus primären Polyaminen und geruchsfreien Aldehyden hergestellt werden. WO 2007/036571 A1 beschreibt geruchsfreie Aldimine enthaltend mindestens eine Hydroxyl-, Mercapto- oder sekundäre Aminogruppe, welche ebenfalls ausgehend von geruchsfreien Aldehyden erhältlich sind. In EP 1 384 709 A1 werden Polyaldimine beschrieben welche Verwendung finden in Zusammensetzungen als Klebstoff, Dichtstoff, Beschichtung oder Belag. Dokument EP 1 772 447 A1 offenbart Aldimine mit aktivem Wasserstoff und deren Verwendung als geschützter Vernetzer für einen Kunststoffvorläufer.

Diese geruchsfreien Aldehyde können in Polymerzusammensetzungen, insbesondere in Polyurethanzusammensetzungen, eine stark weichmachende Wirkung haben, was unerwünscht sein kann. Das relativ hohe Molekulargewicht der Aldehyde führt überdies dazu, dass die daraus hergestellten Aldimine als latente Härter in relativ grosser Menge eingesetzt werden müssen, was ihren Einsatz teuer machen kann.

Aus Eric C. Brown et al: "Modular syntheses of multidentate ligands with variable N-donors: applications to tri-and tetracopper(I) complexes" (Dalton Transactions, 8 June 2007, pages 3035 to 3042) sowie aus DE 4212795 A1 sind Aldimine bekannt.

### Darstellung der Erfindung

Aufgabe der vorliegenden Erfindung ist es, neue Aldimine zur Verfügung zu stellen, welche als latente Härter in härtbaren Zusammensetzungen, insbesondere in ein- oder zweikomponentigen Isocyanatgruppen aufweisenden Zusammensetzungen, eingesetzt werden können.

Überraschenderweise wurde gefunden, dass Aldimine nach Anspruch 1 diese Aufgabe lösen und vorteilhafte Eigenschaften aufweisen. Es handelt sich dabei um bei Raumtemperatur meist flüssige Verbindungen, welche kaum Aldehyd-Geruch aufweisen und aus gut erhältlichen Grundstoffen in einem einfachen Verfahren aus primären Aminen und sterisch gehinderten, eine tertiäre Aminogruppe aufweisenden, aliphatischen Aldehyden herstellbar sind. Die Aldimine besitzen eine gute thermische Stabilität. Ihre tertiäre Aminogruppe weist eine überraschend niedrige Basizität auf und kann unter Umständen in chemischen Reaktionssystemen eine katalytische Wirkung entfalten.

Diese Aldimine sind beispielsweise geeignet als latente Härter für härtbare Zusammensetzungen, welche gegenüber Aminen reaktive Gruppen, wie Epoxidgruppen, Anhydridgruppen und insbesondere Isocyanatgruppen, aufweisen. Insbesondere in Polyurethanzusammensetzungen sind sie sehr gut verträglich und weisen wenig weichmachende Wirkung auf.

Ein weiterer Gegenstand der Erfindung sind einerseits Aldimine gemäss Anspruch 4 und andererseits Aldiminogruppen-haltige Verbindungen gemäss Anspruch 6, welche Umsetzungsprodukte der Aldimine darstellen.

Ein weiterer Gegenstand der Erfindung sind härtbare Zusammensetzungen enthaltend die beschriebenen Aldimine und/oder Aldiminogruppen-haltigen Verbindungen nach Anspruch 8.

Schliesslich bilden ein Verfahren zur Herstellung der Aldimine gemäss Anspruch 5, eine ausgehärtete Zusammensetzung gemäss Anspruch 11, und Verwendungen gemäss Anspruch 12 und 13 sowie ein Artikel gemäss Anspruch 14 weitere Gegenstände der vorliegenden Erfindung.

Weitere Aspekte der Erfindung sind Gegenstand weiterer unabhängiger Ansprüche. Besonders bevorzugte Ausführungsformen der Erfindung sind Gegenstand der abhängigen Ansprüche

### Wege zur Ausführung der Erfindung

Gegenstand der Erfindung sind Aldimine der Formel (I), 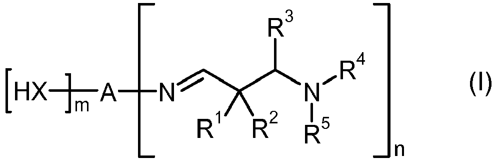 wobei
A entweder für den Rest eines Amins nach Entfernung von n primären aliphatischen Aminogruppen und m HX-Gruppen steht,
oder zusammen mit R⁷ für einen (n+2)-wertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
n für 1 oder 2 oder 3 oder 4, bevorzugt 1 oder 2, steht und
m für 0 oder 1 oder 2 oder 3 oder 4 steht,
mit der Massgabe, dass m+n für 2 oder 3 oder 4 oder 5 steht;
R¹ und R² entweder
   unabhängig voneinander jeweils für einen einwertigen Kohlenwasserstoffrest mit 1 bis 12 C-Atomen stehen,
   oder zusammen für einen zweiwertigen Kohlenwasserstoffrest mit 4 bis 12 C-Atomen, der Teil eines, gegebenenfalls substituierten, carbocyclischen Rings mit 5 bis 8, bevorzugt 6, C-Atomen ist, stehen;
R³ für ein Wasserstoffatom oder eine Alkylgruppe oder eine Arylalkylgruppe oder eine Alkoxycarbonylgruppe, insbesondere mit 1 bis 12 C-Atomen, steht;
R⁴ und R⁵ entweder
   unabhängig voneinander jeweils für einen einwertigen aliphatischen, cycloaliphatischen oder arylaliphatischen Rest mit 1 bis 20 C-Atomen stehen, welcher frei ist von Hydroxylgruppen, und welcher gegebenenfalls Heteroatome in der Form von Ether-Sauerstoff oder tertiärem Amin-Stickstoff enthält,
   oder zusammen für einen zweiwertigen aliphatischen Rest mit 3 bis 20 C-Atomen, der Teil eines, gegebenenfalls substituierten, heterocyclischen Rings mit 5 bis 8, bevorzugt 6, Ringatomen ist, wobei dieser Ring frei ist von Hydroxylgruppen und neben dem Stickstoffatom gegebenenfalls weitere Heteroatome in der Form von Ether-Sauerstoff oder tertiärem Amin-Stickstoff enthält, stehen;
X für O oder S oder N-R⁶ oder N-R⁷ steht,
   wobei R⁶
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (II) steht, 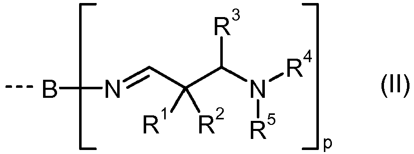 wobei
      p für 0 oder für eine ganze Zahl von 1 bis 10'000 steht, und
      B für einen (p+1)-wertigen Kohlenwasserstoffrest, welcher gegebenenfalls Ether-Sauerstoff, tertiären Amin-Stickstoff,
      Hydroxylgruppen, sekundäre Aminogruppen oder Mercaptogruppen enthält, steht; und
   R⁷ zusammen mit A für einen (n+2)-wertigen Kohlenwasserstoffrest mit 3 bis 20 C-Atomen, welcher gegebenenfalls mindestens ein Heteroatom, insbesondere in Form von Ether-Sauerstoff oder tertiärem Amin-Stickstoff, enthält, steht;
   wobei das Aldimin der Formel (I) nicht für eine der folgenden Verbindungen steht: 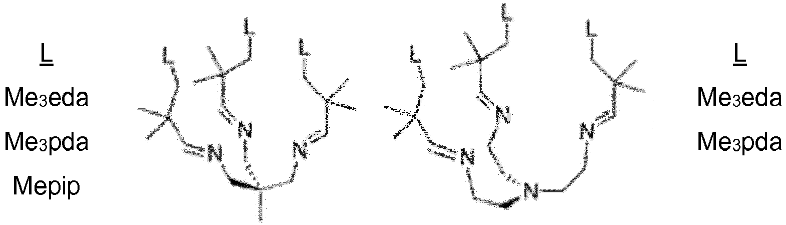 und L-H = 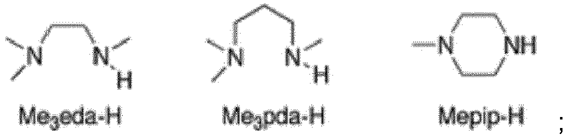 sowie

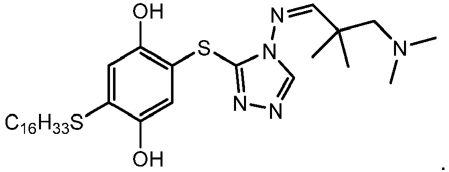

Die gestrichelten Linien in den Formeln in diesem Dokument stellen jeweils die Bindung zwischen einem Substituenten und dem zugehörigen Molekülrest dar.

Der Begriff "primäre Aminogruppe" bezeichnet im vorliegenden Dokument eine Aminogruppe in der Form einer NH₂-Gruppe, die an einen organischen Rest gebunden ist. Der Begriff "sekundäre Aminogruppe" bezeichnet eine Aminogruppe, in der das Stickstoffatom an zwei organische Reste gebunden ist, welche auch gemeinsam Teil eines Rings sein können. Der Begriff "tertiäre Aminogruppe" bezeichnet eine Aminogruppe, in der das Stickstoffatom an drei organische Reste gebunden ist, wobei zwei dieser Reste auch gemeinsam Teil eines Rings sein können (=tertiärer Amin-Stickstoff).

Als "aliphatisch" wird ein Amin oder eine Aminogruppe bezeichnet, worin das Stickstoffatom ausschliesslich an aliphatische, cycloaliphatische oder arylaliphatische Reste gebunden ist.

Der Begriff "aktiver Wasserstoff" bezeichnet im vorliegenden Dokument das Wasserstoffatom einer Hydroxyl-, einer Mercapto- oder einer sekundären Aminogruppe.

Bevorzugt stehen R¹ und R² in Formel (I) jeweils für eine Methylgruppe.

Bevorzugt steht R³ in Formel (I) für ein Wasserstoffatom.

Bevorzugt weisen R⁴ und R⁵ keinen tertiären Amin-Stickstoff auf.

In einer Ausführungsform der Aldimine der Formel (I) steht der Index m für 1 oder für 2 oder für 3 oder für 4, bevorzugt für 1. Solche Aldimine weisen also mindestens einen aktiven Wasserstoff auf.

Besonders bevorzugte Aldimine der Formel (I) mit mindestens einem aktiven Wasserstoff stellen Aldimine der Formel (Ia) dar, 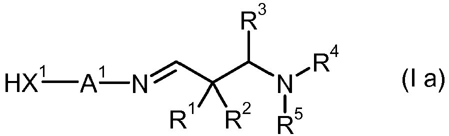 wobei
A¹ keinen aktiven Wasserstoff und keine primären Aminogruppen aufweist und entweder
   für einen zweiwertigen Kohlenwasserstoffrest mit 2 bis 20 C-Atomen, welcher gegebenenfalls mindestens ein Heteroatom, insbesondere in der Form von Ether-Sauerstoff oder tertiärem Amin-Stickstoff, enthält, steht,
      oder
   zusammen mit R⁹ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
X¹ für O oder S oder N-R⁸ oder N-R⁹ steht,
   wobei R⁸
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (IIa) steht, 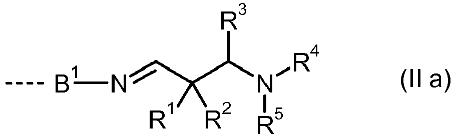 wobei B¹ für einen zweiwertigen, gegebenenfalls Ether-Sauerstoff oder tertiären Amin-Stickstoff aufweisenden, Kohlenwasserstoffrest mit 2 bis 12 C-Atomen steht; und
R⁹ zusammen mit A¹ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht,
und R¹, R², R³, R⁴ und R⁵ die bereits genannten Bedeutungen aufweisen.

In einer weiteren Ausführungsform der Aldimine der Formel (I) steht der Index m für null und der Index n steht für 2 oder 3 oder 4. Solche Aldimine stellen Polyaldimine dar. Mit "Poly" beginnende Substanznamen wie Polyaldimin, Polyamin oder Polyisocyanat bezeichnen im vorliegenden Dokument Substanzen, die formal zwei oder mehr der in ihrem Namen vorkommenden funktionellen Gruppen pro Molekül enthalten.

Besonders bevorzugte Aldimine der Formel (I) mit m = 0 stellen Aldimine der Formel (Ib) dar, 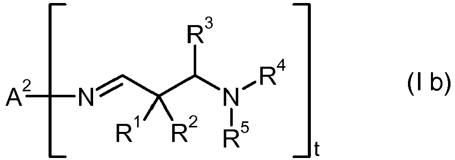 wobei
t für 2 oder 3, bevorzugt 2, steht;
A² für den Rest eines Polyamins mit t primären Aminogruppen nach Entfernung von t primären Aminogruppen steht und keinen aktiven Wasserstoff enthält;
und R¹, R², R³, R⁴ und R⁵ die bereits genannten Bedeutungen aufweisen.

Die Aldimine der Formel (Ib) weisen keinen aktiven Wasserstoff auf.

Aldimine der Formel (I) sind erhältlich aus der Umsetzung von mindestens einem Amin **B** der Formel (III) mit mindestens einem sterisch gehinderten aliphatischen Aldehyd **ALD** der Formel (IV), 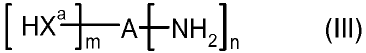 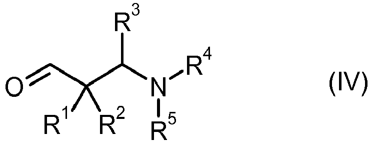 wobei
X^{a} für O oder S oder N-R^{6a} oder N-R⁷ steht,
   wobei R^{6a} entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (III') steht,

   --- B⁅NH₂]ₚ (III')
und m, n, A, B, R¹, R², R³, R⁴, R⁵ und p die bereits genannten Bedeutungen aufweisen.

Die Umsetzung zwischen einem Amin **B** der Formel (III) und einem Aldehyd **ALD** der Formel (IV) erfolgt in einer Kondensationsreaktion unter Abspaltung von Wasser. Solche Kondensationsreaktionen sind bestens bekannt und beschrieben, beispielsweise in Houben-Weyl, "Methoden der organischen Chemie", Vol. XI/2, Seite 73ff. Der Aldehyd **ALD** wird hierbei in Bezug auf die primären Aminogruppen des Amins **B** stöchiometrisch oder in stöchiometrischem Überschuss eingesetzt. Üblicherweise werden solche Kondensationsreaktionen in Anwesenheit eines Lösemittels durchgeführt, mittels welchem das bei der Reaktion entstehende Wasser azeotrop entfernt wird. Zur Herstellung der Aldimine der Formel (I) wird jedoch ein Herstellverfahren ohne Verwendung von Lösemitteln bevorzugt, wobei das bei der Kondensation gebildete Wasser direkt mittels Anlegen von Vakuum aus der Reaktionsmischung entfernt wird. Durch die lösemittelfreie Herstellung erübrigt sich ein Abdestillieren des Lösemittels nach erfolgter Herstellung, was den Herstellungsprozess vereinfacht. Zudem ist das Aldimin so frei von Lösemittelrückständen, welche einen störenden Geruch verursachen könnten.

Als Amin **B** geeignet sind Verbindungen, welche neben einer oder mehreren primären Aminogruppen mindestens eine einen aktiven Wasserstoff tragende Reaktivgruppe in der Form einer Hydroxyl-, Mercapto- oder sekundäre Aminogruppe aufweisen. Beispiele für Amine **B** mit mehr als einer aktiven Wasserstoff tragenden Reaktivgruppe sind
- mehr als eine sekundäre Aminogruppe und eine oder mehrere primäre Aminogruppen tragende aliphatische Amine, wie N,N'-Bis-(3-amino-propyl)ethylendiamin, Triethylentetramin (TETA), Tetraethylenpentamin (TEPA), Pentaethylenhexamin und höhere Homologe linearer Polyethylenamine, N,N'-Bis-(3-aminopropyl)-ethylendiamin, Produkte aus der mehrfachen Cyanoethylierung oder Cyanobutylierung und anschliessender Hydrierung von primären Di- und Polyaminen mit mehreren primären Aminogruppen, wie N,N'-Bis-(3-aminopropyl)-ethylendiamin, N,N'-Bis-(3-aminopropyl)-1,4-diaminobutan, N,N'-Bis-(3-aminopropyl)-2-methyl-1,5-pentandiamin, N,N'-Bis-(3-amino-1-ethylpropyl)-2-methyl-1,5-pentandiamin sowie Polyethylenimine unterschiedlichen Polymerisationsgrades (Molmassen-Bereich 500 bis 1'000'000 g/mol), wie sie beispielsweise unter dem Handelsnamen Lupasol® von BASF in reiner Form oder als wässrige Lösungen erhältlich sind, wobei diese Polyethylenimine neben primären und sekundären auch tertiäre Aminogruppen enthalten;
- mehr als eine Hydroxylgruppe und eine oder mehrere primäre Aminogruppen tragende Hydroxyamine, insbesondere Derivate von polyalkoxylierten drei- oder höherwertigen Alkoholen oder von polyalkoxylierten Polyaminen, sowie Aminozucker, beispielsweise Glucosamin oder Galactosamin;
- mindestens eine Hydroxyl- und mindestens eine sekundäre Aminogruppen tragende Hydroxypolyamine aus der Cyanoethylierung oder Cyanobutylierung und anschliessender Hydrierung von Hydroxyaminen wie N-Hydroxyethyl-1,2-ethandiamin, N-Hydroxypropyl-1,2-ethandiamin, N-Hydroxyethyl-1,3-propandiamin, N3-Hydroxyethyl-1,3-pentandiamin.

Als Amin **B** geeignet sind weiterhin Polyamine, welche zwei oder mehr primäre aliphatische Aminogruppen aufweisen. Beispiele für Amine **B** mit mehr als drei primären aliphatischen Aminogruppen sind Polyvinylamine oder primäre Aminogruppen tragende Copolymerisate, beispielsweise aus Allylamin und (Meth)acrylaten.

Als Amin **B** besonders geeignet sind zum einen Amine **B1** der Formel (IIIa), 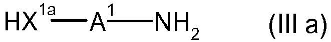 wobei
X^{1a} für O oder S oder N-R^{8a} oder N-R⁹ steht,
   wobei R^{8a} entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (IIIa') steht, 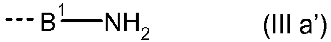
   und A¹, B¹ und R⁹ die bereits genannten Bedeutungen aufweisen.

Die Amine **B1** sind insbesondere geeignet zur Herstellung von Aldiminen der Formel (Ia).

Beispiele für Amine **B1** sind
- Verbindungen mit einer oder zwei primären aliphatischen und einer sekundären Aminogruppe, wie beispielsweise N-Methyl-1,2-ethandiamin, N-Ethyl-1,2-ethandiamin, N-Butyl-1,2-ethandiamin, N-Hexyl-1,2-ethandiamin, N-(2-Ethylhexyl)-1,2-ethandiamin, N-Cyclohexyl-1,2-ethandiamin, 4-Aminomethyl-piperidin, 3-(4-Aminobutyl)-piperidin, N-(2-Aminoethyl)piperazin, Diethylentriamin (DETA), Bis-hexamethylentriamin (BHMT), 3-(2-Aminoethyl)amino-propylamin; Di- und Triamine aus der Cyanoethylierung oder Cyanobutylierung und anschliessender Hydrierung von primären Mono- und Diaminen, beispielsweise N-Methyl-1,3-propandiamin, N-Ethyl-1,3-propandiamin, N-Butyl-1,3-propandiamin, N-Hexyl-1,3-propandiamin, N-(2-Ethylhexyl)-1,3-propandiamin, N-Dodecyl-1,3-propandiamin, N-Cyclohexyl-1,3-propandiamin, 3-Methyl-amino-1-pentylamin, 3-Ethylamino-1-pentylamin, 3-Butylamino-1-pentylamin, 3-Hexylamino-1-pentylamin, 3-(2-Ethylhexyl)amino-1-pentylamin, 3-Dodecylamino-1-pentylamin, 3-Cyclohexylamino-1-pentylamin, Dipropylentriamin (DPTA), N3-(3-Aminopentyl)-1,3-pentandiamin, N5-(3-Aminopropyl)-2-methyl-1,5-pentandiamin, N5-(3-Amino-1-ethylpropyl)-2-methyl-1,5-pentandiamin, und Fettdiamine wie N-Cocoalkyl-1,3-propandiamin, N-Oleyl-1,3-propandiamin, N-Soyaalkyl-1,3-propandiamin, N-Talgalkyl-1,3-propandiamin oder N-(C₁₆₋₂₂-Alkyl)-1,3-propandiamin, wie sie beispielsweise unter dem Handelsnamen Duomeen® von Akzo Nobel erhältlich sind; die Produkte aus der Michael-artigen Addition von aliphatischen primären Di- oder Triaminen mit Acrylnitril, Malein- oder Fumarsäurediestern, Citraconsäurediestern, Acryl- und Methacrylsäureestern, Acryl- und Methacrylsäureamiden und Itaconsäurediestern, umgesetzt im Molverhältnis 1:1;
- aliphatische Hydroxyamine, wie beispielsweise 2-Aminoethanol, 2-Methylaminoethanol (= 2-Amino-1-propanol), 1-Amino-2-propanol, 3-Amino-1-propanol, 4-Amino-1-butanol, 4-Amino-2-butanol, 2-Amino-2-methylpropanol, 5-Amino-1-pentanol, 6-Amino-1-hexanol, 7-Amino-1-heptanol, 8-Amino-1-octanol, 10-Amino-1-decanol, 12-Amino-1-dodecanol, 4-(2-Aminoethyl)-2-hydroxyethylbenzol, 3-Aminomethyl-3,5,5-trimethyl-cyclohexanol; eine primäre Aminogruppe tragende Derivate von Glykolen wie Diethylenglykol, Dipropylenglykol, Dibutylenglykol und höheren Oligomeren und Polymeren dieser Glykole, beispielsweise 2-(2-Aminoethoxy)-ethanol, Triethylenglykolmonoamin (= 2-(2-(2-Aminoethoxy)ethoxy)ethanol), α-(2-Hydroxymethylethyl)-ω-(2-aminomethylethoxy)-poly(oxy(methyl-1,2-ethandiyl)); eine Hydroxylgruppe und eine primäre Aminogruppen tragende Derivate von polyalkoxylierten drei- oder höherwertigen Alkoholen; Produkte aus der einfachen Cyanoethylierung und anschliessender Hydrierung von Glykolen, beispielsweise 3-(2-Hydroxy-ethoxy)-propylamin, 3-(2-(2-Hydroxyethoxy)-ethoxy)-propylamin und 3-(6-Hydroxyhexyloxy)-propylamin;
- aliphatische Mercaptoamine, wie beispielsweise 2-Aminoethanthiol (Cysteamin), 3-Aminopropanthiol, 4-Amino-1-butanthiol, 6-Amino-1-hexanthiol, 8-Amino-1-octanthiol, 10-Amino-1-decanthiol, 12-Amino-1-dodecanthiol und Aminothiozucker wie 2-Amino-2-deoxy-6-thioglucose.

Als Amin **B1** bevorzugt sind N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT und Fettdiamine wie N-Cocoalkyl-1,3-propandiamin, N-Oleyl-1,3-propandiamin, N-Soyaalkyl-1,3-propandiamin und N-Talgalkyl-1,3-propandiamin; Produkte aus der Michael-artigen Additionsreaktion von aliphatischen primären Diaminen mit Malein- und Fumarsäurediestern, Acryl- und Methacrylsäureestern, Acryl- und Methacrylsäureamiden, bevorzugt mit Maleinsäurediestern, insbesondere Maleinsäuredimethyl-, -diethyl-, -dipropyl- und -dibutylester, und mit Acrylsäureestern, insbesondere Acrylsäuremethylester, umgesetzt im Molverhältnis 1:1; sowie aliphatische Hydroxy- oder Mercaptoamine, in denen die primäre Aminogruppe von der Hydroxyl- oder Mercaptogruppe durch eine Kette von mindestens 5 Atomen, oder durch einen Ring, getrennt sind, insbesondere 5-Amino-1-pentanol, 6-Amino-1-hexanol und höhere Homologe davon, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin und höhere Oligo- und Polymere davon, 3-(2-Hydroxy-ethoxy)-propylamin, 3-(2-(2-Hydroxyethoxy)-ethoxy)-propylamin und 3-(6-Hydroxyhexyloxy)-propylamin.

Als Amin **B1** besonders bevorzugt sind Amine, welche ausgewählt sind aus der Gruppe bestehend aus N-Methyl-1,2-ethandiamin, N-Ethyl-1,2-ethandiamin, N-Cyclohexyl-1,2-ethandiamin, N-Methyl-1,3-propandiamin, N-Ethyl-1,3-propandiamin, N-Butyl-1,3-propandiamin, N-Cyclohexyl-1,3-propandiamin, 4-Aminomethyl-piperidin, 3-(4-Aminobutyl)-piperidin, DETA, DPTA, BHMT, Fettdiamine wie N-Cocoalkyl-1,3-propandiamin, N-Oleyl-1,3-propandiamin, N-Soyaalkyl-1,3-propandiamin und N-Talgalkyl-1,3-propandiamin, 5-Amino-1-pentanol, 6-Amino-1-hexanol, 4-(2-Aminoethyl)-2-hydroxyethylbenzol, 3-Aminomethyl-3,5,5-trimethyl-cyclohexanol, 2-(2-Aminoethoxy)-ethanol, Triethylenglykol-monoamin, 3-(2-Hydroxyethoxy)-propylamin, 3-(2-(2-Hydroxyethoxy)-ethoxy)-propylamin und 3-(6-Hydroxyhexyloxy)-propylamin.

Als Amin B besonders geeignet sind zum anderen Amine **B2** der Formel (IIIb), 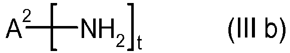 wobei A² und t die bereits genannten Bedeutungen aufweisen.

Die Amine **B2** sind insbesondere geeignet zur Herstellung von Aldiminen der Formel (Ib).

Beispiele für Amine **B2** sind
- aliphatische, cycloaliphatische oder arylaliphatische Diamine, beispielsweise Ethylendiamin, 1,2-Propandiamin, 1,3-Propandiamin, 2-Methyl-1,2-propandiamin, 2,2-Dimethyl-1,3-propandiamin, 1,3-Butandiamin, 1,4-Butandiamin, 1,3-Pentandiamin (DAMP), 1,5-Pentandiamin, 1,5-Diamino-2-methylpentan (MPMD), 1,6-Hexandiamin, 2,5-Dimethyl-1,6-hexandiamin, 2,2,4- und 2,4,4-Trimethylhexamethylendiamin (TMD), 1,7-Heptandiamin, 1,8-Octandiamin, 1,9-Nonandiamin, 1,10-Decandiamin, 1,11-Undecandiamin, 1,12-Dodecandiamin und Methyl-bis-(3-aminopropyl)amin, 1,2-, 1,3- und 1,4-Diaminocyclohexan, Bis-(4-aminocyclohexyl)-methan, Bis-(4-amino-3-methylcyclohexyl)-methan, Bis-(4-amino-3-ethylcyclohexyl)-methan, Bis-(4-amino-3,5-dimethylcyclohexyl)-methan, Bis-(4-amino-3-ethyl-5-methylcyclohexyl)-methan (M-MECA), 1-Amino-3-aminomethyl-3,5,5-trimethylcyclohexan (= Isophorondiamin oder IPDA), 2- und 4-Methyl-1,3-diaminocyclohexan und Mischungen davon, 1,3- und 1,4-Bis-(aminomethyl)cyclohexan, 2,5(2,6)-Bis-(aminomethyl)-bicyclo[2.2.1]heptan (NBDA), 3(4),8(9)-Bis-(aminomethyl)-tricyclo[5.2.1.0^{2,6}]-decan, 1,4-Diamino-2,2,6-trimethylcyclohexan (TMCDA), 3,9-Bis-(3-amino-propyl)-2,4,8,10-tetraoxaspiro[5.5]undecan sowie 1,3- und 1,4-Xylylendiamin;
- Ethergruppen-haltige aliphatische Diamine, beispielsweise Bis-(2-aminoethyl)ether, 3,6-Dioxaoctan-1,8-diamin, 4,7-Dioxadecan-1,10-diamin, 4,7-Dioxadecan-2,9-diamin, 4,9-Dioxadodecan-1,12-diamin, 5,8-Dioxadodecan-3,10-diamin und höhere Oligomere dieser Diamine, Bis-(3-aminopropyl)-polytetrahydrofurane und andere Polytetrahydrofuran-diamine mit Molekulargewichten im Bereich von beispielsweise 350 bis 5200, sowie Polyoxyalkylen-Diamine. Letztere stellen typischerweise Produkte aus der Aminierung von Polyoxyalkylen-Diolen dar und sind beispielsweise erhältlich unter dem Namen Jeffamine® (von Huntsman Chemicals), unter dem Namen Polyetheramin (von BASF) oder unter dem Namen PC Amine® (von Nitroil). Insbesondere geeignete Polyoxyalkylen-Diamine sind Jeffamine® D-230, Jeffamine® D-400, Jeffamine® D-2000, Jeffamine® D-4000, Jeffamine® XTJ-511, Jeffamine® ED-600, Jeffamine® ED-900, Jeffamine® ED-2003, Jeffamine® XTJ-568, Jeffamine® XTJ-569, Jeffamine® XTJ-523, Jeffamine® XTJ-536, Jeffamine® XTJ-542, Jeffamine® XTJ-559; Polyetheramin D 230, Polyetheramin D 400 und Polyetheramin D 2000, PC Amine® DA 250, PC Amine® DA 400, PC Amine® DA 650 und PC Amine® DA 2000;
- aliphatische Triamine wie 4-Aminomethyl-1,8-octandiamin, 1,3,5-Tris(aminomethyl)-benzol, 1,3,5-Tris-(aminomethyl)-cyclohexan;
- Polyoxyalkylen-Triamine, welche typischerweise Produkte aus der Aminierung von Polyoxyalkylen-Triolen darstellen und beispielsweise erhältlich sind unter dem Handelsnamen Jeffamine® (von Huntsman Chemicals), unter dem Namen Polyetheramin (von BASF) oder unter dem Namen PC Amine® (von Nitroil), wie zum Beispiel Jeffamine® T-403, Jeffamine® T-5000; Polyetheramin T403, Polyetheramin T5000; und PC Amine® TA 403, PC Amine® TA 5000.

Als Amin **B2** bevorzugt sind Polyamine, welche ausgewählt sind aus der Gruppe bestehend aus 1,6-Hexamethylendiamin, MPMD, DAMP, IPDA, TMD, 1,3-Xylylendiamin, 1,3-Bis-(aminomethyl)cyclohexan, Bis-(4-aminocyclohexyl)-methan, Bis-(4-amino-3-methylcyclohexyl)-methan, 3(4),8(9)-Bis-(aminomethyl)-tricyclo[5.2.1.0^{2,6}]decan, 1,2-, 1,3- und 1,4-Diaminocyclohexan, 1,4-Diamino-2,2,6-trimethylcyclohexan, 3,6-Dioxaoctan-1,8-diamin, 4,7-Dioxadecan-1,10-diamin, 4-Aminomethyl-1,8-octandiamin und Polyoxyalkylen-Polyamine mit zwei oder drei Aminogruppen, insbesondere die unter dem Handelsnamen Jeffamine® erhältlichen Typen D-230, D-400, D-2000, T-403 und T-5000 von Huntsman und dazu analoge Verbindungen von BASF oder Nitroil, sowie Mischungen der genannten Polyamine. Als Amin **B2** besonders bevorzugt sind die genannten Diamine.

Zur Herstellung eines Aldimins der Formel (I) wird weiterhin mindestens ein sterisch gehinderter aliphatischer Aldehyd **ALD** der Formel (IV) eingesetzt, 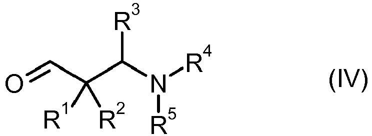 wobei R¹, R², R³, R⁴ und R⁵ die bereits genannten Bedeutungen aufweisen.

Bevorzugt stehen R¹ und R² jeweils für eine Methylgruppe, und R³ steht bevorzugt für ein Wasserstoffatom.

Bevorzugt stehen R⁴ und R⁵ jeweils unabhängig voneinander für Ethyl, Propyl, Isopropyl, Butyl, 2-Ethylhexyl, Cyclohexyl oder Benzyl, oder sie bilden zusammen - unter Einbezug des Stickstoffatoms - einen Ring, insbesondere einen Pyrrolidin-, Piperidin,- Morpholin- oder N-Alkylpiperazin-Ring, wobei dieser Ring gegebenenfalls substituiert ist. Besonders bevorzugt weisen R⁴ und R⁵ keinen tertiären Amin-Stickstoff auf.

Aldehyde **ALD** der Formel (IV) sind insbesondere erhältlich als Produkt einer Mannich-Reaktion oder einer der Mannich-Reaktion analogen α-Aminoalkylierung, wie sie aus der Fachliteratur bekannt ist; sie können deshalb auch als Mannich-Basen bezeichnet werden. Ein Aldehyd **Y1** der Formel (V), ein Aldehyd **Y2** der Formel (VI) und ein sekundäres aliphatisches Amin C der Formel (VII) werden dabei unter Wasserabspaltung zu einem Aldehyd **ALD** umgesetzt, 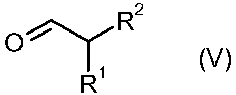 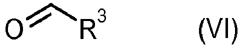 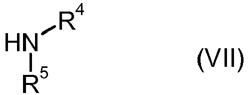 wobei R¹, R², R³, R⁴ und R⁵ die bereits genannten Bedeutungen aufweisen.

Diese Umsetzung kann entweder mit den freien Reagentien **Y1, Y2** und **C** gemäss den Formeln (V), (VI) und (VII) geführt werden, oder die Reagentien können teilweise oder vollständig in derivatisierter Form eingesetzt werden. So kann der Aldehyd **Y1** beispielsweise als Enolat, als Enolether, insbesondere als Silylenolether, oder als Enamin eingesetzt werden. Der Aldehyd **Y2** kann beispielsweise in Form eines Oligomeren - im Fall von Formaldehyd insbesondere als 1,3,5-Trioxan oder als Paraformaldehyd - oder als Hydrat, Hemiacetal, Acetal, N,O-Acetal, Aminal oder Hemiaminal eingesetzt werden. Das sekundäre aliphatische Amin **C** schliesslich kann beispielsweise als Salz, insbesondere als Amin-Hydrochlorid oder als Amin-Hydrosulfat, oder als Silylamin eingesetzt werden. Es ist möglich, einen Teil der Reagentien in freier Form und einen Teil in derivatisierter Form einzusetzen, oder nur von derivatisierten Formen auszugehen. Bei der Verwendung von Reagentien in derivatisierter Form fällt der Aldehyd **ALD** unter Umständen ebenfalls in derivatisierter Form, beispielsweise als Salz, an; in diesem Fall kann er durch geeignete Aufarbeitung in die freie Form gemäss Formel (IV) übergeführt werden. Je nachdem kann es sinnvoll sein, in solchen Umsetzungsreaktionen zusätzlich Hilfsstoffe wie Lewissäuren oder Katalysatoren einzusetzen.

Weiterhin kann die Umsetzung als Eintopfreaktion geführt werden, in der alle drei Reagentien gleichzeitig miteinander reagieren können; oder aber es kann ein stufenweises Vorgehen gewählt werden, indem zwei der Reagentien vorgängig miteinander umgesetzt werden und das so erhaltene Zwischenprodukt anschliessend mit dem dritten Reagens umgesetzt wird, wobei das Zwischenprodukt isoliert werden kann oder nicht. Als solche Zwischenprodukte geeignet sind insbesondere Iminiumsalze, welche aus der Umsetzung eines Aldehyds **Y2**, in freier oder derivatisierter Form, mit einem Salz eines sekundären aliphatischen Amins **C** erhalten werden und welche sich mit einem Aldehyd **Y1**, in freier oder derivatisierter Form, zum entsprechenden Salz eines Aldehyds **ALD** der Formel (IV) umsetzen lassen. Ein solcherart stufenweises Vorgehen kann den Vorteil haben, mildere Reaktionsbedingungen zu ermöglichen und damit eine höhere Produktausbeute zu liefern.

Weiterhin kann die Umsetzung unter Verwendung von Lösemitteln, insbesondere polaren Lösemitteln wie Wasser oder Alkoholen, durchgeführt werden, oder die Umsetzung kann ohne Verwendung von Lösemitteln erfolgen.

In einer bevorzugten Ausführungsform wird die Umsetzung mit allen Reagentien in freier Form als Eintopfreaktion geführt und der Aldehyd **ALD** nach erfolgter Umsetzung durch Destillation gereinigt. Bevorzugt werden dabei keine organischen Lösemittel eingesetzt.

Als Aldehyd **Y1** der Formel (V) geeignet sind beispielsweise die folgenden Aldehyde: Isobutyraldehyd, 2-Methylbutyraldehyd, 2-Ethylbutyraldehyd, 2-Methylvaleraldehyd, 2-Ethylcapronaldehyd, Cyclopentancarboxaldehyd, Cyclohexancarboxaldehyd, 1,2,3,6-Tetrahydrobenzaldehyd, 2-Methyl-3-phenylpropionaldehyd, 2-Phenylpropionaldehyd und Diphenylacetaldehyd. Bevorzugt ist Isobutyraldehyd.

Als Aldehyd **Y2** der Formel (VI) geeignet sind beispielsweise die folgenden Aldehyde: Formaldehyd, Acetaldehyd, Propionaldehyd, Butyraldehyd, Isobutyraldehyd, Phenylacetaldehyd, Benzaldehyd und substituierte Benzaldehyde sowie Glyoxylsäureester, insbesondere Glyoxylsäureethylester. Bevorzugt ist Formaldehyd.

Als Amin C der Formel (VII) geeignet sind beispielsweise die folgenden sekundären aliphatischen Amine: Dimethylamin, Diethylamin, Dipropylamin, Diisopropylamin, Dibutylamin, Diisobutylamin, Di-sek.-Butylamin, Dihexylamin, Di-(2-ethylhexyl)amin, Dicyclohexylamin, N-Methylbutylamin, N-Ethylbutylamin, N-Methylcyclohexylamin, N-Ethylcyclohexylamin, Di-2-methoxyethylamin, Pyrrolidin, Piperidin, N-Methyl-benzylamin, N-Isopropyl-benzylamin, N-tert.Butyl-benzylamin, Dibenzylamin, Morpholin, 2,6-Dimethylmorpholin, Bis-(3-Dimethylaminopropyl)amin, N-Methyl- oder N-Ethylpiperazin.

Als Amin **C** bevorzugt sind Dimethylamin, Diethylamin, Diisopropylamin, Dibutylamin, Diisobutylamin, N-Methylcyclohexylamin, N-Methyl-benzylamin, N-Isopropyl-benzylamin, N-tert.Butyl-benzylamin, Dibenzylamin, Pyrrolidin, Piperidin, Morpholin und 2,6-Dimethylmorpholin.

Bevorzugt wird der Aldehyd **ALD** hergestellt durch die Umsetzung von Isobutyraldehyd als Aldehyd **Y1** der Formel (V), Formaldehyd als Aldehyd **Y2** der Formel (VI) und einem der Amine ausgewählt aus der Gruppe umfassend Dimethylamin, Diethylamin, Diisopropylamin, Dibutylamin, Diisobutylamin, N-Methylcyclohexylamin, N-Methyl-benzylamin, N-Isopropyl-benzylamin, N-tert.Butyl-benzylamin, Dibenzylamin, Pyrrolidin, Piperidin, Morpholin und 2,6-Dimethylmorpholin als Amin C der Formel (VII).

Bevorzugte Aldehyde **ALD** sind 2,2-Dimethyl-3-dimethylamino-propanal, 2,2-Dimethyl-3-diethylamino-propanal, 2,2-Dimethyl-3-dibutylamino-propanal, 2,2-Dimethyl-3-(N-pyrrolidino)-propanal, 2,2-Dimethyl-3-(N-piperidino)-propanal, 2,2-Dimethyl-3-(N-morpholino)-propanal, 2,2-Dimethyl-3-(N-(2,6-dimethyl)morpholino)-propanal, 2,2-Dimethyl-3-(N-benzylmethylamino)-propanal, 2,2-Dimethyl-3-(N-benzylisopropylamino)-propanal und 2,2-Dimethyl-3-(N-cyclohexylmethylamino)-propanal. Die bevorzugten Aldehyde **ALD** weisen keinen zusätzlichen tertiären Amin-Stickstoff und eine vergleichsweise niedrige Basizität auf.

Die Aldehyde **ALD** der Formel (IV) weisen eine Reihe von besonderen Eigenschaften auf. So besitzen sie eine gute thermische Stabilität, weil das C-Atom in α-Stellung zur Aldehydgruppe kein Wasserstoffatom trägt und deshalb die Eliminierung eines sekundären Amins unter Bildung eines Alkens nicht möglich ist. Ferner weisen sie eine überraschend gute Stabilität gegenüber Oxidation durch Luftsauerstoff auf. Weiterhin ist ihre Basizität - bezogen auf den zentralen, in 3-Stellung stehenden tertiären Amin-Stickstoff - überraschenderweise deutlich niedriger als für aliphatische Amine ähnlicher Struktur erwartet; der für die konjugierte Säure eines Aldehyds **ALD** gemessene *p*Kₐ-Wert liegt um ungefähr 2 Einheiten tiefer als jener der konjugierten Säure des zur Herstellung dieses Aldehyds **ALD** eingesetzten sekundären Amins C. Diese überraschenden Eigenschaften stehen eventuell in Zusammenhang mit einer intramolekularen 1,4-Wechselwirkung zwischen Amin- und Aldehydgruppe (Orbitalüberlappung zwischen dem freien Elektronenpaar des Stickstoffs und dem π- bzw. π*-Orbital des Carbonyls), wie sie von P.Y. Johnson et al. (J. Org. Chem., Vol. 40, Nr. 19, 1975; Seiten 2710-2720) anhand von NMR- und UV-spektroskopischen Untersuchungen an β-Aminoaldehyden postuliert wurde.

Schliesslich weisen die Aldehyde **ALD,** auch bei relativ niedrigem Molekulargewicht, keinen oder nur einen geringen, aminartigen Geruch auf. Diese für Aldehyde überraschende Eigenschaft der geringen Geruchsintensität kommt vermutlich einerseits durch die erwähnte intramolekulare 1,4-Wechselwirkung, andererseits durch die sterische Hinderung der Aldehydgruppe, welche an einem tertiären C-Atom steht, zustande.

Aldimine der Formel (I) lassen sich, wie bereits beschrieben, direkt aus Aminen **B** und Aldehyden **ALD** herstellen.

Aldimine der Formel (I), welche als als Substituenten X einen Substituenten N-R⁶ aufweisen, lassen sich gegebenenfalls auf einem leicht anderen Weg als dem bisher beschriebenen herstellen. Dieser Syntheseweg besteht darin, dass ein Aldehyd **ALD** der Formel (IV) mit einem zwei- oder dreiwertigen, bevorzugt zweiwertigen, aliphatischen primären Amin, wie es vorgängig als Amin **B2** bereits beschrieben wurde, in einem ersten Schritt zu einem Zwischenprodukt umgesetzt wird, welches neben einer oder zwei Aldiminogruppen noch eine oder zwei, bevorzugt eine, primäre Aminogruppe enthält. Dieses Zwischenprodukt wird anschliessend in einem zweiten Schritt zu einem Aldimin der Formel (I) umgesetzt, indem die primäre Aminogruppe einfach alkyliert wird. Für die Alkylierung werden insbesondere Verbindungen mit nur einer aktivierten Doppelbindung eingesetzt, welche mit primären Aminen Michael-artige Additionsreaktionen eingehen können; solche Verbindungen werden im Folgenden als "Michael-Akzeptor" bezeichnet.

Die Umsetzung eines Aldehyds **ALD** mit einem Amin **B2** zum eine primäre Aminogruppe aufweisenden Zwischenprodukt erfolgt in einer Kondensationsreaktion unter Abspaltung von Wasser, wie sie weiter oben für die Umsetzung eines Aldehyds **ALD** mit einem Amin **B** der Formel (III) beschrieben wird. Die Stöchiometrie zwischen dem Aldehyd **ALD** und dem Amin **B2** wird dabei aber so gewählt, dass 1 mol Aldehyd **ALD** für 1 mol Amin **B2**, welches zwei primäre Aminogruppen enthält, eingesetzt wird, oder so, dass zwei mol Aldehyd **ALD** für ein mol Amin **B2**, welches drei primäre Aminogruppen enthält, eingesetzt wird. Das dabei eingesetzte Amin **B2** ist in Bezug auf die Aminogruppen bevorzugt asymmetrisch. Es wird ein lösemittelfreies Herstellverfahren bevorzugt, wobei das bei der Kondensation gebildete Wasser mittels Anlegen von Vakuum aus der Reaktionsmischung entfernt wird.

Die Umsetzung des eine primäre Aminogruppe aufweisenden Zwischenprodukts mit dem Michael-Akzeptor erfolgt beispielsweise dadurch, dass das Zwischenprodukt mit einer stöchiometrischen oder leicht überstöchiometrischen Menge des Michael-Akzeptors gemischt wird und die Mischung bei Temperaturen von 20 bis 110 °C bis zum vollständigen Umsatz des Zwischenprodukts zum Aldimin der Formel (I) erwärmt wird. Die Umsetzung erfolgt bevorzugt ohne die Verwendung von Lösemitteln.

Als Amin **B2** für diese Herstellung bevorzugt sind Diamine, in denen die primären Aminogruppen durch eine Kette von mindestens 5 Atomen, oder durch einen Ring, getrennt sind, insbesondere 1,5-Diamino-2-methylpentan, 1,6-Hexamethylendiamin, 2,2,4- und 2,4,4-Trimethylhexamethylendiamin und Mischungen davon, 1,10-Decandiamin, 1,12-Dodecandiamin, 1,3- und 1,4-Diaminocyclohexan, Bis-(4-aminocyclohexyl)-methan, Bis-(4-amino-3-methylcyclohexyl)-methan, 1-Amino-3-aminomethyl-3,5,5-trimethylcyclohexan, 1,3- und 1,4-Bis-(aminomethyl)cyclohexan, 2,5(2,6)-Bis-(aminomethyl)-bicyclo-[2.2.1]heptan, 3(4),8(9)-Bis-(aminomethyl)-tricyclo[5.2.1.0^{2,6}]decan, 1,4-Di-amino-2,2,6-trimethylcyclohexan (TMCDA), 3,9-Bis-(3-aminopropyl)-2,4,8,10-tetraoxaspiro[5.5]undecan, 1,3- und 1,4-Xylylendiamin, sowie die genannten Ethergruppen-haltigen aliphatischen Diamine und Polyoxyalkylen-Diamine.

Beispiele für geeignete Michael-Akzeptoren sind Malein- oder Fumarsäurediester wie Dimethylmaleinat, Diethylmaleinat, Dibutylmaleinat, Diethylfumarat; Citraconsäurediester wie Dimethylcitraconat; Acryl- oder Methacrylsäureester wie Methyl(meth)acrylat, Ethyl(meth)acrylat, Butyl(meth)acrylat, Lauryl(meth)acrylat, Stearyl(meth)acrylat, Tetrahydrofuryl(meth)acrylat, Isobornyl(meth)acrylat; Itaconsäurediester wie Dimethylitaconat; Zimtsäureester wie Methylcinnamat; Vinylphosphonsäurediester wie Vinylphosphonsäuredimethylester; Vinylsulfonsäureester, insbesondere Vinylsulfonsäurearylester; Vinylsulfone; Vinylnitrile wie Acrylnitril, 2-Pentennitril oder Fumaronitril; 1-Nitroethylene wie β-Nitrostyrol; und Knoevenagel-Kondensationsprodukte, wie beispielsweise solche aus Malonsäurediestern und Aldehyden wie Formaldehyd, Acetaldehyd oder Benzaldehyd. Bevorzugt sind Maleinsäurediester, Acrylsäureester, Phosphonsäurediester und Vinylnitrile.

Diejenigen Ausführungsformen von Aldiminen der Formel (I), welche mindestens eine Gruppe HX aufweisen, können gegebenenfalls im Gleichgewicht stehen mit cyclischen Formen, wie sie in Formel (VIII) beispielhaft für den Fall mit Index m=1 gezeigt sind. Diese cyclischen Formen sind im Fall von Aminoaldiminen cyclische Aminale, beispielsweise Imidazolidine oder Tetrahydropyrimidine; im Fall von Hydroxyaldiminen cyclische Aminoacetale, beispielsweise Oxazolidine oder Tetrahydrooxazine; im Fall von Mercaptoaldiminen cyclische Thioaminale, beispielsweise Thiazolidine oder Tetrahydrothiazine.

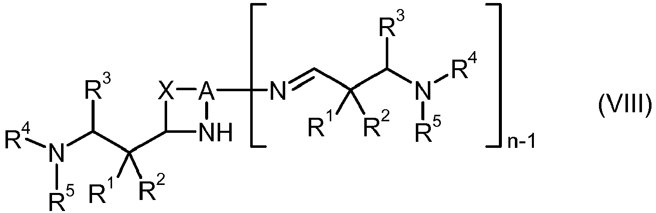

In der Formel (VIII) weisen n, A, R¹, R², R³, R⁴, R⁵ und X die bereits genannten Bedeutungen auf.

Überraschenderweise neigen die meisten HX-Gruppen enthaltenden Aldimine der Formel (I) nicht zur Cyclisierung. Insbesondere für Aminoaldimine kann mittels IR- und NMR-spektroskopischen Methoden gezeigt werden, dass diese Verbindungen überwiegend in der offenkettigen, also der Aldimin-Form, vorliegen, während die cyclische, also die Aminal-Form, nicht oder nur in Spuren vorkommt. Auch Hydroxy- und Mercaptoamine, in denen die primäre Aminogruppe von der Hydroxyl- beziehungsweise der Mercaptogruppe durch eine Kette von mindestens 5 Atomen, oder durch einen Ring, getrennt sind, zeigen kaum Cyclisierung.

Bei den Aldiminen der Formel (I) handelt es sich um neue, bisher nicht beschriebene Verbindungen mit überraschenden Eigenschaften. Sie enthalten sterisch gehinderte Aldiminogruppen, welche am in α-Stellung stehenden C-Atom kein Wasserstoffatom aufweisen und deshalb nicht zu Enaminogruppen tautomerisieren können. Dadurch stellen diese Aldiminogruppen besonders gut geschützte ("blockierte") primäre Aminogruppen dar, welche unter Ausschluss von Feuchtigkeit mit gegenüber Aminogruppen reaktiven Verbindungen keine oder nur geringe Reaktivität zeigen. Weiterhin weisen die Aldimine der Formel (I) eine tertiäre Aminogruppe auf, welche unter Umständen in chemischen Reaktionssystemen eine katalytische Wirkung entfalten kann; die von der tertiären Aminogruppe ausgehende Basizität der Aldimine der Formel (I) ist aber überraschend niedrig. Weiterhin weisen die Aldimine der Formel (I), auch bei relativ niedrigem Molekulargewicht des zugrunde liegenden Aldehyds **ALD**, keinen oder nur einen geringen, aminartigen Geruch auf.

Die Aldimine der Formel (I) besitzen eine gute thermische Stabilität, da das C-Atom in α-Stellung zur Aldiminogruppe wie erwähnt kein Wasserstoffatom trägt und deshalb die Eliminierung eines sekundären Amins unter Bildung eines Alkens nicht möglich ist.

Die Aldimine der Formel (I) sind unter geeigneten Bedingungen lagerstabil. Bei Zutritt von Feuchtigkeit können ihre Aldiminogruppen über Zwischenstufen formal zu Aminogruppen hydrolysieren, wobei die entsprechenden, zur Herstellung der Aldimine verwendeten, Aldehyde **ALD** der Formel (IV) freigesetzt werden, welche, wie bereits beschrieben, geruchsarm oder geruchsfrei sind. Da diese Hydrolysereaktion reversibel ist und das chemische Gleichgewicht deutlich auf der Aldiminseite liegt, ist davon auszugehen, dass in Abwesenheit von gegenüber Aminen reaktiven Verbindungen nur ein Teil der Aldiminogruppen teilweise oder ganz hydrolysieren. Überraschenderweise lässt sich die Hydrolyse der Aldiminogruppen trotz der Anwesenheit von tertiären Aminogruppen mittels Säuren katalysieren.

Die Aldimine der Formel (I) sind aus gut erhältlichen AusgangsSubstanzen in einem relativ einfachen Verfahren herstellbar. Falls bei ihrer Herstellung dünnflüssige Amine **B** der Formel (III) verwendet wurden, sind die entsprechenden Aldimine der Formel (I) teilweise ebenfalls dünnflüssige Verbindungen.

Die Aldimine der Formel (I) lassen sich sehr breit verwenden. Sie lassen sich zum Beispiel überall dort einsetzen, wo sie als Quelle von Aldehyden **ALD** der Formel (IV) oder von Aminen **B** der Formel (III) dienen können. Insbesondere können sie in der Funktion von geschützten Aminen, beziehungsweise geschützten Aldehyden, in Aldehyd- und/oder Amin-reaktiven Systemen eingesetzt werden und dort bei Bedarf gezielt entschützt werden. Insbesondere finden sie in Systemen Anwendung, in welchen Verbindungen vorhanden sind, welche mit primären Aminen reagieren. Die Entschützung erfolgt hydrolytisch, beispielsweise durch Kontakt mit Wasser oder Feuchtigkeit, insbesondere Luftfeuchtigkeit. Überraschenderweise lässt sich die Hydrolyse der Aldiminogruppen trotz der Anwesenheit von tertiären Aminogruppen genauso mittels Säuren katalysieren wie bei Aldiminen ohne tertiäre Aminogruppen im Molekül.

Andererseits finden Aldimine der Formel (I) mit dem Index m grösser null Verwendung beim Aufbau von weiter funktionalisierten Reaktionsprodukten dieser Aldimine. So können Aldimine der Formel (I) mit dem Index m grösser null mit Verbindungen, welche mit der Gruppe HX reagieren können, umgesetzt werden, insbesondere in Additionsreaktionen. Geeignete solche Additionsreaktionen eingehende Verbindungen tragen Reaktivgruppen wie zum Beispiel Isocyanatgruppen, Epoxidgruppen, Anhydridgruppen oder mehr oder weniger stark aktivierte Doppel- oder Dreifachbindungen wie (Meth)acrylatgruppen, Acrylamidgruppen, 1-Ethinylcarbonylgruppen, 1-Propinylcarbonylgruppen, Maleimidgruppen, Citraconimidgruppen, Vinylgruppen, Isopropenylgruppen oder Allylgruppen. Die Aldiminogruppen tragenden Umsetzungsprodukte aus solchen Additionsreaktionen lassen sich bei Bedarf zu Aldehyden **ALD** der Formel (IV) und Verbindungen mit primären Aminogruppen hydrolysieren und darauf für weitere Reaktionen nutzen, beispielsweise für Vernetzungsreaktionen, wobei sich die Hydrolysereaktion mittels Säuren katalysieren lässt.

Weiterhin können die Aldimine der Formel (I) mit dem Index m grösser null zur Herstellung von Aldiminogruppen-haltigen Verbindungen eingesetzt werden, welche beispielsweise als latente Härter oder als Co-Monomere für reaktive Zusammensetzungen, insbesondere für Isocyanatgruppen aufweisende Zusammensetzungen, geeignet sind.

Ferner können die Aldimine der Formel (I) als Katalysatoren für chemische Reaktionssysteme eingesetzt werden, beispielsweise in härtbaren, Isocyanatgruppen aufweisenden Zusammensetzungen, insbesondere um deren Aushärtungszeit zu verkürzen.

Schliesslich können die Aldimine der Formel (I) als Quelle für kationische Verbindungen verwendet werden, indem die tertiären Aminogruppen teilweise oder ganz zu Ammoniumgruppen protoniert oder zu quaternären Ammoniumgruppen alkyliert werden. Durch Protonierung oder Alkylierung von Aldiminen der Formel (I) sind Aldimine der Formel (IX) zugänglich, 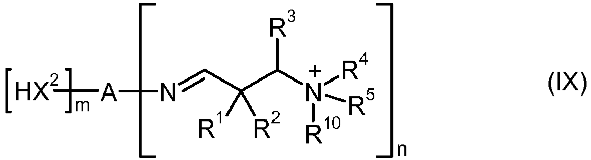 wobei
R¹⁰ für ein Wasserstoffatom oder für einen Alkyl-, Cycloalkyl- oder Arylalkylrest mit 1 bis 20 C-Atomen steht;
X² für O oder S oder N-R¹¹ oder N-R⁷ steht,
   wobei R¹¹
   entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
   oder für einen Substituenten der Formel (IX') steht; 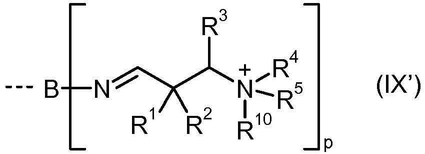
und m, n, p, A, B, R¹, R², R³, R⁴ und R⁵ und R⁷ die bereits genannten Bedeutungen aufweisen.

Aldimine der Formel (IX) sind weiterhin erhältlich ausgehend von einem der vorgängig erwähnten Aminen **B** der Formel (III) und einem Aldehyd **ALD** der Formel (IV), wobei die tertiären Aminogruppen des Aldehyds **ALD** vor der Umsetzung mit dem Amin **B** teilweise oder ganz protoniert oder alkyliert werden.

Zur Protonierung der Aldimine der Formel (I) oder der Aldehyde **ALD** können beliebige Brønsted-Säuren eingesetzt werden, beispielsweise Salzsäure, Schwefelsäure, Phosphorsäure, Carbonsäuren wie Essigsäure oder Benzoesäure sowie Sulfonsäuren wie Methansulfonsäure oder p-Toluolsulfonsäure. Zur Alkylierung der Aldimine der Formel (I) oder der Aldehyde **ALD** können bekannte Alkylierungsmittel, insbesondere Methylierungsmittel, eingesetzt werden, beispielsweise Methyliodid, Dimethylsulfat, Dimethylphosphonat, Diazomethan, Fluorschwefelsäuremethylester oder Trimethyloxonium-tetrafluoroborat.

Es ist dem Fachmann klar, dass zu einem kationischen Aldimin der Formel (IX) auch ein Anion gehört, das die positive Ladung des Aldimins ausgleicht.

Besonders geeignet sind die Aldimine der Formel (I) oder der Formel (IX) als Bestandteil von Zusammensetzungen basierend auf Isocyanaten oder Epoxidharzen, insbesondere für Anwendungen wie Klebstoffe, Dichtstoffe, Vergussmassen, Beschichtungen, Bodenbeläge, Anstriche, Lacke, Primer oder Schäume. Bevorzugt enthalten solche Zusammensetzungen mindestens eine Säure, insbesondere eine organische Carbon- oder Sulfonsäure, oder eine zu diesen Säuren hydrolysierbare Verbindung, wobei die Säure überraschenderweise die Hydrolyse der Aldiminogruppen trotz dem Vorhandensein von tertiären Aminogruppen katalysiert.

Insbesondere eignen sich die Aldimine der Formel (I) oder die Aldimine der Formel (IX) als Härter oder als Vorläufer für Härter für ein- oder zweikomponentige, Isocyanatgruppen aufweisende Zusammensetzungen wie Klebstoffe, Dichtstoffe, Vergussmassen, Beschichtungen, Bodenbeläge, Anstriche, Lacke, Primer oder Schäume.

Wie bereits erwähnt, enthalten die Aldimine der Formel (I) sterisch gehinderte und nicht zu Enaminogruppen tautomerisierbare Aldiminogruppen, welche besonders gut geschützte ("blockierte") primäre Aminogruppen darstellen. Zusammen mit Isocyanatgruppen aufweisenden Verbindungen können die Aldimine der Formel (I) unter Ausschluss von Feuchtigkeit lagerstabile, das heisst weitgehend viskositätskonstante, Mischungen bilden. Besonders lagerstabil sind Mischungen mit Verbindungen, welche freie aliphatische Isocyanatgruppen aufweisen, und/oder mit Verbindungen, welche - beispielsweise mit Phenolen - blockierte aromatische Isocyanatgruppen aufweisen.

Als "aliphatisch" wird eine Isocyanatgruppe oder ein Isocyanat bezeichnet, wenn die Isocyanatgruppe an einen aliphatischen, cycloaliphatischen oder arylaliphatischen Rest gebunden ist, im Gegensatz zu einem aromatischen Isocyanat oder einer aromatischen Isocyanatgruppe, wo die Isocyanatgruppe an einen aromatischen Rest gebunden ist.

Zusammensetzungen aus Isocyanatgruppen aufweisenden Verbindungen und Aldiminen der Formel (I) reagieren bei Kontakt mit Wasser unter Hydrolyse der Aldiminogruppen zu Harnstoffgruppen aufweisenden Verbindungen. Die Isocyanatgruppen reagieren dabei mit den durch die Hydrolyse der Aldiminogruppen formal frei werdenden primären Aminogruppen, wobei ein Aldehyd **ALD** freigesetzt wird. Im Verhältnis zu den Aldiminogruppen überschüssige Isocyanatgruppen reagieren direkt mit Feuchtigkeit und bilden ebenfalls Harnstoffgruppen. Bei geeigneter Stöchiometrie zwischen Isocyanatgruppen und Aldiminogruppen härtet die Zusammensetzung als Ergebnis dieser Reaktionen aus; dieser Prozess wird auch als Vernetzung bezeichnet. Die Reaktion der Isocyanatgruppen mit den hydrolysierenden Aldiminogruppen muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert. Überraschenderweise wird die säurekatalysierte Hydrolyse der Aldiminogruppen durch das Vorhandensein von tertiären Aminogruppen nicht beeinträchtigt.

Die tertiäre Aminogruppe der Aldimine der Formel (I) kann eine katalytische Wirkung auf die Reaktion der Isocyanatgruppen haben und kann deshalb die Vernetzung beschleunigen. Diese beschleunigende Wirkung wird zusätzlich unterstützt durch die Tatsache, dass die tertiäre Aminogruppe im Aldehyd-Teil des Aldimins lokalisiert ist. Es ist dabei aber vorteilhaft, dass die Basizität der tertiären Aminogruppen vergleichsweise niedrig ist, da stark basische tertiäre Amine die direkte Reaktion der Isocyanatgruppen, insbesondere mit Wasser, übermässig beschleunigen können, was sich bei der Aushärtung störend auswirken kann. Bei der Hydrolyse der Aldiminogruppen werden Aldehyde **ALD** der Formel (IV) enthaltend die tertiäre Aminogruppe freigesetzt. Aufgrund ihrer relativ geringen Grösse sind die Aldehyde **ALD** in der aushärtenden Zusammensetzung recht gut beweglich, was ihre katalytische Wirkung auf weitere Isocyanatgruppen potentiell zusätzlich erhöht. Nach erfolgter Aushärtung verbleiben die freigesetzten Aldehyde **ALD** in der ausgehärteten Zusammensetzung. Sie weisen dort eine ausgezeichnete Verträglichkeit auf, neigen nicht zum Ausschwitzen und haben nur eine geringe weichmachende Wirkung, was oft sehr vorteilhaft ist.

Es ist auch möglich, die Aldimine der Formel (I) zusammen mit Wasser aufzubewahren, unter der Voraussetzung, dass die Aldimine getrennt von Isocyanatgruppen gelagert werden. Erst wenn die Wasser-Aldimin-Mischung mit Isocyanatgruppen in Kontakt kommt, läuft die Hydrolyse vollständig ab. Die Reaktion zwischen Aldiminen der Formel (I) und Isocyanatgruppen ist nämlich auch dann stark verzögert im Vergleich zur Reaktion der entsprechenden primären Amine, wenn die Aldimine zusammen mit Wasser aufbewahrt werden.

Ebenfalls möglich ist die Verwendung von Aldiminen der Formel (I) oder der Formel (IX) in Zusammensetzungen, welche unter dem Einfluss von Wärme aushärten, beispielsweise durch die Verwendung von Verbindungen mit thermisch labilen, blockierten Isocyanatgruppen. Weiterhin möglich ist die Verwendung von Aldiminen der Formel (I) oder der Formel (IX) in Zusammensetzungen, welche reaktive Warm- oder Heissschmelzklebstoffe darstellen. Solche Klebstoffe enthalten schmelzbare, insbesondere Isocyanatgruppen aufweisende, Verbindungen; sie sind bei Raumtemperatur fest und werden warm oder heiss appliziert.

Ein weiterer Gegenstand der vorliegenden Erfindung sind Aldiminogruppen-haltige Verbindungen **AV**, welche Additionsprodukte darstellen aus der Umsetzung von mindestens einem Aldimin der Formel (I) mit m=1, insbesondere mindestens einem Aldimin der Formel (Ia), mit mindestens einer Verbindung **D**, die mindestens eine, bevorzugt mindestens zwei, Reaktivgruppen trägt, welche mit der Gruppe HX Additionsreaktionen eingehen kann, wobei die Aldiminogruppen-haltige Verbindung **AV** eine Aldiminogruppen-haltige Verbindung **AV1** der Formel (X) darstellt, 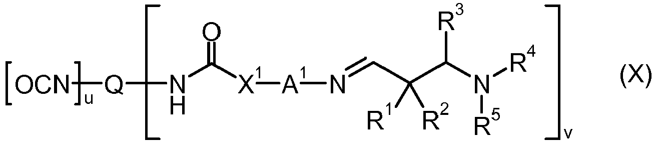 wobei
u für 0 oder 1 oder 2 oder 3 oder 4 oder 5 steht,
v für 1 oder 2 oder 3 oder 4 oder 5 oder 6 steht,
mit der Massgabe, dass (u+v) für 2 oder 3 oder 4 oder 5 oder 6 steht;
Q für den Rest eines (u+v) Isocyanatgruppen aufweisenden Polyisocyanates nach Entfernung aller Isocyanatgruppen steht;
und A¹, X¹, R¹, R², R³, R⁴ und R⁵ die bereits genannten Bedeutungen aufweisen.

Eine Aldiminogruppen-haltige Verbindung **AV1** der Formel (X) ist erhältlich durch die Umsetzung von mindestens einem Polyisocyanat der Formel (XI) mit mindestens einem nur einen aktiven Wasserstoff aufweisenden Aldimin der bereits genannten Formel (Ia).

Q⁅NCO]ᵤ₊ᵥ (XI)

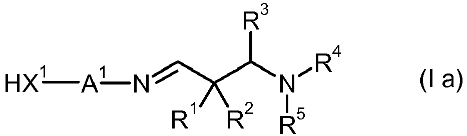

In der Formel (XI) weisen Q, u und v die bereits beschriebenen Bedeutungen auf.

Ein geeignetes Polyisocyanat der Formel (XI) ist in einer Ausführungsform ein Isocyanatgruppen aufweisendes Polyurethanpolymer **PUP.**

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Ein geeignetes Isocyanatgruppen aufweisendes Polyurethanpolymer **PUP** ist erhältlich durch die Umsetzung von mindestens einem Polyol mit mindestens einem Polyisocyanat.

Als Polyole für die Herstellung eines Polyurethanpolymers **PUP** können beispielsweise die folgenden Polyole oder Mischungen davon eingesetzt werden:
- Polyetherpolyole, auch Polyoxyalkylenpolyole oder Oligoetherole genannt, welche Polymerisationsprodukte von Ethylenoxid, 1,2-Propylenoxid, 1,2- oder 2,3-Butylenoxid, Tetrahydrofuran oder Mischungen davon sind, eventuell polymerisiert mit Hilfe eines Startermoleküls, wie beispielsweise Wasser, Ammoniak, 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.

Als Polyetherpolyole besonders geeignet sind Polyoxyalkylendiole und -triole, insbesondere Polyoxyalkylendiole. Besonders geeignete Polyoxyalkylendi- und triole sind Polyoxyethylendi- und -triole sowie Polyoxypropylendi- und -triole.

Besonders geeignet sind Polyoxypropylendiole und -triole mit einem Ungesättigtheitsgrad tiefer als 0.02 mEq/g und einem Molekulargewicht im Bereich von 1000 bis 30'000 g/mol, sowie Polyoxypropylendiole und -triole mit einem Molekulargewicht von 400 bis 8'000 g/mol. Unter 'Molekulargewicht' oder 'Molgewicht' versteht man im vorliegenden Dokument stets das Molekulargewichtsmittel Mₙ. Insbesondere geeignet sind Polyoxypropylendiole mit einem Ungesättigtheitsgrad tiefer als 0.02 mEq/g und einem Molekulargewicht im Bereich von 1000 bis 12'000, insbesondere zwischen 1000 und 8000 g/mol. Solche Polyetherpolyole werden beispielsweise unter dem Handelsnamen Acclaim® von Bayer vertrieben.

Ebenfalls besonders geeignet sind sogenannte "EO-endcapped" (ethylene oxide-endcapped) Polyoxypropylendiole und -triole. Letztere sind spezielle Polyoxypropylenpolyoxyethylenpolyole, die beispielsweise dadurch erhalten werden, dass reine Polyoxypropylenpolyole nach Abschluss der Polypropoxylierung mit Ethylenoxid alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.

Insbesondere geeignet sind Polyesterpolyole, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Caprolacton und Startern wie den vorgenannten zwei- oder dreiwertigen Alkoholen.

Besonders geeignete Polyesterpolyole sind Polyesterdiole.
- Polycarbonatpolyole, wie sie durch Umsetzung beispielsweise der oben genannten - zum Aufbau der Polyesterpolyole eingesetzten - Alkohole mit Dialkylcarbonaten, wie Dimethylcarbonat, Diarylcarbonaten, wie Diphenylcarbonat, oder Phosgen zugänglich sind.

Besonders geeignet sind Polycarbonatdiole.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen.
- Polyacrylat- und Polymethacrylatpolyole.
- Polyhydroxyfunktionelle Fette und Öle, beispielsweise natürliche Fette und Öle, inbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschliessender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschliessender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere, wie sie beispielsweise von der Firma Kraton Polymers hergestellt werden; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylnitril, Vinylchlorid, Vinylacetat, Vinylalkohol, Isobutylen und Isopren, beispielsweise polyhydroxyfunktionelle Acrylnitril/Butadien-Copolymere, welche zum Beispiel aus carboxylterminierten Acrylnitril/Butadien-Copolymeren (kommerziell erhältlich unter dem Namen Hycar® CTBN von Noveon) und Epoxiden oder Aminoalkoholen herstellbar sind; sowie hydrierte polyhydroxyfunktionelle Polymere oder Copolymere von Dienen.

Diese genannten Polyole weisen bevorzugt ein mittleres Molekulargewicht von 250 - 30'000 g/mol, insbesondere von 400 - 20'000 g/mol, und weisen bevorzugt eine mittlere OH-Funktionalität im Bereich von 1.6 bis 3 auf.

Zusätzlich zu diesen genannten Polyolen können kleine Mengen von niedrigmolekularen zwei- oder mehrwertigen Alkoholen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, hydriertes Bisphenol A, dimere Fettalkohole wie beispielsweise Dimerfettsäurediole, 1,1,1-Trimethylolethan, 1,1,1-Trimethylolpropan, Glycerin, Pentaerythrit, niedrigmolekulare Alkoxylierungsprodukte der vorgenannten zwei- und mehrwertigen Alkohole, sowie Mischungen der vorgenannten Alkohole bei der Herstellung eines Polyurethanpolymers **PUP** mitverwendet werden.

Als Polyisocyanate für die Herstellung eines Polyurethanpolymers **PUP** können aliphatische, cycloaliphatische oder aromatische Polyisocyanate, insbesondere Diisocyanate, verwendet werden, beispielsweise die folgenden:
- monomere aliphatische Diisocyanate wie beispielsweise 1,6-Hexamethylendiisocyanat (HDI), 2-Methylpentamethylen-1,5-diisocyanat, 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat und beliebige Gemische dieser Isomeren, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin.
- monomere aromatische Diisocyanate wie beispielsweise 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI).
- Oligomere und Polymere der vorgenannten monomeren aliphatischen oder aromatischen Diisocyanate.
- Beliebige Mischungen der vorgenannten Polyisocyanate.
Bevorzugt sind monomere Diisocyanate, insbesondere MDI, TDI, HDI und IPDI.

Die Herstellung eines Polyurethanpolymers **PUP** erfolgt in bekannter Art und Weise direkt aus den Polyisocyanaten und den Polyolen, oder durch schrittweise Adduktionsverfahren, wie sie auch als Kettenverlängerungsreaktionen bekannt sind.

In einer bevorzugten Ausführungsform wird das Polyurethanpolymer **PUP** über eine Reaktion von mindestens einem Polyisocyanat und mindestens einem Polyol hergestellt, wobei die Isocyanatgruppen gegenüber den Hydroxylgruppen im stöchiometrischen Überschuss vorliegen. Vorteilhaft beträgt das Verhältnis zwischen Isocyanat- und Hydroxylgruppen 1.3 bis 5, insbesondere 1.5 bis 3.

Vorteilhaft wird die Umsetzung bei einer Temperatur durchgeführt, bei der die verwendeten Polyole, Polyisocyanate und das gebildete Polyurethanpolymer flüssig vorliegen.

Das Polyurethanpolymer **PUP** weist ein Molekulargewicht von vorzugsweise über 500 g/mol, insbesondere ein solches zwischen 1000 und 50'000 g/mol, bevorzugt ein solches zwischen 2000 und 30'000 g/mol, auf.

Weiterhin weist das Polyurethanpolymer **PUP** bevorzugt eine mittlere Funktionalität im Bereich von 1.8 bis 3 auf.

Ein zur Umsetzung mit einem Aldimin der Formel (Ia) geeignetes Polyisocyanat der Formel (XI) ist in einer weiteren Ausführungsform ein Polyisocyanat **PI** in der Form eines monomeren Diisocyanates oder eines Oligomeren eines monomeren Diisocyanates oder eines Derivates eines monomeren Diisocyanates, wobei dafür dieselben monomeren Diisocyanate geeignet sind, welche als zur Herstellung eines Polyurethanpolymers **PUP** geeignet genannt wurden.

Als Polyisocyanat **PI** eignen sich vor allem Oligomere oder Derivate von monomeren Diisocyanaten, insbesondere von HDI, IPDI, TDI und MDI. Kommerziell erhältliche Typen sind insbesondere HDI-Biurete, beispielsweise als Desmodur® N 100 und N 3200 (Bayer), Tolonate® HDB und HDB-LV (Rhodia) und Duranate® 24A-100 (Asahi Kasei); HDI-Isocyanurate, beispielsweise als Desmodur® N 3300, N 3600 und N 3790 BA (alle von Bayer), Tolonate® HDT, HDT-LV und HDT-LV2 (Rhodia), Duranate® TPA-100 und THA-100 (Asahi Kasei) und Coronate® HX (Nippon Polyurethane); HDI-Uretdione, beispielsweise als Desmodur® N 3400 (Bayer); HDI-Iminooxadiazindione, beispielsweise als Desmodur® XP 2410 (Bayer); HDI-Allophanate, beispielsweise als Desmodur® VP LS 2102 (Bayer); IPDI-Isocyanurate, beispielsweise in Lösung als Desmodur® Z 4470 (Bayer) oder in fester Form als Vestanat® T1890/100 (Degussa); TDI-Oligomere, beispielsweise als Desmodur® IL (Bayer); sowie gemischte Isocyanurate auf Basis TDI / HDI, zum Beispiel als Desmodur® HL (Bayer). Weiterhin besonders geeeignet sind bei Raumtemperatur flüssige Formen von MDI (sogenanntes "modifiziertes MDI"), welche Gemische von MDI mit MDI-Derivaten, wie beispielsweise MDI-Carbodiimiden bzw. MDI-Uretoniminen oder MDI-Urethanen darstellen, bekannt beispielsweise unter Handelsnamen wie Desmodur® CD, Desmodur® PF, Desmodur® PC (alle von Bayer), sowie Gemische aus MDI und MDI-Homologen (polymeres MDI oder PMDI), erhältlich unter Handelsnamen wie Desmodur® VL, VL50, VL R10, VL R20 und Desmodur® VKS 20F (alle von Bayer), Isonate® M 309, Voranate® M 229, Voranate M® 580 (alle von Dow) oder Lupranat® M 10 R (von BASF).

Als Polyisocyanat **PI** bevorzugt sind die Oligomere von HDI und/oder IPDI, insbesondere die Isocyanurate.

Die vorgenannten oligomeren Polyisocyanate **PI** stellen üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen diese Oligomere einen niedrigen Gehalt an monomeren Diisocyanaten auf.

Ebenfalls möglich ist, dass ein zur Umsetzung mit einem Aldimin der Formel (Ia) geeignetes Polyisocyanat der Formel (XI) eine Mischung bestehend aus mindestens einem Polyurethanpolymer **PUP** und mindestens einem Polyisocyanat **PI** ist.

Zur Herstellung einer Aldiminogruppen-haltigen Verbindung **AV1** der Formel (X) wird mindestens ein Polyisocyanat der Formel (XI) mit mindestens einem Aldimin der Formel (Ia) umgesetzt. Dafür geeignet sind die vorgängig beschriebenen Aldimine der Formel (Ia), respektive die dafür bevorzugten Ausführungsformen, wie sie bereits vorgängig im Detail beschrieben wurden.

Die Umsetzung von einem Polyisocyanat der Formel (XI) mit einem Aldimin der Formel (Ia) zu einer Aldiminogruppen-haltigen Verbindung **AV1** der Formel (X) erfolgt unter Bedingungen, wie sie für Reaktionen zwischen den an der jeweiligen Umsetzung beteiligten Reaktivgruppen typischerweise verwendet werden und wie sie vorgängig beschrieben wurden.

Wird diese Additionsreaktion stöchiometrisch durchgeführt, das heisst mit einem Molequivalent aktivem Wasserstoff des Aldimins der Formel (Ia) auf ein Molequivalent Isocyanatgruppen des Polyisocyanats der Formel (XI) - wodurch dessen Reaktivgruppen vollständig umgesetzt werden -, wird als Additionsprodukt eine Aldiminogruppen-haltige Verbindung **AV1** der Formel (X) mit dem Index u gleich null erhalten. Eine solche Verbindung **AV1** ist ein Polyaldimin.

Wird diese Additionsreaktion hingegen unterstöchiometrisch geführt, das heisst mit weniger als einem Molequivalent aktivem Wasserstoff des Aldimins der Formel (Ia) auf ein Molequivalent Isocyanatgruppen des Polyisocyanats der Formel (XI) - wodurch die Isocyanatgruppen nur teilweise umgesetzt werden --, wird als Additionsprodukt eine heterofunktionelle Verbindung erhalten, das heisst eine Aldiminogruppen-haltige Verbindung **AV1** der Formel (X), die neben einer oder mehreren Aldiminogruppen noch mindestens eine Isocyanatgruppe aufweist. Der Index u in der Formel (X) ist in diesem Fall grösser als null.

Eine Aldiminogruppen-haltige Verbindung **AV1** der Formel (X), welche Isocyanatgruppen aufweist - welche also einen Index u in der Formel (X) grösser als null aufweist -, wird aufgrund der Lagerstabilität bevorzugt ausgehend von einem Polyisocyanat der Formel (XI) hergestellt, welches ausschliesslich aliphatische Isocyanatgruppen aufweist.

Die Aldiminogruppen-haltigen Verbindungen **AV1** der Formel (X) sind unter geeigneten Bedingungen, insbesondere unter Ausschluss von Feuchtigkeit, lagerstabil.

Bei der unterstöchiometrischen Umsetzung eines Polyurethanpolymers **PUP** mit einem Aldimin der Formel (Ia) reagiert die Gruppe HX bevorzugt mit den im Polyurethanpolymer **PUP** üblicherweise vorhandenen monomeren Diisocyanaten. Auf diese Weise wird der Gehalt an monomeren Diisocyanaten im Polyurethanpolymer **PUP** stark reduziert.

Eine weitere bevorzugte Ausführungsform einer Aldiminogruppen-haltigen Verbindung **AV** ist eine Aldiminogruppen-haltige Verbindung **AV2** der Formel (XII), 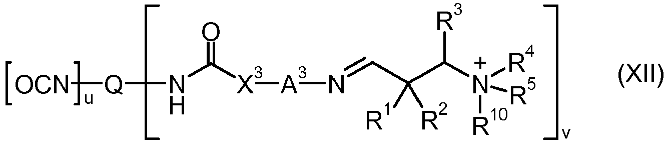 wobei
A³ keinen aktiven Wasserstoff und keine primären Aminogruppen aufweist und entweder
   für einen zweiwertigen Kohlenwasserstoffrest mit 2 bis 20 C-Atomen, welcher gegebenenfalls mindestens ein Heteroatom, insbesondere in der Form von Ether-Sauerstoff oder tertiärem Amin-Stickstoff, enthält, steht,
      oder
   zusammen mit R¹² für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
X³ für O oder S oder N-R¹¹ oder N-R¹² steht,
   wobei R¹² zusammen mit A³ für einen dreiwertigen Kohlenwasserstoffrest mit 3 bis 20 C-Atomen, welcher gegebenenfalls mindestens ein Heteroatom, insbesondere in der Form von Ether-Sauerstoff oder tertiärem Amin-Stickstoff, enthält, steht;
und Q, u, v, R¹, R², R³, R⁴, R⁵, R¹⁰ und R¹¹ die bereits genannten Bedeutungen aufweisen.

Eine Aldiminogruppen-haltige Verbindung **AV2** der Formel (XII) ist in einer Ausführungsform erhältlich ausgehend von einer Aldiminogruppen-haltigen Verbindung **AV1** der Formel (X), welche protoniert oder alkyliert wird. Zur Protonierung, beziehungsweise Alkylierung, können die gleichen Brønsted-Säuren, beziehungsweise Alkylierungsmittel, eingesetzt werden, wie sie zur Herstellung von Aldiminen der Formel (IX) bereits beschrieben wurden.

In einer weiteren Ausführungsform ist eine Aldiminogruppen-haltige Verbindung **AV2** der Formel (XII) erhältlich ausgehend von einem Aldimin der Formel (IX) mit m=1 und n=1, welches mit einem Polyisocyanat der bereits genannten Formel (XI) umgesetzt wird.

Die auf die beschriebene Weise erhaltenen Aldiminogruppen-haltigen Verbindungen **AV** sind, wie die Aldimine der Formel (I), nahezu oder ganz geruchsfrei. Sie sind unter geeigneten Bedingungen, insbesondere unter Ausschluss von Feuchtigkeit, lagerstabil. Heterofunktionelle Aldiminogruppen-haltige Verbindungen **AV**, die neben Aldiminogruppen zusätzliche für Polyreaktionen zugängliche Reaktivgruppen enthalten, sind dann lagerstabil, wenn sie von Reaktionen dieser Reaktivgruppen auslösenden Faktoren, wie beispielsweise Hitze oder UV-Strahlung, ferngehalten werden.

Bei Zutritt von Feuchtigkeit können die Aldiminogruppen der Aldiminogruppen-haltigen Verbindungen **AV** über Zwischenstufen formal zu Aminogruppen hydrolysieren, wobei der entsprechende, zur Herstellung des Aldimins verwendete Aldehyd **ALD** der Formel (IV) freigesetzt wird, welcher ebenfalls nahezu oder ganz geruchsfrei ist. Da diese Hydrolysereaktion reversibel ist und das chemische Gleichgewicht deutlich auf der Aldiminseite liegt, ist davon auszugehen, dass in Abwesenheit von gegenüber Aminen reaktiven Gruppen nur ein Teil der Aldiminogruppen teilweise oder vollständig hydrolysieren. Im speziellen Fall von heterofunktionellen Aldiminogruppen-haltigen Verbindungen **AV**, die gegenüber Aminen reaktive Gruppen, insbesondere Isocyanatgruppen, enthalten, reagieren die hydrolysierenden Aldiminogruppen hingegen weiter, beispielsweise mit Isocyanatgruppen zu Harnstoffgruppen. In diesem Fall kommt es zur Vernetzung der heterofunktionellen Aldiminogruppen-haltigen Verbindung **AV**, welche auch ohne Beteiligung weiterer Substanzen direkt zu einer ausgehärteten Polymerzusammensetzung führen kann. Die Reaktion der gegenüber Aminen reaktiven Gruppen mit den hydrolysierenden Aldiminogruppen muss dabei nicht notwendigerweise über Aminogruppen erfolgen. Selbstverständlich sind auch Reaktionen mit Zwischenstufen der Hydrolysereaktion möglich. Beispielsweise ist es denkbar, dass die hydrolysierende Aldiminogruppe in der Form eines Halbaminals direkt mit den gegenüber Aminen reaktiven Gruppen reagiert.

Die Aldiminogruppen-haltigen Verbindungen **AV** können beispielsweise verwendet werden als Quelle für kationische Aldiminogruppen-haltige Verbindungen, indem die tertiären Aminogruppen teilweise oder ganz zu Ammoniumgruppen protoniert oder zu quaternären Ammoniumgruppen alkyliert werden.

Ein weiterer Gegenstand der Erfindung sind härtbare Zusammensetzungen, im Folgenden auch Polyurethanzusammensetzungen genannt, welche mindestens ein Polyisocyanat sowie entweder mindestens ein Aldimin der Formel (I) oder der Formel (IX) oder mindestens eine Aldiminogruppen-haltige Verbindung **AV** enthalten.

Der Begriff "Polyisocyanat" umfasst im vorliegenden Dokument Verbindungen mit zwei oder mehr Isocyanatgruppen, unabhängig davon, ob es sich dabei um monomere Diisocyanate, oligomere Polyisocyanate oder Isocyanatgruppen aufweisende Polymere mit einem relativ hohen Molekulargewicht handelt.

Als Aldimin der Formel (I) geeignet sind die vorgängig detailliert beschriebenen Aldimine der Formel (I), respektive die bevorzugten Ausführungsformen davon, insbesondere die Aldimine der Formel (Ia) oder die Aldimine der Formel (Ib). Geeignete Aldimine der Formel (IX) wurden vorgängig bereits beschrieben. Als Aldiminogruppen-haltige Verbindung **AV** geeignet sind die vorgängig detailliert beschriebenen Aldiminogruppen-haltigen Verbindungen **AV**, respektive die bevorzugten Ausführungsformen davon, insbesondere die Aldiminogruppen-haltigen Verbindungen **AV1** der Formel (X). Als Aldiminogruppen-haltige Verbindung **AV1** besonders geeignet sind solche, bei denen der Index u für null steht, also Polyaldimine.

Aldimine der Formel (I) sind weiterhin besonders geeignet solche, welche ausgehend von Aldehyden **ALD** mit relativ niedriger Basizität erhältlich sind. Diese sind ihrerseits Produkte von Aminen **C** mit relativ niedriger Basizität, wie insbesondere Morpholine und N-Alkyl-benzylamine. Aldehyde **ALD** mit relativ niedriger Basizität sind insbesondere 2,2-Dimethyl-3-(N-morpholino)-propanal, 2,2-Dimethyl-3-(N-(2,6-dimethyl)morpholino)-propanal, 2,2-Dimethyl-3-(N-benzylmethylamino)-propanal und 2,2-Dimethyl-3-(N-benzyl-isopropylamino)-propanal.

Bevorzugt sind härtbare Zusammensetzungen enthaltend mindestens ein Polyisocyanat und entweder mindestens ein Aldimin der Formel (I) oder mindestens eine Aldiminogruppen-haltige Verbindung **AV.**

In einer Ausführungsform ist die härtbare Zusammensetzung einkomponentig.

Als "einkomponentig" wird im vorliegenden Dokument eine härtbare Zusammensetzung bezeichnet, bei welcher alle Bestandteile der Zusammensetzung vermischt im gleichen Gebinde gelagert werden, und welche bei Raumtemperatur über einen längeren Zeitraum lagerstabil ist, sich also in ihren Anwendungs- oder Gebrauchseigenschaften durch die Lagerung nicht oder nur unwesentlich verändert, und welche nach der Applikation durch die Einwirkung von Feuchtigkeit und / oder Wärme aushärtet.

Die einkomponentige härtbare Zusammensetzung umfasst mindestens ein Polyisocyanat. Dessen Isocyanatgruppen können in der Zusammensetzung entweder als freie Isocyanatgruppen oder als blockierte Isocyanatgruppen oder in gemischter Form vorliegen.

Unter einer "blockierten Isocyanatgruppe" wird im vorliegenden Dokument eine Isocyanatgruppe verstanden, welche durch die vorgängige Umsetzung einer freien Isocyanatgruppe mit einem aus dem Stand der Technik bekannten Blockierungsmittel, beispielsweise einem Phenol, einem Ketoxim, einem Pyrazol oder einem Malonsäurediester, in ihrer Reaktivität gegenüber Nucloephilen so stark reduziert ist, dass sie zusammen mit geeigneten Härtern bei Raumtemperatur lagerstabil ist und erst unter der Einwirkung von Hitze und / oder Feuchtigkeit mit diesen Härtern zu reagieren beginnt, wobei das Blockierungsmittel je nach Typ freigesetzt oder nicht freigesetzt wird.

Die einkomponentige härtbare Zusammensetzung kann feuchtigkeitshärtend und / oder hitzehärtend sein.

Unter einer "hitzehärtenden Zusammensetzung" wird im vorliegenden Dokument eine Zusammensetzung mit blockierten Isocyanatgruppen verstanden, bei welcher beim Erhitzen auf eine geeignete Temperatur, typischerweise im Bereich von 120 bis 200 °C, in speziellen Fällen schon bei Temperaturen ab 80 °C, die Isocyanatgruppen soweit aktiviert werden, dass mit geeigneten Härtern eine Vernetzung und somit Aushärtung eintritt. Dieser Vorgang wird auch als Einbrennen bezeichnet und erfolgt üblicherweise nach der Applikation der Zusammensetzung.

Bevorzugt liegen die Isocyanatgruppen des Polyisocyanates in der einkomponentigen härtbaren Zusammensetzung als freie Isocyanatgruppen vor, insbesondere als freie aliphatische Isocyanatgruppen.

In der einkomponentigen härtbaren Zusammensetzung beträgt das Verhältnis zwischen Aldiminogruppen und Isocyanatgruppen insbesondere 0.1 bis 1.1, bevorzugt 0.3 bis 0.9, besonders bevorzugt 0.4 bis 0.8, Equivalent Aldiminogruppen pro Equivalent Isocyanatgruppen, wobei die Isocyanatgruppen entweder in freier oder blockierter Form vorliegen können.

Als einkomponentige härtbare Zusammensetzung bevorzugt ist eine einkomponentige feuchtigkeitshärtende Zusammensetzung, welche mindestens ein aliphatische Isocyanatgruppen aufweisendes Polyisocyanat **P1** und entweder mindestens ein Aldimin der Formel (I) oder mindestens eine Aldiminogruppen-haltige Verbindung **AV** umfasst.

Als Polyisocyanat **P1** geeignet sind einerseits aliphatische Isocyanatgruppen aufweisende Polyurethanpolymere **PUP1.** Ein Polyurethanpolymer **PUP1** ist auf dieselbe Weise aus Polyolen und Polyisocyanaten erhältlich, wie vorgängig für das Polyurethanpolymer **PUP** beschrieben. Als Polyole sind die bereits erwähnten Polyole geeignet, und als Polyisocyanate sind die bereits erwähnten aliphatischen Polyisocyanate geeignet, wobei monomere aliphatische Diisocyanate bevorzugt sind, insbesondere IPDI, HDI, TMDI und HMDI.

Als Polyisocyanat **P1** geeignet sind andererseits Polyisocyanate **PI1** in der Form eines monomeren aliphatischen Diisocyanates oder eines Oligomeren davon, wie sie bereits vorgängig erwähnt wurden, insbesondere ein Oligomer von HDI oder IPDI.

Als Polyisocyanat **P1** weiterhin geeignet sind Gemische enthaltend mindestens ein Polyurethanpolymer **PUP1** und mindestens ein Polyisocyanat **PI1**.

Die einkomponentige feuchtigkeitshärtende Zusammensetzung umfasst neben mindestens einem Polyisocyanat **P1** weiterhin entweder mindestens ein Aldimin der Formel (I) oder mindestens eine Aldiminogruppen-haltige Verbindung **AV.** Als Aldimin der Formel (I) bevorzugt sind die Aldimine der Formel (Ib), und als Aldiminogruppen-haltige Verbindung **AV** bevorzugt sind Aldiminogruppen-haltige Verbindungen **AV1** der Formel (X). Eine Aldiminogruppen-haltige Verbindung **AV1** der Formel (X) kann in der Zusammensetzung auch in situ gebildet werden, indem ein Aldimin der Formel (Ia) in geeigneter unterstöchiometrischer Menge zu einer Zusammensetzung enthaltend mindestens ein Polyisocyanat **P1** gegeben wird, wobei die Aldiminogruppen-haltige Verbindung **AV1** in der vorgängig beschriebenen Weise gebildet wird. Dabei wird ein Teil des Polyisocyanates **P1** als Polyisocyanat der Formel (XI) in die Aldiminogruppen-haltige Verbindung **AV1** eingebaut.

Gegebenenfalls enthält die einkomponentige feuchtigkeitshärtende Zusammensetzung weitere Bestandteile, insbesondere die in Polyurethanzusammensetzungen üblicherweise eingesetzten Hilfs- und Zusatzstoffe, beispielsweise die folgenden:
- Weichmacher, beispielsweise Carbonsäureester wie Phthalate, beispielsweise Dioctylphthalat, Diisononylphthalat oder Diisodecylphthalat, Adipate, beispielsweise Dioctyladipat, Azelate und Sebacate, organische Phosphor- und Sulfonsäureester oder Polybutene;
- nicht-reaktive thermoplastische Polymere, wie beispielsweise Homo- oder Copolymere von ungesättigten Monomeren, insbesondere aus der Gruppe umfassend Ethylen, Propylen, Butylen, Isobutylen, Isopren, Vinylacetat und Alkyl(meth)acrylate, insbesondere Polyethylene (PE), Polypropylene (PP), Polyisobutylene, Ethylenvinylacetat-Copolymere (EVA) und ataktische Poly-α-Olefine (APAO);
- Lösemittel;
- anorganische und organische Füllstoffe, zum Beispiel gemahlene oder gefällte Calciumcarbonate, welche gegebenenfalls mit Fettsäuren, insbesondere Stearaten, beschichtet sind, Baryt (BaSO₄, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln;
- Fasern, beispielsweise aus Polyethylen;
- Pigmente, beispielsweise Titandioxid oder Eisenoxide;
- Katalysatoren, welche die Hydrolyse der Aldiminogruppen beschleunigen, insbesondere Säuren, beispielsweise organische Carbonsäuren wie Benzoesäure, Salicylsäure oder 2-Nitrobenzoesäure, organische Carbonsäureanhydride wie Phthalsäureanhydrid, Hexahydrophthalsäureanhydrid und Hexahydromethylphthalsäureanhydrid, Silylester von organischen Carbonsäuren, organische Sulfonsäuren wie Methansulfonsäure, p-Toluolsulfonsäure oder 4-Dodecylbenzolsulfonsäure, Sulfonsäureester, andere organische oder anorganische Säuren, oder Mischungen der vorgenannten Säuren und Säureester;
- Katalysatoren, welche die Reaktion der Isocyanatgruppen beschleunigen, beispielsweise Organozinnverbindungen wie Dibutylzinndiacetat, Dibutylzinndilaurat, Dibutylzinndichlorid, Dibutylzinndiacetylacetonat und Dioctylzinndilaurat, Bismutverbindungen wie Bismuttrioctoat und Bismuttris(neodecanoat), und tertiäre Aminogruppen enthaltende Verbindungen wie 2,2'-Dimorpholinodiethylether und 1,4-Diazabicyclo[2.2.2]octan;
- Rheologie-Modifizierer wie beispielsweise Verdickungsmittel oder Thixotropiermittel, zum Beispiel Harnstoffverbindungen, Polyamidwachse, Bentonite oder pyrogene Kieselsäuren;
- Reaktivverdünner und Vernetzer, beispielsweise monomere Diisocyanate wie MDI, PMDI, TDI, HDI, 1,12-Dodecamethylendiisocyanat, Cyclohexan-1,3- oder 1,4-diisocyanat, IPDI, Perhydro-2,4'- und -4,4'-diphenylmethandiisocyanat, 1,3- und 1,4-Tetramethylxylylendiisocyanat, sowie Oligomere und Derivate dieser Polyisocyanate, insbesondere in Form von Isocyanuraten, Carbodiimiden, Uretoniminen, Biureten, Allophanaten oder Iminooxadiazindionen, Addukte monomerer Polyisocyanate mit kurzkettigen Polyolen, sowie Adipinsäuredihydrazid und andere Dihydrazide, sowie Polyisocyanate mit blockierten aromatischen Isocyanatgruppen, wie beispielsweise die Desmocap®-Typen 11, 12 und XP 2540 (alle von Bayer) und die Trixene®-Typen BI 7641, BI 7642, BI 7770, BI 7771, BI 7772, BI 7774 und BI 7779 (alle von Baxenden);
- blockierte Amine, beispielsweise in Form von Ketiminen, Oxazolidinen, Enaminen oder anderen Aldiminen;
- Trocknungsmittel, wie beispielsweise Molekularsiebe, Calciumoxid, hochreaktive Isocyanate wie p-Tosylisocyanat, Orthoameisensäureester, Alkoxysilane wie Tetraethoxysilan, Organoalkoxysilane wie Vinyltrimethoxysilan, und Organoalkoxysilane, welche in α-Stellung zur Silangruppe eine funktionelle Gruppe aufweisen;
- Haftvermittler, insbesondere Organoalkoxysilane ("Silane") wie beispielsweise Epoxysilane, Vinylsilane, (Meth)acrylsilane, Isocyanatosilane, Carbamatosilane, Alkylsilane, S-(Alkylcarbonyl)-mercaptosilane und Aldiminosilane, sowie oligomere Formen dieser Silane;
- Stabilisatoren gegen Wärme, Licht- und UV-Strahlung;
- flammhemmende Substanzen;
- oberflächenaktive Substanzen wie beispielsweise Netzmittel, Verlaufsmittel, Entlüftungsmittel oder Entschäumer;
- Biozide wie beispielsweise Algizide, Fungizide oder das Pilzwachstum hemmende Substanzen.

Es ist vorteilhaft, beim Einsatz solcher weiteren Bestandteile darauf zu achten, dass diese die Lagerstabilität der Zusammensetzung nicht stark beeinträchtigen. Das heisst, dass diese Bestandteile während der Lagerung die zur Vernetzung führenden Reaktionen, wie Hydrolyse der Aldiminogruppen oder Vernetzung der Isocyanatgruppen, nicht in signifikantem Ausmass auslösen dürfen. Insbesondere bedeutet dies, dass all diese Bestandteile kein oder höchstens Spuren von Wasser enthalten sollten. Es kann sinnvoll sein, gewisse Bestandteile vor dem Einmischen in die Zusammensetzung chemisch oder physikalisch zu trocknen.

Bevorzugt enthält die einkomponentige feuchtigkeitshärtende Zusammensetzung mindestens einen Katalysator. Der Katalysator ist insbesondere eine der genannten Säuren, wie Benzoesäure oder Salicylsäure, oder eine der genannten Metallverbindungen, oder eines der genannten tertiären Amine. Es kann durchaus vorteilhaft sein, unterschiedliche Katalysatoren, bzw. unterschiedliche Katalysatorenarten, einzusetzen.

Die einkomponentige feuchtigkeitshärtende Zusammensetzung wird unter Ausschluss von Feuchtigkeit hergestellt und aufbewahrt. Sie ist lagerstabil, d.h. sie kann unter Ausschluss von Feuchtigkeit in einer geeigneten Verpackung oder Anordnung, wie beispielsweise einem Fass, einem Eimer, einem Beutel, einer Kartusche oder einer Flasche über einen Zeitraum von beispielsweise mehreren Monaten aufbewahrt werden, ohne dass sie sich in ihren Anwendungseigenschaften oder in ihren Eigenschaften nach der Aushärtung in einem für ihren Gebrauch relevanten Ausmass verändert. Je nach Konsistenz der Zusammensetzung ist es üblich, die Lagerstabilität über die Messung der Viskosität zu ermitteln.

Die Aldiminogruppen des Aldimins der Formel (I) und / oder der Aldiminogruppen-haltigen Verbindung **AV** haben die Eigenschaft, bei Kontakt mit Feuchtigkeit zu hydrolysieren. Die dabei formal frei werdenden primären Aminogruppen reagieren dabei mit den in der Zusammensetzung vorhandenen Isocyanatgruppen zu Harnstoffgruppen, und es wird der entsprechende Aldehyd **ALD** der Formel (IV) freigesetzt. Im Verhältnis zu den Aldiminogruppen überschüssige Isocyanatgruppen reagieren direkt mit Feuchtigkeit und bilden ebenfalls Harnstoffgruppen. Gegebenenfalls vorhandene blockierte Isocyanatgruppen reagieren im Allgemeinen unter Freisetzung des Blockierungsmittels ebenfalls zu Harnstoffgruppen, wobei diese Reaktion gegebenenfalls erst unter Einwirkung von Hitze abläuft. Als Ergebnis dieser Reaktionen härtet die Zusammensetzung zu einem festen Material aus; dieser Prozess wird auch als Vernetzung bezeichnet. Die Reaktion der Isocyanatgruppen mit dem hydrolysierenden Aldimin muss dabei nicht notwendigerweise über freie Aminogruppen erfolgen. Selbstverständlich sind auch Reaktionen mit Zwischenstufen der Hydrolysereaktion möglich. Beispielsweise ist es denkbar, dass eine hydrolysierende Aldiminogruppe in der Form eines Halbaminals direkt mit einer Isocyanatgruppe reagiert.

Das für die Aushärtungsreaktion benötigte Wasser kann entweder aus der Luft stammen (Luftfeuchtigkeit), oder aber die Zusammensetzung kann mit einer Wasser enthaltenden Komponente in Kontakt gebracht werden, zum Beispiel durch Besprühen, oder es kann der Zusammensetzung bei der Applikation eine Wasser enthaltende Komponente zugesetzt werden.

Die einkomponentige feuchtigkeitshärtende Zusammensetzung härtet bei Kontakt mit Feuchtigkeit im Allgemeinen ohne die Bildung von Blasen aus. Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge zugesetzten Wassers beeinflussen.

In einer weiteren Ausführungsform ist die härtbare Zusammensetzung zweikomponentig und enthält mindestens ein Polyisocyanat **P2** und entweder mindestens ein Aldimin der Formel (I) oder mindestens eine Aldiminogruppen-haltige Verbindung **AV.**

Als "zweikomponentig" wird im vorliegenden Dokument eine härtbare Zusammensetzung bezeichnet, bei welcher die Bestandteile der Zusammensetzung in zwei verschiedenen Komponenten vorliegen, welche in voneinander getrennten Gebinden gelagert werden und welche jeweils für sich lagerstabil sind. Die beiden Komponenten werden als Komponente **K1** und als Komponente **K2** bezeichnet. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit und / oder Wärme abläuft oder vervollständigt wird.

Das Aldimin der Formel (I) oder die Aldiminogruppen-haltige Verbindung **AV** kann entweder ein Bestandteil der Komponente **K1**, oder ein Bestandteil der Komponente **K2**, oder ein Bestandteil von beiden Komponenten **K1** und **K2** sein.

Die Komponente **K1** der zweikomponentigen Polyurethanzusammensetzung enthält als Polyisocyanat mindestens ein Polyisocyanat **P2.**

Als Polyisocyanat **P2** geeignet ist ein Polyisocyanat **PI2** in der Form eines monomeren Diisocyanates oder eines Oligomeren eines monomeren Diisocyanates oder eines Derivates eines monomeren Diisocyanates. Als Polyisocyanat **PI2** sind die vorgängig erwähnten Polyisocyanate **PI** geeignet, wobei technische Formen von oligomerem IPDI, HDI und TDI, sowie insbesondere PMDI und bei Raumtemperatur flüssige Formen von MDI bevorzugt sind.

Als Polyisocyanat **P2** weiterhin geeignet ist ein Polyurethanpolymer **PUP**, wie es vorgängig beschrieben wurde.

Als Polyisocyanat **P2** geeignet sind schliesslich auch Gemische aus Polyurethanpolymeren **PUP** und Polyisocyanaten **PI2**, insbesondere Gemische aus mindestens einem MDI-basierten Polyurethanpolymer **PUP** und mindestens einem monomeren und/oder polymeren MDI.

Neben dem Polyisocyanat **P2** kann die Komponente **K1** mindestens ein Aldimin der Formel (I) oder mindestens eine Aldiminogruppen-haltige Verbindung **AV** enthalten. In diesem Fall weist das Polyisocyanat **P2** bevorzugt aliphatische Isocyanatgruppen auf. Bevorzugt enthält die Komponente **K1** aber weder ein Aldimin der Formel (I) noch eine Aldiminogruppen-haltige Verbindung **AV.**

Die Komponente **K2** der zweikomponentigen Polyurethanzusammensetzung enthält mindestens einen gegenüber Isocyanatgruppen reaktiven Bestandteil, insbesondere ausgewählt aus der Gruppe umfassend Wasser, Polyamine, Polyole, Aminoalkohole, Polythiole oder blockierte Amine. Bevorzugt enthält die Komponente **K2** mindestens ein blockiertes Amin in der Form mindestens eines Aldimins der Formel (I) oder in der Form mindestens einer Aldiminogruppen-haltigen Verbindung **AV.** Dafür geeignet sind die vorgängig detailliert beschriebenen Aldimine der Formel (I), respektive die bevorzugen Ausführungsformen davon, insbesondere Aldimine der Formel (I) mit der Summe der Indizes m+n von 2 oder 3, sowie Aldiminogruppen-haltige Verbindungen **AV**, welche frei sind von Isocyanatgruppen und eine Funktionalität in Bezug auf die Umsetzung mit Isocyanatgruppen von 2 oder 3 aufweisen. Besonders bevorzugt sind Aldimine der Formeln (Ia) und (Ib), sowie Aldiminogruppen-haltige Verbindungen **AV1** der Formel (X) mit dem Index u = 0 und dem Index v = 2 oder 3.

Als Polyamine in der Komponente **K2** geeignet sind primäre aliphatische Polyamine, wie sie als Amine **B2** der Formel (IIIb) bereits beschrieben wurden; sekundäre aliphatische Polyamine wie beispielsweise N,N'-Dibutyl-ethylendiamin; N,N'-Di-tert.butyl-ethylendiamin, N,N'-Diethyl-1,6-hexandiamin, 1-(1-Methylethyl-amino)-3-(1-methylethyl-aminomethyl)-3,5,5-trimethylcyclohexan (Jefflink® 754 von Huntsman), N4-Cyclohexyl-2-methyl-N2-(2-methylpropyl)-2,4-pentandiamin, N,N'-Dialkyl-1,3-xylylendiamin, Bis-(4-(N-alkylamino)-cyclohexyl)-methan, N-alkylierte Polyetheramine, beispielsweise die Jeffamine®-Typen SD-231, SD-401, SD-404 und SD-2001 (alle von Huntsman), Produkte aus der Michael-artigen Addition der beispielhaft erwähnten primären aliphatischen Polyamine an Michaelakzeptoren wie Maleinsäurediester, Fumarsäurediester, Citraconsäurediester, Acrylsäureester, Methacrylsäureester, Zimtsäureester, Itaconsäurediester, Vinylphosphonsäurediester, Vinylsulfonsäurearylester, Vinylsulfone, Vinylnitrile, 1-Nitroethylene oder Knoevenagel-Kondensationsprodukte wie beispielsweise solche aus Malonsäurediestern und Aldehyden wie Formaldehyd, Acetaldehyd oder Benzaldehyd; aliphatische Polyamine mit primären und sekundären Aminogruppen, wie beispielsweise N-Butyl-1,6-hexandiamin; primäre und/oder sekundäre aromatische Polyamine wie beispielsweise m- und p-Phenylendiamin, 4,4'-Diaminodiphenylmethan (MDA), 3,3'-Dichloro-4,4'-diaminodiphenylmethan (MOCA), Mischungen von 3,5-Dimethylthio-2,4- und - 2,6-toluylendiamin (erhältlich als Ethacure® 300 von Albemarle), Mischungen von 3,5-Diethyl-2,4- und -2,6-toluylendiamin (DETDA), 3,3',5,5'-Tetraethyl-4,4'-diaminodiphenylmethan (M-DEA), 3,3',5,5'-Tetraethyl-2,2'-dichloro-4,4'-diaminodiphenylmethan (M-CDEA), 3,3'-Diisopropyl-5,5'-dimethyl-4,4'-diaminodiphenylmethan (M-MIPA), 3,3',5,5'-Tetraisopropyl-4,4'-diaminodiphenylmethan (M-DIPA), 4,4'-Diaminodiphenylsulfon (DDS), 4-Amino-N-(4-aminophenyl)-benzolsulfonamid, 5,5'-Methylendianthranilsäure, Dimethyl-(5,5'-Methylendianthranilat), 1,3-Propylen-bis-(4-aminobenzoat), 1,4-Butylen-bis-(4-aminobenzoat), Polytetramethylenoxid-bis-(4-aminobenzoat) (erhältlich als Versalink® von Air Products), 1,2-Bis-(2-aminophenylthio)-ethan, N,N'-Dialkyl-p-phenylendiamin, N,N'-Dialkyl-4,4'-diaminodiphenylmethan, 2-Methylpropyl-(4-chloro-3,5-diaminobenzoat) und tert.Butyl-(4-chloro-3,5-diaminobenzoat); sowie Polyamine mit mehr als drei Aminogruppen.

Als Polyole in der Komponente **K2** geeignet sind dieselben Polyole, wie sie bereits als geeignet zur Herstellung eines Polyurethanpolymers **PUP** erwähnt wurden, sowie diejenigen niedrigmolekularen zwei- oder mehrwertigen Alkohole, wie sie vorgängig als geeignet zum Mitverwenden bei der Herstellung eines Polyurethanpolymers **PUP** erwähnt wurden.

Als Aminoalkohole in der Komponente **K2** geeigent sind Verbindungen, welche mindestens eine primäre oder sekundäre Aminogruppe und mindestens eine Hydroxylgruppe aufweisen, wie beispielsweise die aliphatischen Hydroxyamine, welche bereits vorgängig als geeignete Amine **B1** zur Herstellung der Aldimine der Formel (I) erwähnt wurden, sowie weiterhin beispielsweise Diethanolamin, 2-(Methylamino)ethanol, 2-(Ethylamino)ethanol, 2-(Butylamino)ethanol und 2-(Cyclohexylamino)ethanol.

Als Polythiole in der Komponente **K2** geeignet sind beispielsweise unter dem Markennamen Thiokol® bekannte flüssige Mercapto-terminierte Polymere, beispielsweise die Typen LP-3, LP-33, LP-980, LP-23, LP-55, LP-56, LP-12, LP-31, LP-32 und LP-2 (Morton Thiokol; beispielsweise erhältlich von SPI Supplies, USA, oder von Toray Fine Chemicals, Japan), sowie Polyester von Thiocarbonsäuren, beispielsweise Pentaerythritoltetramercaptoacetat, Trimethylolpropantrimercaptoacetat, Glykoldimercaptoacetat, Pentaerythritoltetra-(3-mercaptopropionat), Trimethylolpropantri-(3-mercapto-propionat) und Glykoldi-(3-mercaptopropionat).

Neben den Aldiminen der Formel (I) und den Aldiminogruppen-haltigen Verbindungen **AV** können auch weitere blockierte Amine als Bestandteil der Komponente **K2** eingesetzt werden, insbesondere Ketimine, Oxazolidine, Enamine sowie andere Aldimine. Solche anderen Aldimine sind erhältlich ausgehend von anderen Aldehyden als den vorgängig genannten Aldehyden **ALD** der Formel (IV), wie beispielsweise Isobutyraldehyd oder den Produkten aus der Veresterung von Carbonsäuren, wie insbesondere Laurinsäure, mit 3-Hydroxypivalaldehyd. Ketimine sind beispielsweise erhältlich aus der Umsetzung der vorgängig beschriebenen Amine **B** der Formel (III) mit Ketonen. Geeignete Oxazolidine sind insbesondere Polyoxazolidine, wie zum Beispiel Härter OZ (Bayer). Geeignete Enamine sind beispielsweise erhältlich aus der Umsetzung von Aminen mit mehreren sekundären Aminogruppen mit aliphatischen oder cycloaliphatischen Aldehyden oder Ketonen, die am C-Atom in α-Stellung zur Carbonylgruppe mindestens ein Wasserstoffatom aufweisen.

Gegebenenfalls enthält die Komponente **K2** Wasser, insbesondere die zur Hydrolyse der Aldiminogruppen und anderen blockierten Aminogruppen benötigte Wassermenge, oder ein Teil davon.

Bevorzugt enthält die Komponente **K2** weiterhin mindestens einen Katalysator in Form einer metallorganischen Verbindung und / oder eines tertiären Amins und / oder einer Säure, insbesondere einer organischen Carbon- oder Sulfonsäure.

Die zweikomponentige Polyurethanzusammensetzung enthält gegebenenfalls weitere Bestandteile. Im Fall der Komponente **K1** sind dies insbesondere die Hilfs- und Zusatzstoffe, wie sie vorgängig für eine einkomponentige feuchtigkeitshärtende Zusammensetzung erwähnt wurden. Im Fall der Komponente **K2** sind neben diesen zusätzlich noch weitere Hilfs- und Zusatzstoffe möglich, welche zusammen mit freien Isocyanatgruppen nicht oder nur kurzzeitig lagerfähig sind. Insbesondere sind dies Katalysatoren wie:
Verbindungen von Zink, Mangan, Eisen, Chrom, Cobalt, Kupfer, Nickel, Molybdän, Blei, Cadmium, Quecksilber, Antimon, Vanadium, Titan, Zirconium oder Kalium, wie Zink(II)-acetat, Zink(II)-2-ethylhexanoat, Zink(II)-laurat, Zink(II)-oleat, Zink(II)-naphthenat, Zink(II)-acetylacetonat, Zink(II)-salicylat, Mangan(II)-2-ethylhexanoat, Eisen(III)-2-ethylhexanoat, Eisen(III)-acetylacetonat, Chrom(III)-2-ethylhexanoat, Cobalt(II)-naphthenat, Cobalt(II)-2-ethylhexanoat, Kupfer(II)-2-ethylhexanoat, Nickel(II)-naphthenat, Phenylquecksilber-neodecanoat, Blei(II)-acetat, Blei(II)-2-ethylhexanoat, Blei(II)-neodecanoat, Blei(II)-acetylacetonat, Aluminiumlactat, Aluminiumoleat, Aluminium(III)-acetylacetonat, Diisopropoxytitan-bis-(ethylacetoacetat), Dibutoxytitan-bis-(ethylacetoacetat), Dibutoxytitan-bis-(acetylacetonat), Kalium-acetat, Kaliumoctoat; tertiäre Amine wie Triethylamin, Tributylamin, N-Ethyl-diisopropylamin, N,N,N',N'-Tetramethyl-ethylendiamin, Pentamethyl-diethylentriamin und höhere Homologe davon, N,N,N',N'-Tetramethyl-propylendiamin, Pentamethyl-dipropylentriamin und höhere Homologe davon, N,N,N',N'-Tetramethyl-1,3-butandiamin, N,N,N',N'-Tetramethyl-1,6-hexandiamin, Bis-(dimethylamino)-methan, N,N-Dimethylbenzylamin, N,N-Dimethylcyclohexylamin, N-Methyl-dicyclohexylamin, N,N-Dimethyl-hexadecylamin, Bis-(N,N-diethylaminoethyl)-adipat, N,N-Dimethyl-2-phenylethylamin, Tris-(3-dimethylaminopropyl)-amin, 1,4-Diazabicyclo[2.2.2]octan (DABCO), 1,8-Diazabicyclo[5.4.0]undec-7-en (DBU), 1,5-Diazabicyclo[4.3.0]non-5-en (DBN), N-Methylmorpholin, N-Ethylmorpholin, N-Cocomorpholin, N,N'-Dimethylpiperazin, N-Methyl-N'-dimethylaminoethyl-piperazin, Bis-(dimethylaminoethyl)-piperazin, 1,3,5-Tris-(dimethylaminopropyl)-hexahydrotriazin, Bis-(2-dimethylaminoethyl)-ether; stickstoffaromatische Verbindungen wie 4-Dimethylamino-pyridin, N-Methylimidazol, N-Vinylimidazol oder 1,2-Dimethylimidazol; Amidine und Guanidine wie 1,1,3,3-Tetramethylguanidin; tertiäre Amine enthaltend aktive Wasserstoffatome, wie Triethanolamin, Triisopropanolamin, N-Methyldiethanolamin, N,N-Dimethylethanolamin, 3-(Dimethylamino)-propyl-diisopropanolamin, Bis-(3-(dimethylamino)-propyl)-isopropanolamin, Bis-(3-dimethylaminopropyl)amin, 3-(Dimethylamino)-propyl-harnstoff, Mannich-Basen von Phenolen wie 2,4,6-Tris-(dimethylaminomethyl)-phenol oder 2,4,6-Tris-(3-(dimethylamino)-propylaminomethyl)-phenol, Imidazole wie beispielsweise N-Hydroxypropylimidazol oder N-(3-Aminopropyl)-imidazol, sowie Alkoxylierungs- und Polyalkoxylierungsprodukte dieser Verbindungen, beispielsweise Dimethylaminoethoxyethanol; organische Ammoniumverbindungen wie Benzyltrimethylammoniumhydroxid oder alkoxylierte tertiäre Amine; sogenannte "delayed action"-Katalysatoren, welche Modifizierungen bekannter Metall- oder Aminkatalysatoren darstellen, wie Umsetzungsprodukte aus tertiären Aminen und Carbonsäuren oder Phenolen, beispielsweise aus 1,4-Diazabicyclo[2.2.2]octan oder DBU und Ameisensäure oder Essigsäure; sowie Kombinationen der genannten Verbindungen, insbesondere von Metallverbindungen und tertiären Aminen.

Die Herstellung der beiden Komponenten **K1** und **K2** erfolgt getrennt voneinander, für die Komponente **K1** unter Ausschluss von Feuchtigkeit. Die beiden Komponenten **K1** und **K2** sind getrennt voneinander lagerstabil, d.h. sie können jede in einer geeigneten Verpackung oder Anordnung, wie beispielsweise einem Fass, einem Beutel, einem Eimer, einer Kartusche oder einer Flasche, vor ihrer Anwendung während mehreren Monaten bis zu einem Jahr und länger aufbewahrt werden, ohne dass sie sich in ihren jeweiligen Eigenschaften in einem für ihren Gebrauch relevanten Ausmass verändern.

Zur Verwendung der zweikomponentigen Polyurethanzusammensetzung werden die beiden Komponenten **K1** und **K2** miteinander vermischt. Dabei ist darauf zu achten, dass das Mischungsverhältnis so gewählt wird, dass die gegenüber Isocyanatgruppen reaktiven Bestandteile in einem geeigneten Verhältnis zu den Isocyanatgruppen der Komponente **K1** stehen. Insbesondere beträgt das Verhältnis 0.1 bis 1.1, bevorzugt 0.5 bis 0.95, besonders bevorzugt 0.6 bis 0.9, Equivalent der Summe der vorhandenen Hydroxylgruppen, Aminogruppen, Mercaptogruppen und geschützten Aminogruppen pro Equivalent Isocyanatgruppen, wobei geschütze Aminogruppen in Form von Oxazolidinogruppen doppelt gerechnet werden. Bei der Aushärtung reagieren überschüssige Isocyanatgruppen mit Feuchtigkeit, insbesondere mit Luftfeuchtigkeit.

Das Vermischen der beiden Komponenten **K1** und **K2** erfolgt mit einem geeigneten Verfahren, beispielsweise über einen Statikmischer. Das Vermischen kann kontinuierlich oder batchweise erfolgen. Die vermischte Zusammensetzung wird anschliessend auf ein Substrat appliziert, gegebenenfalls mittels eines geeigneten Applikationshilfsmittels. Dabei muss darauf geachtet werden, dass zwischen dem Vermischen der Komponenten und der Applikation nicht zuviel Zeit vergeht, da durch ein zu starkes Vorreagieren der Bestandteile der vermischten Zusammensetzung vor der Applikation die Funktion der ausgehärteten Zusammensetzung gestört sein kann, beispielsweise indem die Haftung zum Substrat nur ungenügend oder verzögert aufgebaut wird. Die maximale Zeitspanne, innerhalb welcher die vermischte Zusammensetzung appliziert sein sollte, wird als "Topfzeit" bezeichnet.

Nach dem Vermischen der Komponenten **K1** und **K2** beginnt die Aushärtung. Die Aldiminogruppen beginnen in der bereits beschriebenen Weise mit den Isocyanatgruppen zu reagieren, sobald sie mit Wasser in Kontakt kommen. Entweder ist das Wasser in der vermischten Zusammensetzung bereits vorhanden - indem es ein Bestandteil der Komponente **K2** war, oder indem es der Zusammensetzung vor oder während dem Vermischen der beiden Komponenten **K1** und **K2** zugesetzt wurde -, oder das Wasser diffundiert in der Form von Luftfeuchtigkeit in die vermischte Zusammensetzung. Im letztgenannten Fall erfolgt die Reaktion der Aldiminogruppen mit den Isocyanatgruppen von aussen nach innen, parallel zum Eindringen der Feuchtigkeit aus der Luft in die Zusammensetzung. Wie bereits beschrieben, muss die Reaktion der Isocyanatgruppen mit den hydrolysierenden Aldiminogruppen nicht notwendigerweise über freie Aminogruppen erfolgen, sondern kann auch über Zwischenstufen der Hydrolysereaktion erfolgen. Auf die gleiche Weise werden die Reaktivgruppen von weiteren gegebenenfalls in der Zusammensetzung vorhandenen blockierten Aminen freigesetzt. Weiterhin reagieren nach dem Vermischen der Komponenten **K1** und **K2** die in der Zusammensetzung vorhandenen Hydroxyl-, Mercapto- und Aminogruppen mit den Isocyanatgruppen. Überschüssige Isocyanatgruppen reagieren insbesondere direkt mit Wasser. Als Ergebnis dieser Reaktionen vernetzt die vermischte Zusammensetzung und härtet schliesslich zu einem festen Material aus.

Die Aushärtung der beschriebenen härtbaren Zusammensetzungen erfolgt im Allgemeinen ohne die Bildung von Blasen, auch bei hoher Aushärtungsgeschwindigkeit. Die Aushärtungsgeschwindigkeit lässt sich über die Art und Menge eines oder mehrerer gegebenenfalls vorhandener Katalysatoren, über die bei der Aushärtung herrschende Temperatur sowie über die Luftfeuchtigkeit bzw. die Menge zugesetzten Wassers beeinflussen.

Wie vorgängig bereits erwähnt, kann die tertiäre Aminogruppe der Aldimine der Formel (I), beziehungsweise der Aldiminogruppen-haltigen Verbindungen **AV**, eine katalytische Wirkung auf die Reaktion der Isocyanatgruppen haben und deshalb die Aushärtung beschleunigen. Dabei ist es vorteilhaft, dass deren Basizität vergleichsweise niedrig ist, da stark basische tertiäre Amine die säurekatalysierte Hydrolyse der Aldiminogruppen stören und/oder die direkte Reaktion der Isocyanatgruppen, insbesondere mit Wasser, übermässig beschleunigen können, was sich bei der Aushärtung störend auswirken kann. Die beschleunigende Wirkung der tertiären Aminogruppen auf die Reaktion der Isocyanatgruppen, insbesondere auf die Reaktion der nach dem Abreagieren der Aldiminogruppen übrigbleibenden Isocyanatgruppen mit vorhandenem Wasser, wird zusätzlich unterstützt durch die Tatsache, dass die tertiäre Aminogruppe im Aldehyd-Teil des Aldimins lokalisiert ist. Bei der Hydrolyse der Aldiminogruppen werden Aldehyde **ALD** der Formel (IV) enthaltend die tertiäre Aminogruppe freigesetzt. Aufgrund ihrer relativ geringen Grösse sind die Aldehyde **ALD** in der aushärtenden Zusammensetzung recht gut beweglich, was ihre katalytische Wirkung auf weitere Isocyanatgruppen potentiell erhöht. Die freigesetzten Aldehyde **ALD** verbleiben weitgehend in der ausgehärteten Zusammensetzung, weisen dort eine ausgezeichnete Verträglichkeit auf, neigen nicht zum Ausschwitzen und haben nur eine geringe weichmachende Wirkung, was oft sehr vorteilhaft ist. Ein weiterer Vorteil der beschriebenen Zusammensetzungen liegt im vergleichsweise geringen Geruch der beschriebenen Aldimine und der bei der Aushärtung freigesetzten Aldehyde **ALD**. Dadurch weisen die Zusammensetzungen vor, während und nach der Aushärtung nur einen geringen Geruch auf.

Als Ergebnis dieser Reaktionen mit Wasser, insbesondere in der Formel von Luftfeuchtigkeit, vernetzt die Zusammensetzung und härtet schliesslich zu einem festen Material aus.

Bevorzugte Anwendungen der beschriebenen härtbaren Zusammensetzungen sind ein- oder zweikomponentige Klebstoffe, Dichtstoffe, Vergussmassen, Beschichtungen, Bodenbeläge, Anstriche, Lacke, Primer oder Schäume. Einzelne Anwendungen sollen im Weiteren kurz beschrieben werden, was jedoch eine anderweitige Anwendung dieser Zusammensetzungen keineswegs einschränken soll.

In einer bevorzugten Ausführungsform wird eine der beschriebenen härtbaren Zusammensetzungen als elastischer Kleb- oder Dichtstoff verwendet. In dieser Anwendung liegt der Gehalt des Polyisocyanates, bevorzugt in Form eines Isocyanatgruppen aufweisenden Polyurethanpolymers, insbesondere im Bereich von 10 - 80 Gewichts-%, bevorzugt von 15 - 70 Gewichts-%, bezogen auf die gesamte Zusammensetzung.

Weiterhin enthält die härtbare Zusammensetzung in der Anwendung als elastischer Kleb- oder Dichtstoff vorteilhaft mindestens einen Füllstoff, wobei dieser sowohl die rheologischen Eigenschaften der nicht ausgehärteten Zusammensetzung als auch die mechanischen Eigenschaften und die Oberflächenbeschaffenheit der ausgehärteten Zusammensetzung beeinflusst. Geeignete Füllstoffe sind die bereits genannten anorganischen und organischen Füllstoffe. Bevorzugt sind Russ, Calciumcarbonate, calcinierte Kaoline, hochdisperse Kieselsäuren, PVC-Pulver sowie flammhemmende Füllstoffe wie Hydrate oder Hydroxide, insbesondere Aluminiumhydroxid. Der Gehalt an Füllstoff liegt insbesondere im Bereich von 10 bis 70 Gewichts-%, bevorzugt von 20 bis 60 Gewichts-%, bezogen auf die gesamte Zusammensetzung. Es kann von Vorteil sein, eine Mischung verschiedener Füllstoffe einzusetzen.

Weiterhin enthält die härtbare Zusammensetzung in der Anwendung als elastischer Kleb- oder Dichtstoff vorteilhaft mindestens einen der bereits genannten Katalysatoren, welcher die Hydrolyse der Aldiminogruppen beziehungsweise die Reaktion der Isocyanatgruppen beschleunigt. Insbesondere geeignet sind Gemische aus organischen Säuren und einer metallorganischen Verbindung oder einem Metallkomplex, aus einer organischen Säure und einer tertiäre Aminogruppen enthaltenden Verbindung, oder Gemische aus organischen Säuren, einer metallorganischen Verbindung oder einem Metallkomplex, und einer tertiäre Aminogruppen enthaltenden Verbindung. Ein typischer Gehalt an Katalysatoren beträgt üblicherweise 0.005 bis 2 Gewichts-% bezogen auf die gesamte Zusammensetzung, wobei dem Fachmann klar ist, welche Einsatzmengen für welche Katalysatoren sinnvoll sind.

Ein elastischer Kleb- oder Dichtstoff kann in der Form einer einkomponentigen oder einer zweikomponentigen Zusammensetzung vorliegen, wobei diese jeweils in der bereits beschriebenen Weise hergestellt und appliziert wird. Ein einkomponentiger elastischer Kleb- oder Dichtstoff enthält bevorzugt mindestens ein Polyurethanpolymer **PUP1**.

Geeignete Anwendungen eines ein- oder zweikomponentigen elastischen Klebstoffes sind beispielsweise das Verkleben von Bauteilen im Hoch- oder Tiefbau und bei der Fertigung oder Reparatur von industriellen Gütern oder Konsumgütern, insbesondere von Fenstern, Haushaltmaschinen oder Transportmitteln wie Fahrzeugen zu Wasser oder zu Lande, bevorzugt Automobile, Busse, Lastkraftwagen, Züge oder Schiffe, sowie das Verkleben von Artikeln der Möbel-, Textil- oder Verpackungsindustrie; oder das Abdichten von Fugen, Nähten oder Hohlräumen in der industriellen Fertigung oder Reparatur, oder im Hoch- oder Tiefbau.

Geeignete Anwendungen eines ein- oder zweikomponentigen elastischen Dichtstoffes sind beispielsweise das Abdichten eines Bauwerkes, insbesondere Fugen im Hoch- oder Tiefbau, oder das Abdichten eines Teils eines Bauwerkes, beispielsweise eines Fensters oder eines Fussbodens, oder das Abdichten eines industriellen Gutes, wie beispielsweise einer Haushaltmaschine oder eines Transportmittels, insbesondere eines Fahrzeugs zu Wasser oder zu Lande, oder eines Teils davon.

In einer weiteren bevorzugten Ausführungsform wird eine der beschriebenen härtbaren Zusammensetzungen als elastische Beschichtung verwendet. In dieser Anwendung liegt der Gehalt des Polyisocyanates insbesondere im Bereich von 10 - 80 Gewichts-%, bevorzugt von 15 - 70 Gewichts-%, bezogen auf die gesamte Zusammensetzung.

Weiterhin enthält die härtbare Zusammensetzung in der Anwendung als elastische Beschichtung vorteilhaft mindestens einen Füllstoff, wobei dieser sowohl die rheologischen Eigenschaften der nicht ausgehärteten Zusammensetzung als auch die mechanischen Eigenschaften und die Oberflächenbeschaffenheit der ausgehärteten Zusammensetzung beeinflusst. Geeignet sind die bereits genannten anorganischen und organischen Füllstoffe. Bevorzugt sind Calciumcarbonate, Baryt und Quarzmehle, sowie flammhemmende Füllstoffe wie Hydrate oder Hydroxide, insbesondere Aluminiumhydroxid. Der Gehalt an Füllstoff liegt insbesondere im Bereich von 10 bis 70 Gewichts-%, bevorzugt von 20 bis 60 Gewichts-%, bezogen auf die gesamte Zusammensetzung. Es kann von Vorteil sein, eine Mischung verschiedener Füllstoffe einzusetzen.

Weiterhin enthält die härtbare Zusammensetzung in der Anwendung als elastische Beschichtung vorteilhaft mindestens einen Katalysator. Dabei sind dieselben Katalysatoren in denselben Mengen geeignet, wie sie bereits als geeignete Bestandteile von elastischen Kleb- und Dichtstoffe erwähnt wurden.

Weiterhin enthält die härtbare Zusammensetzung in der Anwendung als elastische Beschichtung vorteilhaft mindestens einen weiteren der bereits genannten Hilfs- und Zusatzstoffe, insbesondere ausgewählt aus der Gruppe umfassend Pigmente, Verlaufsmittel, Entschäumer und Stabilisatoren.

Eine elastische Beschichtung kann in der Form einer einkomponentigen oder einer zweikomponentigen Zusammensetzung vorliegen, wobei diese jeweils in der bereits beschriebenen Weise hergestellt und appliziert wird.

Vorteilhaft weist die Zusammensetzung eine flüssige Konsistenz mit guten Verlaufseigenschaften auf. Dadurch lässt sie sich einfach als selbstverlaufende Beschichtung auf überwiegend ebene Flächen applizieren, beispielsweise als Bodenbelag.

Bei einem fertigen Bodenbelag handelt es sich häufig um einen Aufbau aus mehreren sich unterscheidenden Schichten. Ein typischer Aufbau kann beispielsweise mit einer sogenannten Grundierung beginnen, welche die Aufgabe hat, den Untergrund für die elastische Polyurethanbeschichtung vorzubereiten. Anschliessend wird beispielsweise die beschriebene Zusammensetzung als elastische Schicht aufgetragen, wobei dieser Auftrag je nach Beschaffenheit des Untergrundes und gewünschter Schichtdicke in einem oder mehreren Arbeitsgängen erfolgen kann. Pro Schicht wird üblicherweise eine Schichtdicke von 0.5 bis 3 mm, insbesondere 0.5 bis 2 mm, appliziert. Schliesslich kann anschliessend eine sogenannte Versiegelung aufgetragen werden, welche in einer dünnen Schicht, beispielsweise in einer Dicke von einigen Mikrometern bis einigen Zehntelmillimetern, die Oberflächenbeschaffenheit des Bodenbelages nochmals beeinflusst. Dabei kann es sich um eine transparente oder um eine pigmentierte Versiegelung handeln.

Im Fall einer zweikomponentigen Beschichtung werden die beiden Komponenten **K1** und **K2** vor der Applikation auf eine geeignete Weise miteinander vermischt und die vermischte Zusammensetzung innerhalb der Topfzeit appliziert.

Die Applikation der härtbaren Zusammensetzung in Form einer elastischen Beschichtung erfolgt typischerweise durch Aufgiessen auf den zu beschichtenden Untergrund und wird im flüssigen Zustand mit Hilfe beispielsweise eines Rakels oder einer Zahntraufel gleichmässig verteilt. Zusätzlich kann das Material mit einer Stachelwalze egalisiert und entlüftet werden. Es ist aber auch eine maschinelle Applikation, beispielsweise in Form einer Spritzapplikation, möglich.

Ein geeigneter Untergrund, auf welchen die Zusammensetzung typischerweise appliziert wird, ist beispielsweise Beton, Zement, Asphalt, Stahl, Holz, Keramik oder ein Kunststoff, wobei der Untergrund durch Reinigen, Bürsten oder Sandstrahlen vorbehandelt sein kann, und/oder eine Grundierung aufweisen kann. Als Grundierungen in Frage kommen beispielsweise Haftvermittlerlösungen oder Primer.

Die beschriebene elastische Beschichtung kann vorteilhaft verwendet werden im Innen- oder Aussenbereich eines Gebäudes oder eines Bauwerks, beispielsweise als Bodenbelag für Innenräume wie Büros, Industriehallen, Turnhallen oder Kühlräume oder im Aussenbereich für Balkone, Terrassen, Brücken, Parkdecks oder Sport- und Spielplätze.

In einer weiteren bevorzugten Ausführungsform wird eine der beschriebenen härtbaren Zusammensetzungen als Anstrich, Lack oder Primer verwendet. In dieser Anwendung stellt das Polyisocyanat bevorzugt entweder ein Polyisocyanat **PI1** oder ein Polyisocyanat **PI2** dar.

Unter einem "Primer" wird im vorliegenden Dokument eine als Voranstrich geeignete Zusammensetzung verstanden, die neben nichtreaktiven flüchtigen Stoffen und optional festen Zuschlägen mindestens ein Polymer und/oder mindestens eine Substanz mit reaktiven Gruppen enthält und die befähigt ist, bei der Applikation auf einem Substrat zu einem festen, gut haftenden Film in einer Schichtdicke von typischerweise mindestens 5 µm auszuhärten, wobei die Aushärtung entweder allein durch das Verdampfen der nichtreaktiven flüchtigen Stoffe, wie beispielsweise Lösemittel oder Wasser, oder durch eine chemische Reaktion, oder durch eine Kombination dieser Faktoren, zustande kommt, und welche eine gute Haftung zu einer nachfolgend aufgebrachten Schicht, beispielsweise einem Kleb- oder Dichtstoff, aufbaut.

In der Anwendung als Anstrich, Lack oder Primer bevorzugt sind einkomponentige Zusammensetzungen, wobei diese aus Gründen der Lagerstabilität bevorzugt ausschliesslich Polyisocyanate mit aliphatischen Isocyanat-gruppen enthalten.

Weiterhin enthält die härtbare Zusammensetzung in der Anwendung als Anstrich, Lack oder Primer vorteilhaft mindestens einen weiteren der bereits genannten Hilfs- und Zusatzstoffe, insbesondere mindestens ein Lösemittel. Geeignete Lösemittel sind beispielsweise Ketone wie Aceton, Methylethylketon, Methylisobutylketon, Diisobutylketon und Mesityloxid, sowie cyclische Ketone wie Cyclohexanon und Methylcyclohexanon; Ester wie Methylacetat, Ethylacetat, Propylacetat, Butylacetat, tert.Butylacetat, Formiate, Propionate oder Malonate; Ether wie Ketonether, Esterether und Dialkylether wie Diisopropylether, Diethylether, Dibutylether, Methyl-tert.butylether, Diethylenglykoldiethylether sowie Ethylenglykoldiethylether; aliphatische und aromatische Kohlenwasserstoffe wie Toluol, Xylol, Heptan, Octan, und Erdölfraktionen wie Naphtha, White Spirit, Petrolether oder Benzin; halogenierte Kohlenwasserstoffe wie Methylenchlorid; sowie N-alkylierte Lactame wie beispielsweise N-Methylpyrrolidon, N-Cyclohexylpyrrolidon oder N-Dodecylpyrrolidon. Der Gehalt an Lösemitteln liegt insbesondere im Bereich von 10 bis 90 Gewichts-%, bevorzugt 20 bis 80 Gewichts-%, bezogen auf die gesamte Zusammensetzung.

Weiterhin kann die härtbare Zusammensetzung in der Anwendung als Anstrich, Lack oder Primer weitere Bestandteile enthalten, insbesondere Triisocyanate wie Tris-(4-isocyanatophenyl)-methan und Tris-(4-isocyanatophenyl)-thiophosphat; Aminosilane, wie beispielsweise 3-Aminopropyltrimethoxysilan, N-(2-Aminoethyl)-3-aminopropyl-trimethoxysilan, Bis-[3-(tri-methoxysilyl)-propyl]-amin, 3-Aminopropyl-dimethoxymethylsilan, 3-Amino-2-methylpropyl-trimethoxysilan, N-(2-Aminoethyl)-3-aminopropyl-dimethoxymethylsilan, 4-Aminobutyl-trimethoxysilan, 4-Aminobutyl-dimethoxymethylsilan, 4-Amino-3-methylbutyl-trimethoxysilan, 4-Amino-3,3-dimethylbutyl-trimethoxysilan, 4-Amino-3,3-dimethylbutyl-dimethoxymethylsilan, [3-(2-Aminoethylamino)-propyl]trimethoxysilan (= 4,7,10-Triazadecyl-trimethoxysilan), 2-Aminoethyl-trimethoxysilan, 2-Aminoethyl-dimethoxymethylsilan, Aminomethyl-trimethoxysilan, Aminomethyl-dimethoxymethylsilan, Aminomethylmethoxydimethylsilan, 7-Amino-4-oxaheptyldimethoxymethylsilan, N-(Methyl)-3-aminopropyltrimethoxysilan, N-(n-butyl)-3-aminopropyltrimethoxysilan; Tris-[3-(tri-methoxysilyl)-propyl]-amin, 1,3,5-tris[3-(trimethoxysilyl)propyl]-1,3,5-triazine-2,4,6(1H,3H,5H)-trion-harnstoff (=Tris(3-(trimethoxysilyl)propyl)isocyanurat) sowie die ensprechendenen Analoga, bei welchen die Methoxygruppe durch einer Ethoxy- oder Isoproxygruppe ersetzt ist; Mercaptosilane, wie beispielsweise 3-Mercaptopropyltrimethoxysilan oder 3-Mercaptopropyltriethoxysilan, Epoxysilane, wie beispielsweise 3-Glycidyloxypropyltrimethoxysilan, 3-Glycidyloxypropyltriethoxysilan; Ureidoalkylsilane sowie Addukte von Amino- und /oder Mercaptosilanen mit Epoxiden oder Epoxysilanen, beispielsweise mit 3-Glycidyloxypropylsilanen; Titanate, bevorzugt solche, welche mindestens einen über eine Sauerstoff-Titan-Bindung an das Titanatom gebundenen Substituenten aufweisen, insbesondere eine Alkoxygruppe, Sulfonatgruppe, Carboxylatgruppe, Dialkylphosphatgruppe, Dialkylpyrophosphatgruppe oder eine Acetylacetonatgruppe, wobei bei mehreren Substituenten diese alle gleich oder untereinander gemischt sein können. Beispiele für geeignete Titanate sind die von Kenrich Petrochemicals unter dem Handelsnamen Ken-React® erhältlichen Typen KR TTS, KR 7, KR 9S, KR 12, KR 26S, KR 33DS, KR 38S, KR 39DS, KR44, KR 134S, KR 138S, KR 158FS, KR212, KR 238S, KR 262ES, KR 138D, KR 158D, KR238T, KR 238M, KR238A, KR238J, KR262A, LICA 38J, KR 55, LICA 01, LICA 09, LICA 12, LICA 38, LICA 44, LICA 97, LICA 99, KR OPPR und KR OPP2, sowie die von DuPont unter dem Handelsnamen Tyzor® erhältlichen Typen ET, TPT, NPT, BTM, AA, AA-75, AA-95, AA-105, TE, ETAM und OGT.

Vorteilhaft enthalten die beschriebenen Anstriche, Lacke oder Primer mindestens einen Haftvermittler in der Form der bereits erwähnten Silane oder Titanate.

Vorteilhaft enthalten die beschriebenen Anstriche, Lacke oder Primer mindestens einen der bereits erwähnten Katalysatoren.

Die beschriebenen Anstriche, Lacke oder Primer werden üblicherweise mittels Pinsel, Filz, Tuch, Schwamm oder einer Spritzpistole auf das Substrat aufgetragen. Dieser Auftrag kann manuell oder automatisch, insbesondere mittels Roboter, erfolgen.

Die beschriebenen Anstriche, Lacke oder Primer reagieren bei Kontakt mit Wasser, insbesondere in Form von Luftfeuchtigkeit, in der bereits beschriebenen Weise, wobei weitere mit Wasser reaktive Komponenten, wie beispielsweise Titanat- oder Silangruppen-haltige Verbindungen, ebenfalls mit Wasser reagieren. Zudem beginnen nach erfolgtem Auftrag der Beschichtung gegebenenfalls darin enthaltene flüchtige Lösemittel zu verdunsten. In der Folge bildet sich auf dem Substrat ein fester, gut haftender Film. Dessen Schichtdicke liegt vorteilhafterweise bei ungefähr 5 - 100 µm, insbesondere bei 10 - 50 µm.

Ein Primer wird vorteilhaft verwendet als Haftbrücke für Polymerzusammensetzungen in der Form von Klebstoffen, Dichtstoffen oder Beschichtungen wie beispielsweise Bodenbelägen, insbesondere Polymerzusammensetzungen auf Basis von Polyurethanen mit Isocyanatgruppen und / oder Silangruppen.

Erdingsgemässe Zusammensetzungen werden typischerweise verwendet in einem Verfahren zum Verkleben von einem Substrat **S1** mit einem Substrat **S2**, welches die Schritte umfasst:
i) Applikation einer vorgängig beschriebenen härtbaren Zusammensetzung auf ein Substrat **S1**;
ii) Kontaktieren der applizierten Zusammensetzung mit einem Substrat **S2** innerhalb der Offenzeit der Zusammensetzung;
oder
i') Applikation einer vorgängig beschriebenen Zusammensetzung auf ein Substrat **S1** und auf ein Substrat **S2**;
ii') Kontaktieren der applizierten Zusammensetzung miteinander innerhalb der Offenzeit der Zusammensetzung;
wobei das Substrat **S2** aus dem gleichen oder einem unterschiedlichen Material wie das Substrat **S1** besteht.

Weiterhin kann die erfindungsgemässe Zusammensetzung verwendet werden in einem Verfahren zum Abdichten. Dieses umfasst den Schritt:
i") Applikation einer vorgängig beschriebenen härtbaren Zusammensetzung zwischen ein Substrat **S1** und ein Substrat **S2**, so dass die Zusammensetzung mit dem Substrat **S1** und dem Substrat **S2** in Kontakt steht;
wobei das Substrat **S2** aus dem gleichen oder einem unterschiedlichen Material wie das Substrat **S1** besteht.
Üblicherweise wird der Dichtstoff in eine sogenannte Fuge eingepresst.

Weiterhin kann die erfindungsgemässe Zusammensetzung verwendet werden in einem Verfahren zum Beschichten eines Substrates **S1**. Dieses umfasst den Schritt:
i'") Applikation einer vorgängig beschriebenen härtbaren Zusammensetzung auf ein Substrat **S1** innerhalb der Offenzeit der Zusammensetzung.

In diesen drei Verfahren sind geeignete Substrate **S1** und/oder **S2** beispielsweise anorganische Substrate wie Glas, Glaskeramik, Beton, Mörtel, Backstein, Ziegel, Gips und Natursteine wie Granit oder Marmor; Metalle oder Legierungen wie Aluminium, Stahl, Buntmetalle, verzinkte Metalle; organische Substrate wie Leder, Stoffe, Papier, Holz, mit Harz gebundene Holzwerkstoffe, Harz-Texil-Compositewerkstoffe, Kunststoffe wie Polyvinylchlorid (Hart- und Weich-PVC), Acrylonitril-Butadien-Styrol-Copolymere (ABS), SMC (Sheet Moulding Composites), Polycarbonat (PC), Polyamid (PA), Polyester, PMMA, Polyester, Epoxidharze, Polyurethane (PUR), Polyoxymethylen (POM), Polyolefine (PO), insbesondere mittels Plasma, Corona oder Flammen oberflächenbehandeltes Polyethylen (PE) oder Polypropylen (PP), Ethylen/Propylen-Copolymere (EPM) und Ethylen/Propylen-Dien-Terpolymere (EPDM); beschichtete Substrate wie pulverbeschichtete Metalle oder Legierungen; sowie Farben und Lacke, insbesondere Automobillacke.

Die Substrate können bei Bedarf vor dem Applizieren der Zusammensetzung vorbehandelt werden. Derartige Vorbehandlungen umfassen insbesondere physikalische und/oder chemische Reinigungsverfahren, beispielsweise Schleifen, Sandstrahlen, Bürsten oder dergleichen, oder Behandeln mit Reinigern oder Lösemitteln oder das Aufbringen eines Haftvermittlers, einer Haftvermittlerlösung oder eines Primers.

Im Fall einer zweikomponentigen Zusammensetzung werden die beiden Komponenten **K1** und **K2** kurz vor der Applikation miteinander vermischt.

Im Fall einer hitzehärtenden Zusammensetzung wird die applizierte Zusammensetzung anschliessend an die Verklebung, die Abdichtung oder die Beschichtung eingebrannt, indem sie auf eine geeignete Temperatur erhitzt wird.

Die Applikation der härtbaren Zusammensetzung kann in einem breiten Temperaturspektrum erfolgen. Beispielsweise kann die Zusammensetzung bei Raumtemperatur appliziert werden, wie es für einen elastischen Klebstoff oder einen Dichtstoff typisch ist. Die Zusammensetzung kann aber auch bei tieferen wie auch bei höheren Temperaturen appliziert werden. Letzteres ist insbesondere dann vorteilhaft, wenn die Zusammensetzung hochviskose oder schmelzbare Komponenten enthält, wie sie in Schmelzklebstoffen, beispielsweise Warmschmelzklebstoffen (Warm-Melt) oder Heissschmelzklebstoffen (Hot-Melt) typischerweise vorhanden sind. Die Applikationstemperaturen liegen für Warm-Melts beispielsweise im Bereich von 40 bis 80 °C, bei Hot-Melts im Bereich von 85 bis 200 °C.

Aus diesen beschriebenen Verfahren zum Verkleben, Abdichten bzw. Beschichten - beziehungsweise aus der Verwendung einer der beschriebenen Zusammensetzungen als ein- oder zweikomponentiger Klebstoff, Dichtstoff, Vergussmasse, Beschichtung, Bodenbelag, Anstrich, Lack, Primer oder Schaum - entsteht ein Artikel.

Dieser Artikel ist insbesondere ein Bauwerk, insbesondere ein Bauwerk des Hoch- oder Tiefbaus, oder ein industrielles Gut oder ein Konsumgut, insbesondere ein Fenster, eine Haushaltmaschine, oder ein Transportmittel, insbesondere ein Fahrzeug zu Wasser oder zu Lande, bevorzugt ein Automobil, ein Bus, ein Lastkraftwagen, ein Zug oder ein Schiff, oder ein Anbauteil eines Transportmittels, oder ein Artikel der Möbel-, Textil- oder Verpackungsindustrie.

### Beispiele

### 1. Beschreibung der Messmethoden

Die **Viskosität** wurde auf einem thermostatisierten Kegel-Platten-Viskosimeter Physica UM (Kegeldurchmesser 20 mm, Kegelwinkel 1°, Kegelspitze-Platten-Abstand 0.05 mm, Schergeschwindigkeit 10 bis 1000 s⁻¹) gemessen.

Der **Amingehalt**, das heisst der totale Gehalt an freien Aminogruppen und blockierten Aminogruppen (Aldiminogruppen) in den hergestellten Verbindungen, wurde titrimetrisch bestimmt (mit 0.1N HClO₄ in Eisessig, gegen Kristallviolett) und ist stets angegeben in mmol N/g.

Der ***p*Ka-Wert** für die konjugierte Säure einer Mannich-Base wurde näherungsweise anhand des Halbneutralisations-Potentials bei der potentiometrischen Titration von ca. 1 mmol Mannich-Base in 50 ml Wasser oder, wo nicht möglich, in 50 ml Wasser/Isopropanol 1/1, mit 0.1N HCl bestimmt, wobei die gemessenen Werte in Wasser/Isopropanol mittels Referenzsubstanzen auf die erwarteten Werte in reinem Wasser umgerechnet wurden.

**Infrarotspektren** wurden auf einem FT-IR Gerät 1600 von Perkin-Elmer gemessen, feste Substanzen als KBr-Presslinge im Direktstrahl, Flüssigkeiten und Öle als unverdünnte Filme (falls nötig durch Lösen der Substanz in CH₂Cl₂ und Eindampfen des Lösemittels aufgebracht) auf einer horizontalen ATR-Messeinheit mit ZnSe-Kristall; die Absorptionsbanden sind in Wellenzahlen (cm⁻¹) angegeben (Messfenster: 4000-650 cm⁻¹); der Zusatz sh weist auf eine als Schulter erscheinende Bande hin, der Zusatz br auf eine breite Bande.

**¹H-NMR-Spektren** wurden auf einem Spektrometer des Typs Bruker DPX-300 bei 300.13 MHz gemessen; die chemischen Verschiebungen δ sind angegeben in ppm relativ zu Tetramethylsilan (TMS), Kopplungskonstanten *J* sind angegeben in Hz; echte und Pseudo-Kopplungsmuster wurden nicht unterschieden.

### 2. Herstellung von Aldehyden

### 2,2-Dimethyl-3-(N-pyrrolidino)-propanal

In einem Rundkolben wurden unter Stickstoffatmosphäre 51.0 g (0.72 mol) Pyrrolidin vorgelegt und in 100 ml abs. Ethanol gelöst. Unter gutem Rühren und Eiskühlung wurden aus einem Eintropftrichter langsam 40.6 g (0.40 mol) 96%ige Schwefelsäure zugetropft, wobei darauf geachtet wurde, dass die Temperatur der entstehenden Suspension nicht über 50 °C anstieg. Darauf wurden 23.8 g (0.79 mol) Paraformaldehyd und 54.5 g (0.76 mol) Isobutyraldehyd zugegeben und die Reaktionsmischung im Ölbad bei 120 °C während 2 Stunden unter Rückfluss gerührt. Die klare Reaktionsmischung wurde auf Raumtemperatur abgekühlt, in 500 ml 10%iger Natriumsulfit-Lösung aufgenommen und mit 10N NaOH basisch gestellt. Die organische Phase wurde im Scheidetrichter abgetrennt und beiseite gestellt. Die wässrige Phase wurde zweimal mit je 50 ml Ethylacetat extrahiert, die Extrakte mit wasserfreiem Na₂SO₄getrocknet und eingeengt. Die organischen Phasen wurden vereint und im Vakuum fraktioniert. Ausbeute: 79.2 g (71% d.Th. (*d.Th.*=*der Theorie*)) als farblose, klare, aminartig riechende Flüssigkeit mit einem Amingehalt von 6.34 mmol N/g und einer Viskosität von 3 mPa·s bei 20 °C.

### Alternative Herstellung:

In einem Rundkolben wurden unter Stickstoffatmosphäre 59.8 g (0.72 mol) 36%iger wässriger Formaldehyd und 53.8 g (0.75 mol) Isobutyraldehyd vorgelegt. Unter gutem Rühren und Eiskühlung wurden aus einem Eintropftrichter langsam 51.0 g (0.72 mol) Pyrrolidin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene trübe, farblose Reaktionsmischung wurde im Ölbad bei 100 °C während 18 Stunden unter Rückfluss gerührt, auf Raumtemperatur abgekühlt und die Phasen im Scheidetrichter getrennt. Die organische Phase wurde ohne weitere Aufbereitung im Vakuum fraktioniert. Das Produkt destillierte bei einer Kopftemperatur von 80 °C und einem Druck von 17 mbar. Ausbeute: 95.9 g (86% d.Th.) farblose, klare Flüssigkeit mit einem Amingehalt von 6.34 mmol N/g.
*p*Ka ≈ 9.2.
IR: 2962, 2928sh, 2873, 2782, 2692 (CHO), 1724 (C=O), 1460, 1400, 1362, 1351,1327,1293,1238,1202,1141,1114,1059,1036,1002,965,916,906,874, 774. ¹H-NMR (CDCl₃, 300 K): δ 9.56 (*s*, 1 H, CHO), 2.63 (*s*, 2 H, NC*H*₂C(CH₃)₂), 2.49 (*m*, 4 H, NC*H*₂CH₂^{cycl.}), 1.71 (*m,* 4 H, NCH_{2C}*H*₂^{cycl.})1.08 (*s*, 6 H, CH₂C(C*H*₃)₂).

### 2,2-Dimethyl-3-(N-morpholino)-propanal

In einem Rundkolben wurden unter Stickstoffatmosphäre 83.1 g (1.00 mol) 36%iger wässriger Formaldehyd und 75.0 g (1.04 mol) Isobutyraldehyd vorgelegt. Unter gutem Rühren und Eiskühlung wurden aus einem Eintropftrichter langsam 87.1 g (1.00 mol) Morpholin zugetropft, wobei darauf geachtet wurde, dass die Temperatur der Reaktionsmischung nicht über 20 °C anstieg. Nach erfolgter Zugabe liess man eine Stunde bei Raumtemperatur rühren. Die entstandene klare, farblose Reaktionsmischung wurde im Ölbad bei 100 °C während 18 Stunden unter Rückfluss gerührt, auf Raumtemperatur abgekühlt und die Phasen im Scheidetrichter getrennt. Die organische Phase wurde ohne weitere Aufbereitung im Vakuum fraktioniert. Das Produkt destillierte bei einer Kopftemperatur von 97 °C und einem Druck von 14 mbar. Ausbeute: 145.5 g (85% d.Th.) als farblose, klare, fast geruchlose Flüssigkeit mit einem Amingehalt von 5.72 mmol N/g und einer Viskosität von 11 mPa·s bei 20 °C.
*p*Ka ≈ 6.3.
IR: 2958,2981,2850,2803,2695 (CHO),1979,1722 (C=O), 1455, 1402, 1374, 1361,1319,1280,1268,1206,1137,1115,1070,1035,1015,951,906,864,801, 774. ¹H-NMR (CDCl₃, 300 K): δ 9.55 (*s*, 1 H, CHO), 3.63 (*d, J* = 9.4, 4 H, OCH₂^{cycl.}), 2.47 (*s*, 2 H, NC*H*₂C(CH₃)₂), 2.44 (*m*, 4 H, NCH₂^{cycl.}), 1.08 (*s*, 6 H, CH₂C(C*H*₃)₂).

### 2,2-Dimethyl-3-(N-(2,6-dimethyl)morpholino)-propanal

Unter gleichen Bedingungen wie zur Herstellung von 2,2-Dimethyl-3-(N-morpholino)-propanal wurden 41.7 g (0.50 mol) 36%iger wässriger Formaldehyd mit 37.9 g (0.53 mol) Isobutyraldehyd und 57.6 g (0.50 mol) 2,6-Dimethylmorpholin (BASF; Isomerengemisch) umgesetzt und aufgearbeitet. Das Produkt destillierte bei einer Kopftemperatur von 104 °C und einem Druck von 20 mbar. Ausbeute: 80.8 g (81% d.Th.) als farblose, klare, schwach aminartig riechende Flüssigkeit, die einen Amingehalt von 4.99 mmol N/g aufwies.
*p*Ka ≈ 5.9.
IR: 2970, 2933, 2868, 2799, 2771sh, 2727, 2700sh (CHO), 2627, 1724 (C=O), 1456, 1403, 1373, 1362sh, 1320, 1281, 1240, 1216sh, 1178, 1144, 1077, 1042, 1003, 967, 946, 916, 887sh, 879, 860, 837, 799, 773.
¹H-NMR (CDCl₃, 300 K): δ 9.55 (*s*, 1 H, CHO), 3.59 (*ddq*, *J* = 10.0 / 6.3 / 2.3, 2 H, OC*H*CH₃), 2.57 (*qd*, *J* = 10.5 / 2.1, 2×1 H von CH(CH₃)-C*H*₂N), 2.44 (*s*, 2 H, NC*H*₂C(CH₃)₂), 1.86 (*dd*, *J* = 11.4 / 10.0, 2×1 H von CH(CH₃)-C*H*₂N), 1.11 (*d*, *J* = 6.3, 6 H, OCHC*H*₃), 1.06 (*s*, 6 H, NCH₂C(C*H*₃)₂).

### 2,2-Dimethyl-3-(N-benzylmethylamino)-propanal

Unter gleichen Bedingungen wie zur Herstellung von 2,2-Dimethyl-3-(N-morpholino)-propanal wurden 39.2 g (0.47 mol) 36%iger wässriger Formaldehyd mit 35.6 g (0.49 mol) Isobutyraldehyd und 57.0 g (0.47 mol) N-Benzyl-methylamin umgesetzt und aufgearbeitet. Das Produkt destillierte bei einer Kopftemperatur von 74 °C und einem Druck von 4·10⁻² mbar. Ausbeute: 80.6 g (83% d.Th.) als farblose, klare und fast geruchsfreie Flüssigkeit, die einen Amingehalt von 4.83 mmol N/g aufwies.
*p*Ka ≈ 7.2.
IR: 3084, 3060, 3026, 2962, 2928, 2870, 2840, 2784, 2700 (CHO), 1950, 1876, 1808, 1722 (C=O), 1654, 1602, 1584, 1542, 1494, 1452, 1420, 1398, 1362, 1316, 1290, 1256, 1172, 1118, 1074, 1038, 1024, 1002, 976, 948, 916, 910, 880, 856, 826, 774, 738, 698, 670.
¹H-NMR (CDCl₃, 300 K): δ 9.52 (*s*, 1 H, CHO), 7.33-7.20 (*m*, 5 H, Ph-H), 3.51 (*s*, 2 H, Ph-C*H*₂-N), 2.59 (*s*, 2 H, NC*H*₂C(CH₃)₂), 2.16 (*s*, 3 H, NCH₃), 1.07 (*s*, 6 H, NCH₂C(C*H*₃)₂).

### 2,2-Dimethyl-3-(N-benzylisopropylamino)-propanal

Unter gleichen Bedingungen wie zur Herstellung von 2,2-Dimethyl-3-(N-morpholino)-propanal wurden 28.0 g (0.34 mol) 36%iger wässriger Formaldehyd mit 25.4 g (0.35 mol) Isobutyraldehyd und 50.0 g (0.34 mol) N-Benzyl-isopropylamin umgesetzt und aufgearbeitet. Das Produkt destillierte bei einer Kopftemperatur von 100 °C und einem Druck von 4·10⁻² mbar. Ausbeute: 48.6 g (62% d.Th.) als blassgelbe, klare und fast geruchsfreie Flüssigkeit, die einen Amingehalt von 4.28 mmol N/g aufwies.
*p*Ka ≈ 6.6.
IR: 3084, 3060, 3026, 2962, 2929, 2869, 2823sh, 2806, 2699 (CHO), 1948, 1869, 1722 (C=O), 1602, 1584, 1540, 1494, 1460, 1452, 1398, 1385, 1362, 1320, 1252, 1234, 1207, 1164, 1118, 1093, 1074, 1056, 1027, 1003, 965, 906, 877, 826, 772, 730, 697, 668.
¹H-NMR (CDCl₃, 300 K): δ 9.39 (*s*, 1 H, CHO), 7.32-7.17 (*m*, 5 H, Ph-H), 3.53 (*s*, 2 H, Ph-C*H*₂-N), 2.73 (*sept*., *J* = 6.6, 1 H, NC*H*(CH₃)₂), 2.59 (*s*, 2 H, NC*H*₂C(CH₃)₂), 1.00 (*s*, 6 H, NCH₂C(C*H*₃)₂), 2.73 (*d, J* = 6.6, 6 H, NCH(C*H*₃)₂).

### 2,2-Dimethyl-3-(N-cyclohexylmethylamino)-propanal

Unter gleichen Bedingungen wie zur Herstellung von 2,2-Dimethyl-3-(N-morpholino)-propanal wurden 36.8g (0.44 mol) 36%iger wässriger Formaldehyd mit 33.4 g (0.46 mol) Isobutyraldehyd und 50.0 g (0.44 mol) N-Cyclohexyl-methylamin umgesetzt und aufgearbeitet. Das Produkt destillierte bei einer Kopftemperatur von 69 °C und einem Druck von 4·10⁻² mbar. Ausbeute: 65.8 g (76% d.Th.) als farblose, klare und nach Amin riechende Flüssigkeit, die einen Amingehalt von 4.94 mmol N/g aufwies.
*p*Ka ≈ 8.4.
IR: 2925, 2851, 2796, 2685 (CHO), 1723 (C=O), 1464, 1450, 1422, 1396, 1376, 1381, 1344, 1319sh, 1262, 1200, 1177, 1143, 1110, 1072, 1057sh, 1045, 1025, 1004, 987, 960, 916, 890, 878, 859, 836, 784, 772.
¹H-NMR (CDCl₃, 300 K): δ 9.55 (*s*, 1 H, CHO), 2.55 (*s*, 2 H, NC*H*₂C(CH₃)₂), 2.22 (*m*, 1 H, NCH^{cycl.}), 2.20 (*s*, 3 H, NCH₃), 1.74 (*m*, 4 Cy-H), 1.60 (*m*, 1 Cy-H), 1.28-1.08 (*m*, 5 Cy-H), 1.06 (*s*, 6 H, CH₂C(C*H*₃)₂).

### N-(2,2-Dimethyl-3-oxopropyl)-N-methyl-pyrrolidiniumiodid

Unter Stickstoffatmosphäre wurden 1.18 g (7.5 mmol) 2,2-Dimethyl-3-(N-pyrrolidino)-propanal und 1.06 g (7.5 mmol) Methyliodid in ein Pillenglas eingewogen, dieses verschlossen und gut umgeschwenkt. Die Mischung trübte sofort ein; nach wenigen Minuten begann sich am Glasboden ein weisser Niederschlag zu sammeln. Das Pillenglas wurde bei Raumtemperatur stehen lassen. Nach 90 Minuten war der gesamte Inhalt schneeweiss durchkristallisiert. IR (KBr-Pressling): 3042, 3002, 2982, 2958, 2890, 2874, 2838, 2734, 1754, 1720 (C=O), 1682, 1478, 1466, 1450, 1436, 1424, 1400, 1382, 1364, 1344, 1310, 1276, 1234, 1182, 1166, 1150, 1108, 1058, 1032, 1000, 974, 944, 916, 878, 820, 768, 732.
¹H-NMR (D₂O, 300 K): δ 9.65 (*s*, 1 H, CHO), 3.81 (*s*, 2 H, NC*H*₂C(CH₃)₂), 3.69 und 3.53 (2×*m*, 2×2 H, NC*H*₂CH₂^{cycl.}), 2.93 (*s*, 3 H, NCH₃), 2.22 (*m*, 4 H, NCH₂C*H*₂^{cycl.}), 1.34 (*s*, 6 H, CH₂C(C*H*₃)₂).

### N-(2,2-Dimethyl-3-oxopropyl)-N-methyl-morpholiniumiodid

Unter Stickstoffatmosphäre wurden 1.03 g (5.9 mmol) 2,2-Dimethyl-3-(N-morpholino)-propanal und 0.83 g (5.9 mmol) Methyliodid in ein Pillenglas eingewogen, dieses verschlossen und gut umgeschwenkt. Die Mischung trübte innerhalb von 10 Sekunden ein; nach wenigen Minuten begann sich am Glasboden ein weisser Niederschlag zu sammeln. Das Pillenglas wurde eine Stunde bei Raumtemperatur stehen lassen und dann auf 60 °C erwärmt. Nach 2 Stunden war der gesamte Inhalt schneeweiss durchkristallisiert.
IR (KBr-Pressling): 3018, 2976, 2958, 2872, 2812, 2724, 1768sh, 1722 (C=O), 1685, 1476, 1462, 1420, 1400, 1384, 1370, 1312, 1280, 1248, 1220, 1176, 1144, 1120, 1082, 1066, 1039, 1016, 988, 952, 914, 900, 884, 868, 812, 772.
¹H-NMR (D₂O, 300 K): δ 9.64 (*s*, 1 H, CHO), 4.06 (*m*, 4 H, OCH₂^{cycl.}), 3.87 (*s*, 2 H, NC*H*₂C(CH₃)₂), 3.72 (*m*, 4 H, NCH₂^{cycl.}), 3.21 (*s*, 3 H, NCH₃), 1.37 (*s*, 6 H, CH₂C(C*H*₃)₂).

### 3. Herstellung von Aldiminen

### Beispiel 1: Aldimin A-1

In einem Rundkolben wurden unter Stickstoffatmosphäre 23.3 g 1,6-Hexamethylendiamin (70% in Wasser; Amingehalt 12.16 mmol N/g) vorgelegt. Unter kräftigem Rühren wurden aus einem Eintropftrichter 47.0 g 2,2-Dimethyl-3-(N-morpholino)-propanal zugegeben. Danach wurden die flüchtigen Bestandteile im Vakuum entfernt (10 mbar, 80 °C). Ausbeute: 57.2 g eines klaren, farblosen Öls mit einem Amingehalt von 10.10 mmol N/g und einer Viskosität von 45 mPa·s bei 20 °C.
IR: 2961, 2926, 2872, 2858, 2804sh, 2779, 1665 (C=N), 1459, 1446sh, 1392, 1360, 1338, 1292, 1239, 1199, 1191, 1139, 1116, 1060, 1032, 1001, 964, 937, 905, 875, 727.
¹H-NMR (CDCl₃, 300 K): δ 7.55 (*t, J* = 1.2, 2H, CH=N), 3.34 (*t*×*d*, *J* = 1.2 / 7.1, 4H, CH=N-C*H*₂), 2.49 (*m*, 12H, ^{cycl.}CH₂C*H*₂NC*H*₂C(CH₃)), 1.71 (*m,* 8H, ^{cycl·}C*H*₂CH₂NCH₂C(CH₃)), 1.56 (*m*, 4H, CH=N-CH₂C*H*₂), 1.29 (*m*, 4H, CH=N-CH₂CH₂C*H*₂), 1.07 (*s*, 12 H, CH₂C(C*H*₃)₂).

### Beispiel 2: Aldimin A-2

Unter gleichen Bedingungen wie in Beispiel 1 beschrieben wurden 31.8 g 1,6-Hexamethylendiamin (70% in Wasser; Amingehalt 12.16 mmol N/g) mit 71.0 g 2,2-Dimethyl-3-(N-morpholino)-propanal umgesetzt. Ausbeute: 86.3 g eines klaren, farblosen Öls mit einem Amingehalt von 9.19 mmol N/g und einer Viskosität von 145 mPa·s bei 20 °C.
IR: 2954, 2926, 2849, 2805, 2762sh, 2687, 1980, 1665 (C=N), 1454, 1395, 1375, 1358, 1332, 1317, 1282, 1267, 1204, 1135, 1117, 1070, 1036, 1012, 944, 933, 882, 864, 802, 776sh, 728.
¹H-NMR (CDCl₃, 300 K): δ 7.53 (*t, J* = 1.2, 2 H, CH=N), 3.64 (*d*, *J* = 9.3, 8 H, OCH₂^{cycl.}), 3.34 (*m*, 4 H, CH=N-C*H*₂), 2.46 (*m*, 8 H, NCH₂^{cycl.}), 2.34 (*s*, 4 H, NC*H*₂C(CH₃)₂), 1.57 (*m*, 4 H, CH=N-CH₂C*H*₂), 1.28 (*m*, 4 H, CH=N-CH₂CH₂C*H*₂), 1.06 (*s*, 12 H, CH₂C(C*H*₃)₂).

### Beispiel 3: Aldimin A-3

Unter gleichen Bedingungen wie in Beispiel 1 beschrieben wurden 13.4 g Polyetherdiamin (Polyoxypropylen-Diamin mit einem mittleren Molekulargewicht von ca. 240 g/mol; Jeffamine® D-230, Huntsman; Amingehalt 8.29 mmol N/g) mit 20.0 g 2,2-Dimethyl-3-(N-morpholino)-propanal umgesetzt. Ausbeute: 31.4 g eines klaren, farblosen Öls mit einem Amingehalt von 7.14 mmol N/g und einer Viskosität von 170 mPa·s bei 20 °C.
IR: 2961, 2926, 2887, 2850, 2803, 2763sh, 2690, 1980, 1663 (C=N), 1454, 1373, 1316, 1282, 1268, 1204, 1135sh, 1114, 1070, 1036, 1012, 1001sh, 946sh, 929, 897, 882, 864, 802, 775, 665.
¹H-NMR (CDCl₃, 300 K): δ 7.57 (*s*, 2 H, CH=N), 3.64 (*m*, 8 H, OCH₂^{cycl.}), 3.6-3.1 (*m*, ca. 12 H, OCH₂C*H*(CH₃)), 2.46 (*m*, 8 H, NCH₂^{cycl.}), 2.34 (*s*, 4 H, NC*H*₂C(CH₃)₂), 1.18-0.97 (*m*, ca. 24 H, OCH₂CH(C*H*₃) und CH₂C(C*H*₃)₂).

### Beispiel 4: Aldimin A-4

Unter gleichen Bedingungen wie in Beispiel 1 beschrieben wurden 14.55 g 1-Amino-3-aminomethyl-3,5,5-trimethylcyclohexan (Isophorondiamin oder IPDA; Vestamin® IPD, Degussa; Amingehalt 11.67 mmol N/g) mit 30.00 g 2,2-Dimethyl-3-(N-morpholino)-propanal umgesetzt. Ausbeute: 40.9 g eines klaren, farblosen Öls mit einem Amingehalt von 8.29 mmol N/g und einer Viskosität von 6.8 Pa·s bei 20 °C.
IR: 2952, 2914, 2908, 2894, 2849, 2805, 2764sh, 1980, 1663 (C=N), 1454, 1396sh, 1377, 1361, 1352sh, 1332, 1317, 1282, 1267, 1204, 1136, 1116, 1070, 1051, 1036, 1012, 968, 928, 881, 864, 802.
¹H-NMR (CDCl₃, 300 K): δ 7.61 und 7.60 (2×*s*, Verhältnis ca. 3/1, 1 H, CH=N [Isomere]), 7.49 (2×*s*, Verhältnis ca. 3/1, 1 H, CH=N [Isomere]), 3.64 (*m*, 8 H, OCH₂^{cycl.}), 3.30 (*m*, 1 H, CH=N-C*H*^{cycl.}), 3.12 und 3.01 (2×*d*, *J* = 11.1, 2 H, CH=N-C*H*₂C^{cycl.}), 2.47 (*m*, 8 H, NCH₂^{cycl.}), 2.34 (*s*, 4 H, NC*H*₂C(CH₃)₂), 1.53-0.85 (*m*, 27 H, CH₂^{cycl.} und CH₃).

### Beispiel 5: Aldimin A-5

Unter gleichen Bedingungen wie in Beispiel 1 beschrieben wurden 6.25 g 1-Amino-3-aminomethyl-3,5,5-trimethylcyclohexan (Isophorondiamin oder IPDA; Vestamin® IPD, Degussa; Amingehalt 11.67 mmol N/g) mit 15.00 g 2,2-Dimethyl-3-(N-(2,6-dimethyl)morpholino)-propanal umgesetzt. Ausbeute: 19.7 g eines klaren, farblosen Öls mit einem Amingehalt von 7.33 mmol N/g und einer Viskosität von 10.5 Pa·s bei 20 °C.
IR: 2968, 2952, 2930, 2907, 2862, 2808, 2769sh, 2731, 2624, 1727, 1664 (C=N), 1456, 1396sh, 1374, 1363, 1320, 1280, 1248, 1230, 1216, 1178, 1144, 1082, 1040, 1000, 968, 939, 916, 878, 864, 838, 803, 774.
¹H-NMR (CDCl₃, 300 K): δ 7.61 und 7.48 (2×*m*, 2×1 H, CH=N), 3.62 (*m*, 4 H, OC*H*CH₃), 3.31 (*s*, ca. 0.5 H, CH=N-C*H*₂C^{cycl.} [Isomer]), 3.28 (*m*, 1 H, CH=N-C*H*^{cycl.}), 3.11 und 3.01 (2×*d*, *J* = 11.1, ca. 1.5 H, CH=N-C*H*₂C^{cycl.} [Isomer]), 2.62 (*d, J* = 10.0, 4 H von NC*H*₂^{cycl.}), 2.32 und 2.31 (2×*s*, 4 H, NC*H*₂C(CH₃)₂), 1.89 und 1.81 (2×*d*, *J* = 10.0, 4 H von NC*H*₂^{cycl.}), 1.6-0.8 (*m*, 39 H, CH₂^{cycl} und alle CH₃).

### Beispiel 6: Aldimin A-6

Unter gleichen Bedingungen wie in Beispiel 1 beschrieben wurden 6.07 g 1-Amino-3-aminomethyl-3,5,5-trimethylcyclohexan (Isophorondiamin oder IPDA; Vestamin® IPD, Degussa; Amingehalt 11.67 mmol N/g) mit 15.00 g 2,2-Dimethyl-3-(N-benzylmethylamino)-propanal umgesetzt. Ausbeute: 19.7 g eines klaren, farblosen Öls mit einem Amingehalt von 7.24 mmol N/g und einer Viskosität von 2.8 Pa·s bei 20 °C.
IR: 3084, 3060, 3026, 2948, 2916, 2864, 2838, 2806, 2782, 2708, 1944, 1871, 1807, 1726, 1664 (C=N), 1602, 1585, 1541, 1494, 1452, 1418, 1386, 1377, 1362, 1322, 1260, 1249sh, 1203sh, 1188, 1168, 1120, 1074, 1036, 1026, 1000, 974, 939sh, 904, 890, 858, 824, 736, 696, 670.
¹H-NMR (CDCl₃, 300 K): δ 7.63 und 7.50 (2×*s*, Verhältnis ca. 3/1, 1 H, CH=N [Isomere]), 7.49 (*m*, 1 H, CH=N), 7.34-7.18 (*m*, 10 Ph-H), 3.52 und 3.51 (2×*s*, 2×2 H, PhC*H*₂N), 3.30 (*s*, ca. 0.5 H, CH=N-C*H*₂C^{cycl.} [Isomer]), 3.27 (*m*, 1 H, CH=N-C*H*^{cycl.}), 3.10 und 2.99 (2×*d*, *J* = 11.1, ca. 1.5 H, CH=N-C*H*₂C^{cycl.} [Isomer]), 2.47 und 2.46 (2×*s*, 2×2 H, NC*H*₂C(CH₃)₂), 2.17 und 2.16 (2×*s*, 2×2 H, NCH₃), 1.6-0.8 (*m*, 27 H, CH₂^{cycl} und alle C-CH₃).

### Beispiel 7: Aldimin A-7

Unter gleichen Bedingungen wie in Beispiel 1 beschrieben wurden 5.34 g 1-Amino-3-aminomethyl-3,5,5-trimethylcyclohexan (Isophorondiamin oder IPDA; Vestamin® IPD, Degussa; Amingehalt 11.67 mmol N/g) mit 15.00 g 2,2-Dimethyl-3-(N-benzylisopropylamino)-propanal umgesetzt. Ausbeute: 19.2 g eines klaren, blassgelben Honigs mit einem Amingehalt von 6.56 mmol N/g und einer Viskosität von 150 Pa·s bei 20 °C.
IR: 3082, 3060, 3024, 2958, 2922, 2864, 2816, 2710, 1944, 1872, 1805, 1662 (C=N), 1602, 1585, 1494, 1460, 1452sh, 1386, 1362, 1320, 1299, 1240, 1205sh, 1164, 1116, 1092, 1074, 1054, 1026, 998, 966, 940, 890, 845, 826, 774, 728, 696.
¹H-NMR (CDCl₃, 300 K): δ 7.56 und 7.53 (2×*s*, Verhältnis ca. 3/1, 1 H, CH=N [Isomere]), 7.41 (*m*, 1 H, CH=N), 7.34-7.14 (*m*, 10 Ph-H), 3.59 und 3.57 (2×*s*, 2×2 H, PhC*H*₂N), 3.18 (*m*, 1 H, CH=N-C*H*^{cycl.}), 2.98 und 2.89 (2×*d*, *J* = 11.0, 2 H, CH=N-C*H*₂C^{cycl.}), 2.77 (*m*, 2 H, NC*H*(CH₃)₂), 2.44 (*s*, 4 H, NC*H*₂C(CH₃)₂), 1.6-0.8 (*m*, 39 H, CH₂^{cycl} und alle CH₃).

### Beispiel 8: Aldimin A-8

Unter gleichen Bedingungen wie in Beispiel 1 beschrieben wurden 6.31 g 1-Amino-3-aminomethyl-3,5,5-trimethylcyclohexan (Isophorondiamin oder IPDA; Vestamin® IPD, Degussa; Amingehalt 11.67 mmol N/g) mit 15.00 g 2,2-Dimethyl-3-(N-cyclohexylmethylamino)-propanal umgesetzt. Ausbeute: 19.6 g eines klaren, farblosen Honigs mit einem Amingehalt von 7.38 mmol N/g und einer Viskosität von 28 Pa·s bei 20 °C.
IR: 2924, 2850, 2807, 1726, 1664 (C=N), 1462sh, 1450, 1378, 1362, 1345sh, 1315sh, 1260, 1200, 1176, 1140, 1116, 1103sh, 1071sh, 1044, 1026, 1001sh, 986, 971sh, 940sh, 917, 890, 860, 836, 785.
¹H-NMR (CDCl₃, 300 K): δ 7.64 und 7.62 (2×*s*, Verhältnis ca. 3/1, 1 H, CH=N [Isomere]), 7.50 (*m*, 1 H, CH=N), 3.33 (*s*, ca. 0.5 H, CH=N-C*H*₂C^{cycl.} [Isomer]), 3.28 (*m*, 1 H, CH=N-C*H*^{cycl.}), 3.13 und 3.01 (2×*d*, *J* = 11.1, ca. 1.5 H, CH=N-C*H*₂C^{cycl.} [Isomer]), 2.40 (*s*, 4 H, NC*H*₂C(CH₃)₂), 2.23 (*m*, 8 H, N(C*H*₃)CH^{cycl.}), 1.74 (*m*, 8 Cy-H), 1.59 (*m*, 2 Cy-H), 1.4-0.9 (*m*, 37 H, Cy-H und alle C-CH₃).

### Beispiel 9: Aldimin A-9

Unter gleichen Bedingungen wie in Beispiel 1 beschrieben wurden 9.05 g 2-(2-Aminoethoxy)-ethanol (DGA; Diglycolamine® Agent, Huntsman; Amingehalt 9.39 mmol N/g) mit 15.00 g 2,2-Dimethyl-3-(N-morpholino)-propanal umgesetzt. Ausbeute: 22.5 g eines klaren, farblosen Öls mit einem Amingehalt von 7.61 mmol N/g und einer Viskosität von 130 mPa·s bei 20 °C. IR: 3415br (OH), 2953, 2919, 2887, 2850, 2805, 1665 (C=N), 1454, 1397, 1376, 1358, 1335, 1318, 1282, 1268, 1228br, 1206, 1115, 1068, 1036, 1012, 949, 927, 883, 863, 802.
¹H-NMR (CDCl₃, 300 K): δ 7.63 (*t*, *J* = 1.3, 1 H, CH=N), 3.75-3.55 (*m*, 12 H, OCH₂^{cycl.} und HOC*H*₂C*H*₂OC*H*₂C*H*₂N=CH), 2.47 (*m*, 4 H, NCH₂^{cycl.}), 2.43 (br *s*, 1 H, OH), 2.36 (*s*, 2 H, NC*H*₂C(CH₃)₂), 1.07 (*s*, 6 H, CH₂C(C*H*₃)₂).

### Beispiel 10: Aldimin A-10

Unter gleichen Bedingungen wie in Beispiel 1 beschrieben wurden 17.38 g N-Cyclohexyl-1,3-propandiamin (BASF; Amingehalt 12.84 mmol N/g) mit 20.00 g 2,2-Dimethyl-3-(N-morpholino)-propanal umgesetzt. Ausbeute: 35.3 g eines klaren, farblosen Öls mit einem Amingehalt von 9.56 mmol N/g und einer Viskosität von 52 mPa·s bei 20 °C.
IR: 3304br (NH), 2952sh, 2924, 2849, 2808, 2687, 1726, 1664 (C=N), 1450, 1396, 1376sh, 1360, 1349sh, 1332, 1317, 1282, 1267, 1204, 1134, 1116, 1070, 1036, 1012, 932br, 886, 864, 846, 802, 735br.

### Beispiel 11: Aldimin A-11

In einem Birnkölbchen wurden 2.15 g (7.1 mmol) N-(2,2-Dimethyl-3-oxopropyl)-N-methyl-pyrrolidiniumiodid unter Stickstoffatmosphäre in ca. 2 ml Wasser gelöst und die Lösung unter Rühren mit 0.58 g (3.5 mmol) 1,6-Hexamethylendiamin (70% in Wasser; Amingehalt 12.16 mmol N/g) versetzt. Anschliessend wurde die entstandene klare, farblose Lösung im Ölbad erwärmt und die flüchtigen Bestandteile im Vakuum entfernt (10 mbar, 80 °C). Man erhielt ein blassgelbes, klares, glasartiges Produkt.

### Alternative Herstellung:

Unter Stickstoffatmosphäre wurden 1.00 g Aldimin ***A-1*** eingewogen und mit 0.73 g (5.1 mmol) Methyliodid in ein Pillenglas eingewogen, dieses verschlossen und gut umgeschwenkt. Die Mischung trübte sofort schwach ein; nach wenigen Minuten begann sich am Glasboden ein Niederschlag in Form eines blassgelben Öls zu sammeln. Das Pillenglas wurde eine Stunde bei Raumtemperatur stehen lassen und dann auf 60 °C erwärmt. Nach 2 Stunden war der gesamte Inhalt zu einem hellgelben, glasartigen Öl erstarrt.
IR: 2962, 2928, 2852, 2834sh, 2780sh, 1724 (C=O), 1664 (C=N), 1460, 1386, 1366, 1342, 1306, 1262, 1222, 1168, 1142, 1114, 1098, 1042, 1002, 970, 942, 928, 904, 890, 824, 789, 730.
¹H-NMR (D₂O, 300 K): δ 7.88 (*s*, 2 H, CH=N), 3.68 und 3.51 (2×*m*, 8 H und 4 H, ^{cycl.}CH₂C*H*_{2N}(CH₃)C*H*₂C(CH₃)₂), 3.48 (*t*, *J* = 7.0, 4 H, CH=N-C*H*₂), 2.97 (*s*, 6 H, NCH₃), 2.20 (br *m*, 8 H, ^{cycl.}C*H*₂CH₂N), 1.59 (*m,* 4 H, CH=N-CH₂C*H*₂), 1.31 (*m*, 16 H, CH=N-CH₂CH₂C*H*₂ und CH₂C(C*H*₃)₂).

### Vergleichsbeispiel 12: Aldimin A-12

In einem Rundkolben wurden unter Stickstoffatmosphäre 50.9 g (0.18 mol) destilliertes 2,2-Dimethyl-3-lauroyloxy-propanal vorgelegt. Unter kräftigem Rühren wurden aus einem beheizten Eintropftrichter 10.0 g (0.17 mol N) 1,6-Hexamethylendiamin (BASF; Amingehalt 17.04 mmol N/g) langsam zugegeben, wobei sich die Mischung erwärmte und zunehmend eintrübte. Danach wurden die flüchtigen Bestandteile im Vakuum entfernt (10 mbar, 80 °C). Ausbeute: 57.7 g eines klaren, blassgelben Öls mit einem Amingehalt von 2.94 mmol N/g.

### Vergleichsbeispiel 13: Aldimin A-13

Unter gleichen Bedingungen wie in Beispiel 12 beschrieben wurden 74.3 g (0.26 mol) destilliertes 2,2-Dimethyl-3-lauroyloxy-propanal mit 30.0 g (0.25 mol N) Polyetherdiamin (Polyoxypropylen-Diamin mit einem mittleren Molekulargewicht von ca. 240 g/mol; Jeffamine® D-230, Huntsman; Amingehalt 8.29 mmol N/g) umgesetzt. Ausbeute: 99.5 g eines klaren, blassgelben Öls mit einem Amingehalt von 2.50 mmol N/g.

### Vergleichsbeispiel 14: Aldimin A-14

Unter gleichen Bedingungen wie in Beispiel 12 beschrieben wurden 55.0 g (0.19 mol) destilliertes 2,2-Dimethyl-3-lauroyloxy-propanal mit 15.6 g (0.18 mol N) 1-Amino-3-aminomethyl-3,5,5-trimethylcyclohexan (Isophorondiamin oder IPDA; Vestamin® IPD, Degussa; Amingehalt 11.67 mmol N/g) umgesetzt. Ausbeute: 67.1 g eines klaren, farblosen Öls mit einem Amingehalt von 2.73 mmol N/g.

### 4. Herstellung einer Verbindung AV1

### Beispiel 15: Aldimin A-15

In einer ersten Stufe wurden 590 g Polyol Acclaim® 4200 N (Polypropylenoxid-Diol, OH-Zahl 28.5 mg KOH/g; Bayer), 1180 g Polyol Caradol® MD34-02 (Polypropylenoxidpolyethylenoxid-Triol, OH-Zahl 35.0 mg KOH/g; Shell) und 230 g Isophorondiisocyanat (IPDI; Vestanat® IPDI, Degussa) nach bekanntem Verfahren bei 80 °C zu einem NCO-terminierten Polyurethanpolymeren mit einem titrimetrisch bestimmten Gehalt an freien Isocyanat-Gruppen von 2.1 Gewichts-% und einer Viskosität bei 20 °C von 22 Pa·s umgesetzt.

Zu 10.00 g dieses Polyurethanpolymers (5.0 mEquiv. NCO) wurden bei Raumtemperatur 1.31 g (5.0 mmol) Aldimin ***A-9*** aus Beispiel 9 gegeben, die Mischung mittels eines Zentrifugalmischers (SpeedMixer™ DAC 150, FlackTek Inc.) innig vermengt und dann auf 60 °C erwärmt. Nach 24 Stunden war die NCO-Bande im FT-IR-Spektrum (bei 2265 cm⁻¹) nicht mehr nachweisbar. Man erhielt eine klare, homogene und geruchlose Flüssigkeit mit einem AminGehalt von 0.44 mmol N/g und einer Viskosität bei 20 °C von 110 Pa·s.
IR: 3340br (NH), 2967, 2929, 2893sh, 2864, 1720 (C=O), 1666 (C=N), 1529, 1454,1372,1344,1299,1282,1239, 1094, 1013, 925, 908sh, 865, 835, 775, 660.

### 5. Herstellung von härtbaren Zusammensetzungen

### Beispiele 16 bis 20 und Vergleichsbeispiel 21:

In einem Polypropylenbecher mit Schraubverschluss wurden das Polyurethanpolymer *1*, dessen Herstellung nachfolgend beschrieben wird, mittels eines Zentrifugalmischers (SpeedMixer™ DAC 150, FlackTek Inc.; 1 min. bei 2500 U/min) mit einem Aldimin sowie mit Katalysatoren zu einer homogenen Masse vermischt, die so erhaltene Masse sofort in eine innenlackierte Aluminiumtube abgefüllt und diese luftdicht verschlossen. Die eingesetzten Aldimine und die Katalysator-Typen für jedes der Beispiele sind in der Tabelle 1 in Gewichtsteilen aufgeführt.

Das Polyurethanpolymer *1* wurde wie folgt hergestellt:
590 g Polyol Acclaim® 4200 N (Polypropylenoxid-Diol, OH-Zahl 28.5 mg KOH/g; Bayer), 1180 g Polyol Caradol® MD34-02 (Polypropylenoxidpolyethylenoxid-Triol, OH-Zahl 35.0 mg KOH/g; Shell) und 230 g Isophorondiisocyanat (IPDI; Vestanat® IPDI, Degussa) wurden nach bekanntem Verfahren bei 80 °C zu einem NCO-terminierten Polyurethanpolymeren mit einem titrimetrisch bestimmten Gehalt an freien Isocyanat-Gruppen von 2.1 Gewichts-% und einer Viskosität bei 20 °C von 22 Pa·s umgesetzt.

Das Verhältnis zwischen den Isocyanatgruppen und den blockieten Aminogruppen beträgt für alle Beispiele 1.0 / 0.7.

**Tabelle 1: Zusammensetzung der Beispiele 16 bis 20 und des Vergleichsbeispiels 21.**

| Beispiel | **16** | **17** | **18** | **19** | **20** | **21 (Vgl)** |
|---|---|---|---|---|---|---|
| Polyurethanpolymer *1* | 50.0 | 50.0 | 50.0 | 50.0 | 50.0 | 50.0 |
| Aldimin | ***A-4**,* 4.21 | ***A-5**,* 4.79 | ***A-6**,* 4.89 | ***A-7**,* 5.40 | ***A-8**,* 4.74 | - |
| Säure-Katalysator^{a} | 0.05 | 0.05 | 0.05 | 0.05 | 0.05 | - |
| Zinn-Katalysator^{b} | 0.05 | 0.05 | 0.05 | 0.05 | 0.05 | 0.05 |

| | | | | | | |
|---|---|---|---|---|---|---|
| ^{a} Salicylsäure. ^{b} 5 Gew.-% Dibutylzinndilaurat in Diisodecylphthalat. | | | | | | |

Die so erhaltenen Zusammensetzungen wurden auf Lagerstabilität, Hautbildungszeit sowie auf mechanische Eigenschaften nach der Aushärtung geprüft.

Die **Lagerstabilität** wurde über die Veränderung der Viskosität während der Lagerung in der Wärme bestimmt. Dazu wurde die Zusammensetzung in der verschlossenen Tube im Ofen bei 60 °C gelagert und die Viskosität bei 20 °C ein erstes Mal nach 4 Stunden und ein zweites Mal nach 7 Tagen Lagerdauer gemessen. Die Lagerstabilität ergibt sich aus der prozentualen Zunahme des zweiten Viskositätswerts gegenüber dem ersten.

Zur Messung der **Hautbildungszeit** (Zeit bis zur Klebefreiheit, "tack-free time") wurde ein kleiner Teil der während 4 Stunden bei 60 °C gelagerten Zusammensetzung in einer Schichtdicke von ca. 2 mm auf Pappkarton aufgetragen und im Normklima (23±1 °C, 50±5% relative Luftfeuchtigkeit) die Zeit bestimmt, die es dauerte, bis beim leichten Antippen der Oberfläche der Zusammensetzung mittels einer Pipette aus LDPE erstmals keine Rückstände auf der Pipette mehr zurückblieben.

Zur Bestimmung der **mechanischen Eigenschaften** wurde mit dem Hauptteil der Zusammensetzung Filme von ca. 3 mm Dicke hergestellt, indem die Zusammensetzung in eine plane PTFE-Form gegossen und während 7 Tagen im Normklima ausgehärtet wurde. Es wurden klare, klebefreie und elastische Polyurethanfilme erhalten, welche vollständig blasenfrei waren. Aus den Filmen wurden Hanteln mit einer Länge von 75 mm, bei einer Steglänge von 30 mm und einer Stegbreite von 4 mm, ausgestanzt und diese gemäss DIN EN 53504 auf **Zugfestigkeit, Bruchdehnung** und **E-Modul** (bei 0.5-5% Dehnung) geprüft (Zuggeschwindigkeit: 200 mm/min).

Die Ergebnisse dieser Prüfungen sind in der Tabelle 2 aufgeführt.

**Tabelle 2: Eigenschaften der Beispiele 16 bis 20 und des Vergleichsbeispiels 21.**

| Beispiel | **16** | **17** | **18** | **19** | **20** | **21 (Vgl)** |
|---|---|---|---|---|---|---|
| Viskosität nach 4h (Pa·s) | 21.8 | 21.0 | 21.5 | 23.1 | 24.5 | 19.0 |
| Viskosität nach 7d (Pa·s) | 23.6 | 21.7 | 21.7 | 23.8 | 26.5 | 19.9 |
| Viskositätszunahme (%)^{a} | 8 | 3 | 1 | 3 | 8 | 5 |
| Hautbildungszeit (min.) | 60 | 65 | 70 | 75 | 60 | >480 |
| Zugfestigkeit (MPa) | 4.1 | 3.9 | 3.4 | 3.6 | 3.6 | n.b.^{b} |
| Bruchdehnung (%) | 520 | 500 | 510 | 530 | 590 | n.b.^{b} |
| E-Modul (MPa) | 1.7 | 1.3 | 1.3 | 1.4 | 1.2 | n.b.^{b} |

| | | | | | | |
|---|---|---|---|---|---|---|
| ^{a} = ((Viskosität nach 7d /Viskosität nach 4h)-1)×100%. ^{b}nicht bestimmt. | | | | | | |

### Beispiele 22 bis 27:

### Elastische 1K-Dichtstoffe (z.B. für Dilatationsfugen)

Für jedes Beispiel wurden die jeweiligen Bestandteile gemäss Tabelle 3 in den angegebenen Gewichtsteilen ohne vorherige Trocknung in einem Vakuummischer unter Feuchtigkeitsausschluss zu einer knollenfreien, homogenen Paste verarbeitet, diese unverzüglich in eine innenlackierte Aluminium-Kartusche abgefüllt und die Kartusche luftdicht verschlossen.

Das Polyurethanpolymer *1* wurde wie bei Beispiel 16 beschrieben hergestellt.

Das Harnstoff-Verdickungsmittel wurde wie folgt hergestellt:
In einem Vakuummischer wurden 3000 g Diisodecylphthalat (DIDP; Palatinol® Z, BASF) und 480 g 4,4'-Methylendiphenyldiisocyanat (MDI; Desmodur® 44 MC L, Bayer) vorgelegt und leicht aufgewärmt. Dann wurden unter starkem Rühren 270 g Monobutylamin langsam zugetropft. Die entstehende Paste wurde unter Vakuum und Kühlung eine Stunde weitergerührt.

Das Verhältnis zwischen den Isocyanatgruppen und der Summe der blockierten Aminogruppen (wobei Oxazolidinogruppen doppelt gezählt sind) beträgt für alle Beispiele 1.0 / 0.67.

**Tabelle 3: Zusammensetzung der elastischen 1K-PUR-Dichtstoffe.**

| Beispiel | **22** | **23** | **24 (Vgl)** | **25 (Vgl)** | **26 (Vgl)** | **27 (Vgl)** |
|---|---|---|---|---|---|---|
| Polyurethanpolymer *1* | 24.0 | 24.0 | 24.0 | 24.0 | 24.0 | 24.0 |
| Aldimin | ***A-3**,* 2.25 | ***A-4**,* 1.93 | ***A-13**,* 3.22 | ***A-14**,* 2.95 | - | - |
| Oxazolidin^{a} | - | - | - | - | 0.99 | - |
| Weichmacher^{b} | 2.15 | 2.47 | 1.18 | 1.45 | 3.41 | 4.7 |
| Kreide | 38.0 | 38.0 | 38.0 | 38.0 | 38.0 | 38.0 |
| Verdickungsmittel^{c} | 28.0 | 28.0 | 28.0 | 28.0 | 28.0 | 28.0 |
| Titandioxid | 4.5 | 4.5 | 4.5 | 4.5 | 4.5 | 4.5 |
| Epoxysilan^{d} | 0.2 | 0.2 | 0.2 | 0.2 | 0.2 | 0.2 |
| Säure-Katalysator^{e} | 0.6 | 0.6 | 0.6 | 0.6 | 0.6 | - |
| Zinn-Katalysator^{f} | 0.3 | 0.3 | 0.3 | 0.3 | 0.3 | 0.6 |

| | | | | | | |
|---|---|---|---|---|---|---|
| ^{a} Härter OZ, Bayer; Amingehalt = 4.05 mmol/g. ^{b}Diisodecylphthalat (DIDP; Palatinol® Z, BASF). ^{c} Harnstoff-Verdickungsmittel. ^{d} 3-Glycidoxypropyltriethoxysilan (Dynasylan® GLYEO, Degussa). ^{e} Salicylsäure (5 Gew.-% in Dioctyladipat). ^{f} Dibutylzinndilaurat (5 Gew.-% in Diisodecylphthalat). | | | | | | |

Die so erhaltenen Dichtstoffe wurden auf Hautbildungszeit, Durchhärtungsgeschwindigkeit, Klebrigkeit, Staining und mechanische Eigenschaften nach der Aushärtung geprüft.

Die **Hautbildungszeit** wurde bestimmt wie bei Beispiel 16 beschrieben.

Die **Durchhärtungsgeschwindigkeit** wurde bestimmt, indem der Dichtstoff mittels Kartuschenpistole durch eine Rundspitze (Öffnung 10 mm) als waagrechter, frei hängender Konus mit einer Länge von ca. 50 mm und einer Dicke in der Mitte von 30 mm auf ein an der Wand befestigtes Stück Pappkarton aufgetragen wurde, während 7 Tagen im Normklima belassen, dann vertikal mittig aufgeschnitten und die Dicke der ausgehärteten Schicht mit einem Massstab gemessen wurde.

Die **Klebrigkeit** wurde durch Einpressen des Daumens auf den während einem bzw. 3 Tagen gelagerten Shore A-Prüfkörper mit dem Finger festgestellt und rein qualitativ danach bewertet, wie lange der Prüfkörper beim Anheben der Hand am Daumen kleben blieb (hoch/mittel/gering/keine).

Das **Staining** wurde anhand der Grösse (gross/mittel/klein/keines) des Fettrings beurteilt, der sich eine Woche nach der Applikation eines Shore A-Prüfkörpers auf einem Blatt Papier gebildet hatte, auf welchem der Prüfkörper frisch aufgebracht worden war.

Zur Bestimmung von **mechanischen Eigenschaften** nach der Aushärtung wurden die Shore A-Härte, die Zugfestigkeit, Bruchdehnung und die Dehnspannung bei 100% gemessen. Die **Shore A-Härte** wurde bestimmt nach DIN 53505 an während 14 Tagen im Normklima ausgehärteten Prüfkörpern. Zur Prüfung der weiteren mechanischen Eigenschaften wurde der Dichtstoff 2 Stunden nach der Herstellung mittels einer Presse zu einem Film von ca. 2 mm Dicke gepresst, der Film während 14 Tagen im Normklima ausgehärtet und nach DIN EN 53504 auf **Zugfestigkeit**, **Bruchdehnung** und **Dehnspannung bei 100%** (Zuggeschwindigkeit: 200 mm/min) geprüft.

Die Ergebnisse der Prüfungen sind in der Tabelle 4 aufgeführt.

**Tabelle 4: Eigenschaften der elastischen 1K-PUR-Dichtstoffe.**

| Beispiel | **22** | **23** | **24 (Vgl)** | **25 (Vgl)** | **26 (Vgl)** | **27 (Vgl)** |
|---|---|---|---|---|---|---|
| Hautbildungszeit (min.) | 50 | 90 | 165 | 245 | 90 | >480 |
| Durchhärtung (mm) | 10 | 8 | 6 | 6 | 15 | 8* |
| Klebrigkeit nach 1 Tag | mittel | keine | hoch | gering | hoch | hoch |
| Klebrigkeit nach 3 Tagen | mittel | keine | hoch | keine | mittel | hoch |
| Staining | keines | keines | mittel | mittel | mittel | gross |
| Shore A-Härte | 28 | 36 | 21 | 31 | 35 | 27 |
| Zugfestigkeit (MPa) | 2.7 | 3.0 | 2.5 | 2.6 | 1.4 | 1.9 |
| Bruchdehnung (%) | 1000 | 930 | 1040 | 1000 | 530 | 1000 |
| Dehnspannung bei 100% Dehnung (MPa) | 0.5 | 0.6 | 0.4 | 0.4 | 0.6 | 0.4 |

| | | | | | | |
|---|---|---|---|---|---|---|
| * deutliche Blasenbildung. | | | | | | |

### Beispiele 28 und Vergleichsbeispiele 29 bis 31:

### Elastische 1K-Klebstoffe (z.B. für Konstruktion, Montage)

Für jedes Beispiel wurden die jeweiligen Bestandteile gemäss Tabelle 5 in den angegebenen Gewichtsteilen ohne vorherige Trocknung in einem Vakuummischer unter Feuchtigkeitsausschluss zu einer knollenfreien, homogenen Paste verarbeitet, diese unverzüglich in eine innenlackierte Aluminium-Kartusche abgefüllt und die Kartusche luftdicht verschlossen.

Das Polyurethanpolymer *1* bzw. das Harnstoff-Verdickungsmittel wurden wie bei Beispiel 16 bzw. bei Beispiel 22 beschrieben hergestellt.

Das Verhältnis zwischen den Isocyanatgruppen und der Summe der blockierten Aminogruppen (wobei Oxazolidinogruppen doppelt gezählt sind) beträgt für alle Beispiele 1.0 / 0.52.

**Tabelle 5: Zusammensetzung der elastischen 1K-PUR-Klebstoffe.**

| Beispiel | **28** | **29 (Vgl)** | **30 (Vgl)** | **31 (Vgl)** |
|---|---|---|---|---|
| Polyurethanpolymer *1* | 35.0 | 35.0 | 35.0 | 35.0 |
| Aldimin | ***A-4**,* 2.73 | ***A-14**,* 4.17 | - | - |
| Oxazolidin^{a} | - | - | 1.40 | - |
| Vernetzer^{b} | 0.8 | 0.8 | 0.8 | 0.8 |
| Kreide | 27.0 | 27.0 | 27.0 | 27.0 |
| Verdickungsmittel^{c} | 28.27 | 26.83 | 29.60 | 31.50 |
| Titandioxid | 4.5 | 4.5 | 4.5 | 4.5 |
| Epoxysilan^{d} | 0.2 | 0.2 | 0.2 | 0.2 |
| Säure-Katalysator^{e} | 1.0 | 1.0 | 1.0 | - |
| Zinn-Katalysator^{f} | 0.5 | 0.5 | 0.5 | 1.0 |

| | | | | |
|---|---|---|---|---|
| ^{a} Härter OZ, Bayer; Amingehalt = 4.05 mmol/g. ^{b} HDI-Trimerisat (Desmodur® N-3600, Bayer), NCO-Gehalt = 23.0 Gew.-%. ^{c} Harnstoff-Verdickungsmittel. ^{d} 3-Glycidoxypropyltriethoxysilan (Dynasylan® GLYEO, Degussa). ^{e} Salicylsäure (5 Gew.-% in Dioctyladipat). ^{f} Dibutylzinndilaurat (5 Gew.-% in Diisodecylphthalat). | | | | |

Die so erhaltenen Klebstoffe wurden auf Hautbildungszeit, Durchhärtungsgeschwindigkeit, Staining und mechanische Eigenschaften nach Aushärtung geprüft wie bei Beispiel 22 beschrieben, wobei statt der Spannung bei 100% Dehnspannung das **E-Modul** (bei 0.5-5% Dehnung) angegeben ist. Die Ergebnisse der Prüfungen sind in der Tabelle 6 aufgeführt.

**Tabelle 6: Eigenschaften der elastischen 1K-PUR-Klebstoffe.**

| Beispiel | **28** | **29 (Vgl)** | **30 (Vgl)** | **31 (Vgl)** |
|---|---|---|---|---|
| Hautbildungszeit (min.) | 70 | 190 | 50 | >480 |
| Durchhärtung (mm) | 8 | 8 | 12 | 9 |
| Staining | keines | klein | klein | gross |
| Shore A-Härte | 43 | 43 | 43 | 45 |
| Zugfestigkeit (MPa) | 2.5 | 2.2 | 1.4 | n.m.* |
| Bruchdehnung (%) | 620 | 640 | 320 | n.m.* |
| E-Modul (MPa) | 3.7 | 3.0 | 3.7 | n.m.* |

| | | | | |
|---|---|---|---|---|
| * nicht messbar wegen starker Blasenbildung. | | | | |

### Beispiele 32 bis 37 und Vergleichsbeispiele 38 bis 40: 2K-Vergussmassen

Für jedes Beispiel wurden die jeweiligen Bestandteile der Komponente *K2* gemäss Tabelle 7 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt.

**Tabelle 7: Zusammensetzung derzweikomponentigen PUR-Vergussmassen.**

| Beispiel | **32** | **33** | **34** | **35** | **36** | **37** | **38 (Vgl)** | **39 (Vgl)** | **40 (Vgl.)** |
|---|---|---|---|---|---|---|---|---|---|
| **Komp. *K1*:** | | | | | | | | | |
| PMDI^{a} | 30.4 | 30.8 | 29.7 | 30.2 | 32.8 | 31.8 | 29.2 | 28.9 | 29.7 |

| **Komp. *K2*:** | | | | | | | | | |
|---|---|---|---|---|---|---|---|---|---|
| Ricinusöl^{b} | 22.5 | 22.5 | 22.5 | 22.5 | 22.5 | 22.5 | 22.5 | 22.5 | 22.5 |
| Dimerfettsäurediol^{c} | 17.5 | 17.5 | 17.5 | 17.5 | 17.5 | 17.5 | 17.5 | 17.5 | 22.5 |
| Triol^{d} | 4.75 | 4.75 | 4.75 | 4.75 | 4.75 | 4.75 | 4.75 | 4.75 | 4.75 |
| Aldimin | ***A-1**,* 5.0 | ***A-2**,* 5.0 | ***A-3**,* 5.0 | ***A-4**,* 5.0 | ***A-9**,* 5.0 | ***A-10**,* 5.0 | ***A-12**,* 5.0 | ***A-13**,* 5.0 | - |
| Säure-Katalysator^{e} | 0.25 | 0.25 | 0.25 | 0.25 | 0.25 | 0.25 | 0.25 | 0.25 | 0.25 |
| Kreide^{f} | 50.0 | 50.0 | 50.0 | 50.0 | 50.0 | 50.0 | 50.0 | 50.0 | 50.0 |

| | | | | | | | | | |
|---|---|---|---|---|---|---|---|---|---|
| ^{a} Desmodur® VKS 20 F, Bayer; NCO-Gehalt = 30.0 Gew.-%. ^{b} OH-Zahl = 165 mg KOH/g. ^{c} Sovermol® 908, Cognis ;OH-Zahl = 200 mg KOH/g. ^{d} Desmophen® 4011 T, Bayer; OH-Zahl = 550 mg KOH/g. ^{e} Salicylsäure (5 Gew.-% in Dioctyladipat). ^{f} Omyacarb® 5-GU, Omya. | | | | | | | | | |

Dazu wurden die in der Tabelle 7 angegebenen Gewichtsteile an PMDI als Komponente *K1* zugegeben und eingemischt (30 sec. bei 3000 U/min). Das Verhältnis zwischen den Isocyanatgruppen der Komponente *K1* und der Summe der Reaktivgruppen (Hydroxyl- und Aldiminogruppen) der Komponente *K2* beträgt stets 1.1.

Die so erhaltenen zweikomponentigen Polyurethan-Vergussmassen wurden auf Aushärtegeschwindigkeit, mechanische Eigenschaften und Blasenbildung geprüft.

**Tabelle 8: Eigenschaften derzweikomponentigen PUR-Vergussmassen.**

| Beispiel | **32** | **33** | **34** | **35** | **36** | **37** | **38 (Vgl)** | **39 (Vgl)** | **40 (Vgl)** |
|---|---|---|---|---|---|---|---|---|---|
| Klebefreiheit (min.)^{a} | 10 | 20 | 30 | 22 | 30 | 38 | 38 | 58 | 48 |
| Shore D nach 1 Tag | 81 | 84 | 81 | 82 | 88 | 77 | 58 | 60 | 60 |
| Shore D nach 3 Tagen | 87 | 93 | 90 | 87 | 92 | 92 | 72 | 73 | 75 |
| Shore D nach 7 Tagen | 91 | 94 | 93 | 91 | 96 | 94 | 83 | 84 | 82 |
| Shore D getempert^{b} | 88 | 94 | 94 | 91 | 96 | 96 | 89 | 86 | 85 |
| Zugfestigkeit (MPa) | 21.9 | 31.2 | 23.2 | 20.2 | 32.3 | 22.7 | 11.0 | 11.0 | 8.1 |
| Bruchdehnung (%) | 3 | 6 | 16 | 20 | 5 | 5 | 65 | 75 | 60 |
| E-Modul (MPa) | 610 | 740 | 570 | 505 | 830 | 640 | 100 | 85 | 100 |
| Blasenbildung | keine | keine | keine | keine | keine | keine | keine | keine | viele |

| | | | | | | | | | |
|---|---|---|---|---|---|---|---|---|---|
| ^{a} Zeit bis zur Klebefreiheit (tack-free time). ^{b} 4 h bei 105°C der während 7 Tagen im Normklima ausgehärteten Prüfkörper. | | | | | | | | | |

Hinweise zur Aushärtegeschwindigkeit wurden zum einen durch Messen der **Zeit bis zur Klebefreiheit** erhalten, welche gleich wie die Hautbildungszeit gemessen wurde wie in Beispiel 16 beschrieben. Zum anderen wurde der weitere Verlauf der Aushärtung durch periodisches Messen der **Shore D-Härte** nach DIN 53505 verfolgt.

Die Messung der **Zugfestigkeit, Bruchdehnung** und **E-Modul** (bei 0.5-3% Dehnung) erfolgte nach DIN EN 53504 an gegossenen Filmen von ca. 2 mm Dicke, welche während 7 Tagen im Normklima ausgehärtet wurden (Zuggeschwindigkeit: 10 mm/min).

Die **Blasenbildung** wurde qualitativ beurteilt anhand der Menge Blasen, die bei der Aushärtung eines Films mit einer Schichtdicke von 2 mm im Normklima auftraten.

Die Ergebnisse dieser Prüfungen sind in Tabelle 8 aufgeführt.

### Beispiele 41 bis 43: Semistrukturelle 2K-Klebstoffe

Für jedes Beispiel wurden die jeweiligen Bestandteile der Komponente *K2* gemäss Tabelle 9 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 2 min. bei 3000 U/min) zu einer homogenen Creme gemischt. Dazu wurden die in Tabelle 9 angegebenen Gewichtsteile an PMDI als Komponente *K1* zugegeben und eingemischt (30 sec. bei 3000 U/min).

**Tabelle 9: Zusammensetzung der semistrukturellen 2K-PUR-Klebstoffe.**

| Beispiel | **41** | **42** | **43** |
|---|---|---|---|
| **Komponente *K1*:** | | | |
| PMDI^{a} | 25.0 | 25.4 | 25.0 |

| K**omponente *K2*:** | | | |
|---|---|---|---|
| Ricinusöl^{b} | 22.5 | 22.4 | 22.4 |
| Dimerfettsäurediol^{c} | 7.5 | - | - |
| PPG 1000^{d} | 12.5 | 22.4 | 22.4 |
| Triol^{e} | 2.25 | 2.25 | 2.25 |
| Aldimin | ***A-2**,* 5.0 | ***A-4**,* 5.0 | ***A-9**,* 5.0 |
| Säure-Katalysator^{f} | 0.25 | 0.25 | 0.25 |
| Kreide^{g} | 50.0 | 50.0 | 50.0 |

| | | | |
|---|---|---|---|
| ^{a} Desmodur® VKS 20 F, Bayer; NCO-Gehalt = 30.0 Gew.-%. ^{b} OH-Zahl = 165 mg KOH/g. ^{c} Sovermol^{®} 908, Cognis; OH-Zahl = 200 mg KOH/g. d Desmophen® 1112 BD, Bayer; OH-Zahl = 112 mg KOH/g. ^{e} Desmophen® 4011 T, Bayer; OH-Zahl = 550 mg KOH/g. ^{f} Salicylsäure (5 Gew.-% in Dioctyladipat). ⁹ Omyacarb® 5-GU, Omya. | | | |

Das Verhältnis zwischen den Isocyanatgruppen der Komponente *K1* und der Summe der Reaktivgruppen (Hydroxyl- und Aldiminogruppen) der Komponente *K2* beträgt stets 1.1.

Die so erhaltenen zweikomponentigen Polyurethan-Klebstoffe wurden auf Aushärtegeschwindigkeit, mechanische Eigenschaften und Blasenbildung geprüft wie bei Beispiel 32 beschrieben. Die Ergebnisse der Prüfungen sind in Tabelle 10 aufgeführt.

**Tabelle 10: Eigenschaften der semistrukturellen 2K-PUR-Klebstoffe.**

| Beispiel | **41** | **42** | **43** |
|---|---|---|---|
| Klebefreiheit (min.)^{a} | 23 | 37 | 35 |
| Shore D nach 1 Tag | 54 | 49 | 56 |
| Shore D nach 3 Tagen | 74 | 72 | 71 |
| Shore D nach 7 Tagen | 79 | 78 | 81 |
| Shore D getempert^{b} | 72 | 74 | 75 |
| Zugfestigkeit (MPa) | 10.2 | 9.5 | 10.2 |
| Bruchdehnung (%) | 53 | 86 | 64 |
| E-Modul (MPa) | 85 | 49 | 91 |
| Blasenbildung | keine | keine | keine |

| | | | |
|---|---|---|---|
| ^{a} Zeit bis zur Klebefreiheit (tack-free time) in Minuten. ^{b}4 h bei 105°C der während 7 Tagen im Normklima ausgehärteten Prüfkörper. | | | |

### Beispiele 44 bis 45 und Vergleichsbeispiel 46:

### Elastische 1K-Beschichtungen (z.B. für Bodenbelag)

Für jedes Beispiel wurden die jeweiligen Bestandteile der Beschichtungsmasse gemäss Tabelle 11 in den angegebenen Gewichtsteilen ohne vorherige Trocknung in einen Polypropylenbecher mit Schraubverschluss eingewogen und mittels eines Zentrifugalmischers (SpeedMixer™ DAC 150, FlackTek Inc.; 1 min. bei 2500 U/min) gemischt, die Mischung sofort in eine innenlackierte Aluminiumtube abgefüllt und diese luftdicht verschlossen. Das jeweilige Verhältnis zwischen den Aldiminogruppen und den Isocyanatgruppen (Aldimin/NCO-Verhältnis) ist in der Tabelle 11 angegeben.

Das Polyurethanpolymer *2* wurde wie folgt hergestellt:
1060 g Polyoxypropylen-Diol (Desmophen® 1111 BD, Bayer; OH-Zahl 111.4 mg KOH/g), 650 g Polyoxypropylen-Diol (Desmophen® 2061 BD, Bayer; OH-Zahl 56.1 mg KOH/g), 770 g Isophorondiisocyanat (Vestanat® IPDI, Degussa) und 0.25 g Dibutylzinndilaurat wurden bei 80 °C zu einem NCO-terminierten Polyurethanpolymeren mit einem Gehalt an freien Isocyanat-Gruppen von 6.8 Gewichts-% umgesetzt.

**Tabelle 11: Zusammensetzung dereinkomponentigen PUR-Beschichtungsmassen.**

| Beispiel | **44** | **45** | **46 (Vgl)** |
|---|---|---|---|
| Polyurethanpolymer *2* | 50.0 | 50.0 | 50.0 |
| IPDI- Trimerisat^{a} | 24.0 | 24.0 | 24.0 |
| Aldimin | ***A-2**,* 21.0 | ***A-4**,* 21.0 | ***A-12**,* 21.0 |
| Säure-Katalysator^{b} | 1.5 | 1.5 | 1.5 |
| Aminkatalysator^{c} | 0.75 | 0.75 | 0.75 |
| Zinn-Katalysator^{d} | 0.75 | 0.75 | 0.75 |
| Entschäumer^{e} | 2.0 | 2.0 | 2.0 |
| Aldimin/NCO-Verhältnis | 0.75 | 0.70 | 0.49 |

| | | | |
|---|---|---|---|
| ^{a} 45 Gew.-% IPDI-Trimerisat (Vestanat® T 1890/100, Degussa; NCO-Gehalt = 17.3 Gew.-%) in Xylol. ^{b}Salicylsäure (5 Gew.-% in Dioctyladipat). ^{c} 2,2'-Dimorpholinodiethylether (DABCO® DMDEE Catalyst, Air Products). ^{d} 10 Gew.-% Dibutylzinndilaurat in Diisodecylphthalat. ^{e} BYK-088 (BYK-Chemie/ALTANA). | | | |

Die so erhaltenen einkomponentigen Polyurethan-Beschichtungsmassen wurden auf Lagerstabilität, Hautbildungszeit, mechanische Eigenschaften, Blasenbildung und Geruchsbildung geprüft.

Die **Lagerstabilität** wurde durch Vergleich der Viskosität der Beschichtungsmasse vor und nach deren Lagerung bei erhöhter Temperatur bestimmt. Dazu wurde die Beschichtungsmasse in der verschlossenen Tube im Ofen bei 40 °C gelagert und ihre Viskosität ein erstes Mal 2 Stunden nach der Herstellung, ein zweites Mal nach 24 Tagen Lagerdauer gemessen.

Die **Hautbildungszeit** wurde wie bei Beispiel 16 beschrieben gemessen. Die **mechanischen Eigenschaften** wurden ebenfalls wie bei Beispiel 16 beschrieben gemessen, wobei die Aushärtungszeit der der Shore-Prüfkörper 28 Tage, der Filme 21 Tage im Normklima betrug.

Die **Geruchsbildung** wurde durch Riechen mit der Nase in einem Abstand von 10 cm an einem ausgehärteten Film qualitativ beurteilt.

Die Ergebnisse dieser Prüfungen sind in Tabelle 12 aufgeführt.

**Tabelle 12: Eigenschaften dereinkomponentigen PUR-Beschichtungsmassen.**

| Beispiel | **44** | **45** | **46 (Vgl)** |
|---|---|---|---|
| Viskosität vor Lagerung (mPa·s) | 380 | 640 | 220 |
| Viskosität nach Lagerung (mPa·s) | 780 | 1120 | 350 |
| Hautbildungszeit (min.) | 31 | 78 | 160 |
| Shore-Härte (A oder D) | 87A | 55D | 69A |
| Zugfestigkeit (MPa) | 15.9 | 23.0 | 8.8 |
| Bruchdehnung (%) | 350 | 260 | 310 |
| E-Modul (MPa) | 85 | 260 | 18 |
| Blasenbildung | keine | keine | einige |
| Geruchsbildung | keine | keine | keine |

### Beispiel 47:Elastische 2K-Beschichtungen (z.B. für Bodenbelag)

Zur Herstellung der Komponente *K1* wurden 64 Gewichtsteile (GT) Polyurethanpolymer 2, 32 GT einer Lösung von 45 Gew.-% IPDI-Trimerisat in Xylol (Vestanat® T 1890/100, Degussa; mit 17.3 Gew.-% NCO), 1 GT SäureKatalysator (5 Gew.-% Salicylsäure in Dioctyladipat ), 0.5 GT Aminkatalysator (2,2'-Dimorpholinodiethylether, DABCO® DMDEE Catalyst, Air Products), 1 GT Zinn-Katalysator (10 Gew.-% Dibutylzinn-dilaurat in Diisodecylphthalat) und 1.5 GT Entschäumer (BYK-088, BYK-Chemie/ALTANA) ohne vorherige Trocknung in einer Polypropylenkartusche eingewogen und mittels eines Zentrifugalmischers (SpeedMixer™ DAC 150, FlackTek Inc.; 30 sec. bei 2500 U/min) gemischt. Dazu wurden 19.5 GT des Aldimins ***A-5*** als Komponente *K2* zugegeben und eingemischt (30 sec. bei 2500 U/min). Das Verhältnis zwischen den Isocyanatgruppen der Komponente *K1* und der Summe der Reaktivgruppen (Hydroxyl- und Aldiminogruppen) der Komponente *K2* beträgt 1.1.

Das Polyurethanpolymer 2 wurde wie bei Beispiel 44 beschrieben hergestellt.

Die so erhaltene zweikomponentige Polyurethan-Beschichtungsmasse wurde auf Zeit bis zur Klebefreiheit, auf mechanische Eigenschaften nach der Aushärtung sowie auf Blasenbildung und Geruchsbildung geprüft.

Die **Zeit bis zur Klebefreiheit** wurde gleich wie die Hautbildungszeit bei Beispiel 16 gemessen. Die **mechanischen Eigenschaften** wurden ebenfalls wie bei Beispiel 16 beschrieben gemessen, wobei die Aushärtungszeit der der Shore-Prüfkörper 28 Tage, der Filme 14 Tage im Normklima betrug. Blasenbildung und Geruchsbildung wurden wie bei Beispiel 44 beschrieben gemessen.

Die Ergebnisse dieser Prüfungen sind in Tabelle 13 aufgeführt.

**Tabelle 13: Eigenschaften der zweikomponentigen PUR-Beschichtungsmasse.**

| Beispiel | **47** |
|---|---|
| Zeit bis zur Klebefreiheit (min.) | 135 |
| Shore A-Härte | 87 |
| Zugfestigkeit (MPa) | 9.5 |
| Bruchdehnung (%) | 370 |
| E-Modul (MPa) | 34 |
| Blasenbildung | keine |
| Geruchsbildung | keine |

### Beispiel 48:1K-Anstrich (geeignet als Lack oder Primer)

Eine Mischung aus 1.00 g Polyurethanpolymer *3*, dessen Herstellung nachfolgend beschrieben wird, 0.29 g Aldimin ***A-4**,* 0.10 g 3-Glycidoxypropyltrimethoxysilan und einer Spatelspitze Salicylsäure wurde mit Ethylacetat auf 50 Gew.-% Feststoffgehalt verdünnt.

Das Polyurethanpolymer 3 wurde wie folgt hergestellt:
1.00 g Polyoxypropylen-Diol (Desmophen® 1112 BD, Bayer; OH-Zahl 112 mg KOH/g), 4.06 g IPDI-Trimerisat (Vestanat® T 1890/100, Degussa) und 5.06 g Ethylacetat wurden nach bekanntem Verfahren bei 60 °C zu einem Polyurethanpolymeren mit einem titrimetrisch bestimmten Gehalt an freien Isocyanat-Gruppen von 5.7 Gewichts-% umgesetzt.

Ein kleiner Teil der erhaltenen Lösung wurde mittels eines Pinsels in dünner Schicht auf eine mit Heptan vorgereinigte Glasplatte (Floatglas; Firma Rocholl, Schönbrunn, Deutschland) aufgetragen und im Normklima stehen gelassen. Nach 30 Minuten war ein klebefreier, glänzend-transparenter und gut haftender Film mit vollständig trockener Oberfläche entstanden. Nach 3 Tagen wies der Film eine Bleistifthärte (Ritzhärte nach Wolff-Wilborn, gemessen nach ISO 15184) von 6H...7H auf. Die Haftung des Films auf dem Glas war ausgezeichnet (kein Absplittern beim Ritzen mit einem Bleistift der Härte 9H).

Ein weiterer Teil der Lösung wurde in der beschriebenen Weise auf eine weitere Glasplatte aufgetragen und im Normklima stehen gelassen. Nach 30 Minuten wurde auf den applizierten Film ein einkomponentiger Polyurethan-Klebstoff (SikaTack® Ultrafast, erhältlich von Sika Schweiz AG) in Form einer Dreiecksraupe aufgebracht und die Glasplatte während 7 Tagen im Normklima gelagert, wobei der Klebstoff aushärtete. Darauf wurde die Haftung der Klebstoffraupe auf dem Untergrund geprüft, indem die Raupe mittels eines Cutters an einem Ende bis knapp an den Untergrund eingeschnitten, das eingeschnittene Ende von Hand festgehalten und dann vorsichtig und langsam, schälend in Richtung des anderen Raupenendes, vom Untergrund abgezogen bzw. abgerissen wurde, wobei die Raupe regelmässig mit dem Cutter senkrecht zur Zugrichtung eingeschnitten wurde, wenn sie zu zerreissen drohte. Die Haftung der Raupe war ausgezeichnet; der Abriss erfolgte zu 100% im Klebstoff (Kohäsionsbruch).

Die übrig bleibende, nicht applizierte Lösung liess sich in einem klimadichten Gefäss während mehreren Wochen ohne signifikante Viskositätserhöhung lagern.

## Patentansprüche

1. Aldimin der Formel (I) 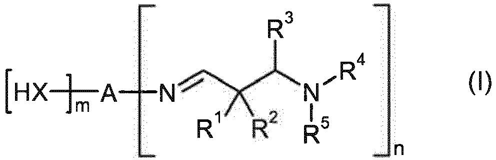 wobei
A entweder
für den Rest eines Amins nach Entfernung von n primären aliphatischen Aminogruppen und m HX-Gruppen steht,
oder
zusammen mit R⁷ für einen (n+2)-wertigen Kohlenwasserstoffrest mit 3 bis 20 C-Atomen, welcher gegebenenfalls mindestens ein Heteroatom enthält, steht;
n für 1 oder 2 oder 3 oder 4 steht und
m für 0 oder 1 oder 2 oder 3 oder 4 steht,
mit der Massgabe, dass m+n für 2 oder 3 oder 4 oder 5 steht;
R¹ und R² entweder
unabhängig voneinander jeweils für einen einwertigen Kohlenwasserstoffrest mit 1 bis 12 C-Atomen stehen,
oder
zusammen für einen zweiwertigen Kohlenwasserstoffrest mit 4 bis 12 C-Atomen, der Teil eines, gegebenenfalls substituierten, carbocyclischen Rings mit 5 bis 8 C-Atomen ist, stehen;
R³ für ein Wasserstoffatom oder eine Alkylgruppe oder eine Arylalkylgruppe oder eine Alkoxycarbonylgruppe steht;
R⁴ und R⁵ entweder
unabhängig voneinander jeweils für einen einwertigen aliphatischen, cycloaliphatischen oder arylaliphatischen Rest mit 1 bis 20 C-Atomen stehen, welcher frei ist von Hydroxylgruppen, und welcher gegebenenfalls Heteroatome in der Form von Ether-Sauerstoff oder tertiärem Amin-Stickstoff enthält,
oder
zusammen für einen zweiwertigen aliphatischen Rest mit 3 bis 20 C-Atomen, der Teil eines, gegebenenfalls substituierten, heterocyclischen Rings mit 5 bis 8 Ringatomen ist, wobei dieser Ring frei ist von Hydroxylgruppen und neben dem Stickstoffatom gegebenenfalls weitere Heteroatome in der Form von Ether-Sauerstoff oder tertiärem Amin-Stickstoff enthält, stehen;
X für O oder S oder N-R⁶ oder N-R⁷ steht,
wobei R⁶
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (II) steht, 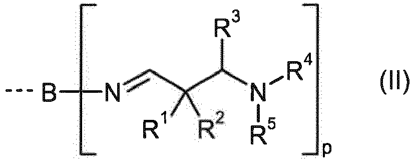
wobei
p für 0 oder für eine ganze Zahl von 1 bis 10'000 steht, und
B für einen (p+1)-wertigen Kohlenwasserstoffrest, welcher gegebenenfalls Ether-Sauerstoff, tertiären Amin-Stickstoff, Hydroxylgruppen, sekundäre Aminogruppen oder Mercaptogruppen enthält, steht; und
R⁷ zusammen mit A für einen (n+2)-wertigen Kohlenwasserstoffrest mit 3 bis 20 C-Atomen, welcher gegebenenfalls mindestens ein Heteroatom enthält, steht;
wobei das Aldimin der Formel (I) nicht für eine der folgenden Verbindungen steht: 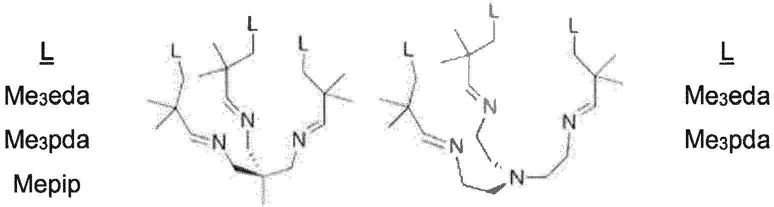 und L-H = 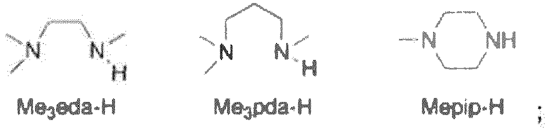 sowie

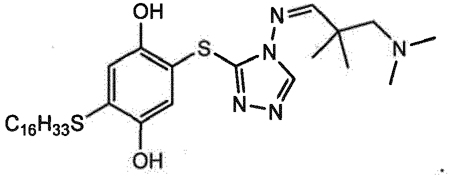

2. Aldimin der Formel (I) gemäss Anspruch 1, **dadurch gekennzeichnet, dass** dieses ein Aldimin der Formel (I a) darstellt, 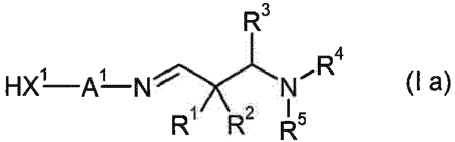 wobei
A¹ keinen aktiven Wasserstoff und keine primären Aminogruppen aufweist und
entweder
für einen zweiwertigen Kohlenwasserstoffrest mit 2 bis 20 C-Atomen, welcher gegebenenfalls mindestens ein Heteroatom enthält, steht,
oder
zusammen mit R⁹ für einen dreiwertigen Kohlenwasserstoffrest mit 3 bis 20 C-Atomen, welcher gegebenenfalls mindestens ein Heteroatom enthält, steht;
X¹ für O oder S oder N-R⁸ oder N-R⁹ steht,
wobei R⁸
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (II a) steht, 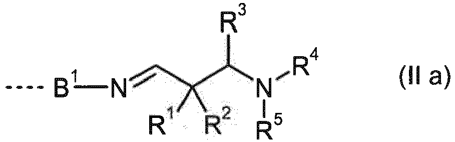
wobei B¹ für einen zweiwertigen, gegebenenfalls Ether-Sauerstoff oder tertiären Amin-Stickstoff aufweisenden, Kohlenwasserstoffrest mit 2 bis 12 C-Atomen steht; und
R⁹ zusammen mit A¹ für einen dreiwertigen Kohlenwasserstoffrest mit 3 bis 20 C-Atomen, welcher gegebenenfalls mindestens ein Heteroatom enthält, steht.

3. Aldimin der Formel (I) gemäss Anspruch 1, **dadurch gekennzeichnet, dass** dieses ein Aldimin der Formel (I b) darstellt, 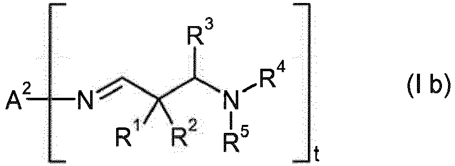 wobei,
m = 0 ist;
t für 2 oder 3 steht;
A² für den Rest eines Polyamins mit t primären Aminogruppen nach Entfernung von t primären primären Aminogruppen steht und keinen aktiven Wasserstoff enthält.

4. Aldimin der Formel (IX), 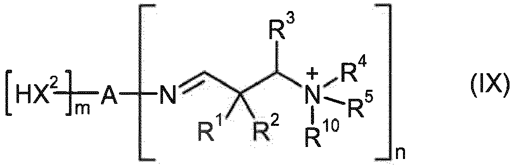 erhalten aus einem Aldimin der Formel (I) gemäss einem der Ansprüche 1 bis 3 durch Protonierung oder Alkylierung,
wobei R¹⁰ für ein Wasserstoffatom oder für einen Alkyl-, Cycloalkyl- oder Arylalkylrest mit 1 bis 20 C-Atomen steht;
X² für O oder S oder N-R¹¹ oder N-R⁷ steht; und
R¹¹
entweder für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder für einen Substituenten der Formel (IX') steht.

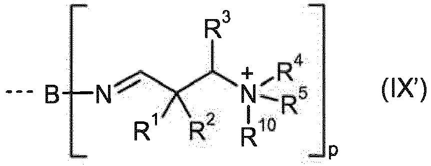

5. Verfahren zur Herstellung eines Aldimins der Formel (I), wie es in einem der Ansprüche 1 bis 3 beschrieben ist, **dadurch gekennzeichnet, dass** ein Aldehyd **ALD** der Formel (IV) mit einem Amin der Formel (III) in einer Kondensationsreaktion umgesetzt wird, 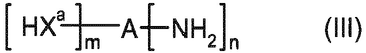 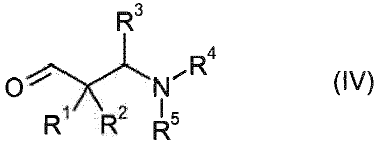
wobei X^{a} für O oder S oder N-R^{6a} oder N-R⁷steht,
wobei R^{6a} entweder
für einen einwertigen Kohlenwasserstoffrest mit 1 bis 20 C-Atomen, welcher gegebenenfalls mindestens eine Carbonsäureester-, Nitril-, Nitro-, Phosphonsäureester-, Sulfon- oder Sulfonsäureester-Gruppe aufweist, steht,
oder
für einen Substituenten der Formel (III') steht.
--- B ⁅NH₂]ₚ (III')

6. Aldiminogruppen-haltige Verbindung **AV** erhalten aus der Umsetzung von mindestens einem Aldimin der Formel (I) gemäss einem der Ansprüche 1 bis 3 mit m=1 mit mindestens einer Verbindung **D**, die mindestens eine Reaktivgruppe trägt, welche mit der Gruppe HX des Aldimins der Formel (I) Additionsreaktionen eingehen kann, **dadurch gekennzeichnet, dass** diese eine Aldiminogruppen-haltige Verbindung **AV1** der Formel (X) darstellt, 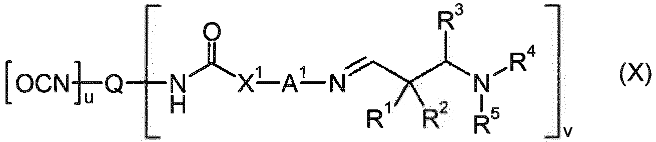 wobei
u für 0 oder 1 oder 2 oder 3 oder 4 oder 5 steht,
v für 1 oder 2 oder 3 oder 4 oder 5 oder 6 steht,
mit der Massgabe, dass (u+v) für 2 oder 3 oder 4 oder 5 oder 6 steht; und
Q für den Rest eines (u+v) Isocyanatgruppen aufweisenden Polyisocyanates nach Entfernung aller Isocyanatgruppen steht.

7. Aldiminogruppen-haltige Verbindung, erhalten aus einer Aldiminogruppen-haltigen Verbindung **AV** gemäss Anspruch 6 durch Protonierung oder Alkylierung, wobei die Aldiminogruppen-haltige Verbindung eine Aldiminogruppen-haltige Verbindung **AV2** der Formel (XII) darstellt, 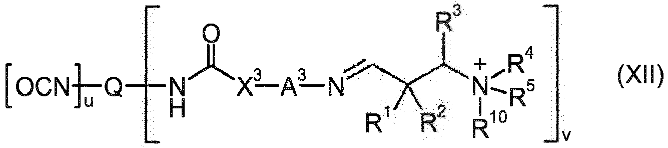 wobei
u für 0 oder 1 oder 2 oder 3 oder 4 oder 5 steht,
v für 1 oder 2 oder 3 oder 4 oder 5 oder 6 steht,
mit der Massgabe, dass (u+v) für 2 oder 3 oder 4 oder 5 oder 6 steht;
Q für den Rest eines (u+v) Isocyanatgruppen aufweisenden Polyisocyanates nach Entfernung aller Isocyanatgruppen steht;
A³ keinen aktiven Wasserstoff und keine primären Aminogruppen aufweist und
entweder
für einen zweiwertigen Kohlenwasserstoffrest mit 2 bis 20 C-Atomen, welcher gegebenenfalls mindestens ein Heteroatom enthält, steht,
oder
zusammen mit R¹² für einen dreiwertigen Kohlenwasserstoffrest mit 3 bis 20 C-Atomen, welcher gegebenenfalls mindestens ein Heteroatom enthält, steht; und
X³ für O oder S oder N-R¹¹ oder N-R¹² steht,
wobei R¹² zusammen mit A³ für einen dreiwertigen Kohlenwasserstoffrest mit 3 bis 20 C-Atomen, welcher gegebenenfalls mindestens ein Heteroatom enthält, steht.

8. Härtbare Zusammensetzung enthaltend mindestens ein Aldimin gemäss einem der Ansprüche 1 bis 3 oder eine Aldiminogruppen-haltige Verbindung **AV** gemäss Anspruch 6, sowie mindestens ein Polyisocyanat.

9. Härtbare Zusammensetzung gemäss Anspruch 8, **dadurch gekennzeichnet, dass** diese Zusammensetzung eine einkomponentige feuchtigkeitshärtende Zusammensetzung ist, und dass das Polyisocyanat ein aliphatische Isocyanatgruppen aufweisendes Polyisocyanat **P1** ist.

10. Härtbare Zusammensetzung gemäss Anspruch 8, **dadurch gekennzeichnet, dass** diese eine zweikomponentige Zusammensetzung bestehend aus einer Komponente **K1** und einer Komponente **K2** ist, und dass das Polyisocyanat ein Polyisocyanat **P2** ist.

11. Ausgehärtete Zusammensetzung erhalten durch die Reaktion einer härtbaren Zusammensetzung gemäss einem der Ansprüche 8 bis 10 und Wasser.

12. Verwendung eines Aldimins gemäss einem der Ansprüche 1 bis 4 oder einer Aldiminogruppen-haltigen Verbindung **AV** gemäss Anspruch 6 in Klebstoffen, Dichtstoffen, Vergussmassen, Beschichtungen, Bodenbelägen, Anstrichen, Lacken, Primern oder Schäumen.

13. Verwendung einer härtbaren Zusammensetzung gemäss Anspruch 8 bis 10 als ein- oder zweikomponentiger Klebstoff, Dichtstoff, Vergussmasse, Beschichtung, Bodenbelag, Anstrich, Lack, Primer oder Schaum.

14. Artikel, welcher unter Benutzung einer Verwendung gemäss Anspruch 13 erhalten wurde.

## Claims

1. Aldimine of the formula (I) 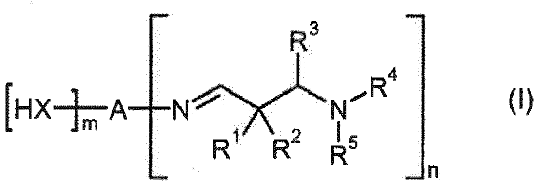 where
A is either
the radical of an amine after removal of n primary aliphatic amino groups and m HX groups,
or
together with R⁷ is an (n+2)-valent hydrocarbon radical which has 3 to 20 carbon atoms and optionally contains at least one heteroatom;
n is 1 or 2 or 3 or 4, and
m is 0 or 1 or 2 or 3 or 4,
with the proviso that m+n is 2 or 3 or 4 or 5;
R¹ and R² are either
each independently a monovalent hydrocarbon radical having 1 to 12 carbon atoms,
or
together are a divalent hydrocarbon radical having 4 to 12 carbon atoms which is part of an optionally substituted carbocyclic ring having 5 to 8 carbon atoms;
R³ is a hydrogen atom or an alkyl group or an arylalkyl group or an alkoxycarbonyl group;
R⁴ and R⁵ are either
each independently a monovalent aliphatic, cycloaliphatic or arylaliphatic radical which has 1 to 20 carbon atoms and is free of hydroxyl groups and optionally contains heteroatoms in the form of ether oxygen or tertiary amine nitrogen,
or
together are a divalent aliphatic radical which has 3 to 20 carbon atoms and is part of an optionally substituted heterocyclic ring having 5 to 8 ring atoms, this ring being free of hydroxyl groups and, as well as the nitrogen atom, optionally containing further heteroatoms in the form of ether oxygen or tertiary amine nitrogen;
X is O or S or N-R⁶ or N-R⁷,
where R⁶ is
either a monovalent hydrocarbon radical which has 1 to 20 carbon atoms and optionally has at least one carboxylic ester, nitrile, nitro, phosphonic ester, sulfone or sulfonic ester group,
or a substituent of the formula (II) 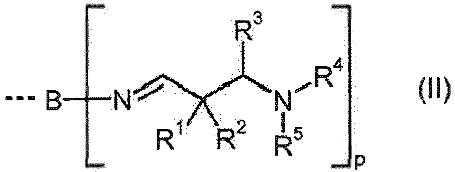
where
p is 0 or an integer from 1 to 10 000, and
B is a (p+1)-valent hydrocarbon radical which optionally contains ether oxygen, tertiary amine nitrogen, hydroxyl groups, secondary amino groups or mercapto groups; and
R⁷ together with A is an (n+2)-valent hydrocarbon radical which has 3 to 20 carbon atoms and optionally contains at least one heteroatom; wherein the aldimine of the formula (I) is nt one of the following compounds: 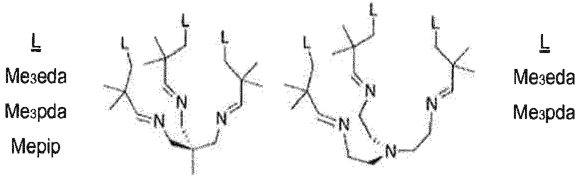 and L-H = 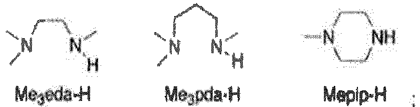 and

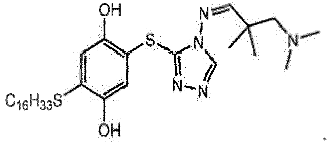

2. An aldimine of the formula (I) as claimed in claim 1, **characterized in that** it is an aldimine of the formula (I a) 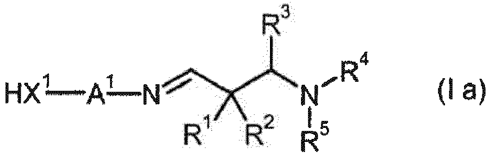 where
A¹ does not have any active hydrogen or any primary amino groups and
is either
a divalent hydrocarbon radical which has 2 to 20 carbon atoms and optionally contains at least one heteroatom,
or
together with R⁹ is a trivalent hydrocarbon radical which has 3 to 20 carbon atoms and optionally contains at least one heteroatom;
X¹ is O or S or N-R⁸ or N-R⁹,
where R⁸ is
either a monovalent hydrocarbon radical which has 1 to 20 carbon atoms and optionally has at least one carboxylic ester, nitrile, nitro, phosphonic ester, sulfone or sulfonic ester group,
or a substituent of the formula (II a) 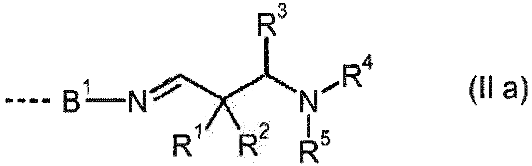
where B¹ is a divalent hydrocarbon radical which has 2 to 12 carbon atoms and optionally has ether oxygen or tertiary amine nitrogen; and
R⁹ together with A¹ is a trivalent hydrocarbon radical which has 3 to 20 carbon atoms and optionally contains at least one heteroatom.

3. An aldimine of the formula (I) as claimed in claim 1, **characterized in that** it is an aldimine of the formula (I b) 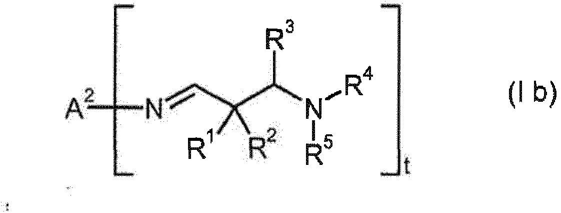 Where
m = 0;
t is 2 or 3;
A² is the radical of a polyamine with t primary amino groups after removal of t primary amino groups and does not contain any active hydrogen.

4. An aldimine of the formula (IX) 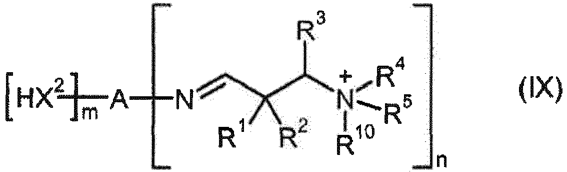
obtained from an aldimine of the formula (I) as claimed in any of claims 1 to 3 by protonation or alkylation,
where R¹⁰ is a hydrogen atom or an alkyl, cycloalkyl or arylalkyl radical having 1 to 20 carbon atoms;
X² is O or S or N-R¹¹ or N-R⁷; and
R¹¹ is
either a monovalent hydrocarbon radical which has 1 to 20 carbon atoms and optionally has at least one carboxylic ester, nitrile, nitro, phosphonic ester, sulfone or sulfonic ester group,
or a substituent of the formula (IX')

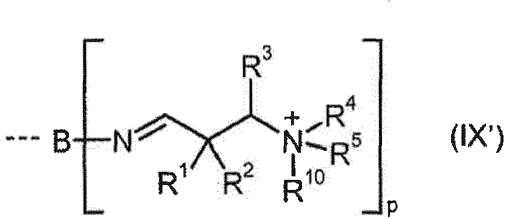

5. A process for preparing an aldimine of the formula (I) as described in any of claims 1 to 3, **characterized in that** an aldehyde **ALD** of the formula (IV) is reacted with an amine of the formula (III) in a condensation reaction 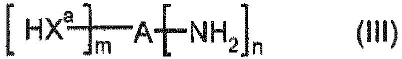 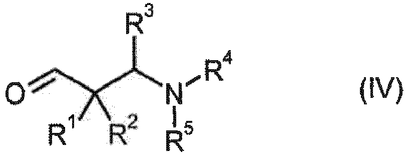
where X^{a} is O or S or N-R^{6a} or N-R⁷,
where R^{6a} is either
a monovalent hydrocarbon radical which has 1 to 20 carbon atoms and optionally has at least one carboxylic ester, nitrile, nitro, phosphonic ester, sulfone or sulfonic ester group,
or
a substituent of the formula (III')
---B⁅NH₂]ₚ (III')

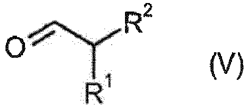

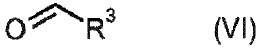

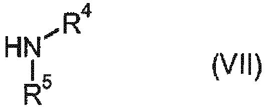

6. An aldimino group-containing compound **AV** obtained from the reaction of at least one aldimine of the formula (I) according to any of claims 1 to 3 where m=1, with at least one compound **D** which bears at least one reactive group which can enter into addition reactions with the HX group of the aldimine of the formula (I), **characterized in that** it is an aldimino group-containing compound **AV1** of the formula (X) 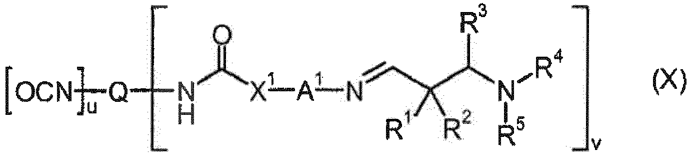 where
u is 0 or 1 or 2 or 3 or 4 or 5,
v is 1 or 2 or 3 or 4 or 5 or 6,
with the proviso that (u+v) is 2 or 3 or 4 or 5 or 6; and
Q is the radical of a polyisocyanate having (u+v) isocyanate groups after removal of all isocyanate groups.

7. An aldimino group-containing compound obtained from an aldimino group-containing compound **AV** as claimed in claim 6 by protonation or alkylation, wherein the aldimino group-containing compound is an aldimino-containing compound **AV2** of the formula (XII) 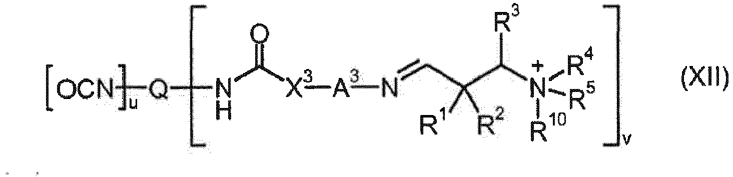 where
u is 0 or 1 or 2 or 3 or 4 or 5,
v is 1 or 2 or 3 or 4 or 5 or 6,
with the proviso that (u+v) is 2 or 3 or 4 or 5 or 6;
Q is the radical of a polyisocyanate having (u+v) isocyanate groups after removal of all isocyanate groups;
A³ does not have any active hydrogen or any primary amino groups and
is either
a divalent hydrocarbon radical which has 2 to 20 carbon atoms and optionally contains at least one heteroatom,
or
together with R¹² is a trivalent hydrocarbon radical which has 3 to 20 carbon atoms and optionally contains at least one heteroatom; and
X³ is O or S or N-R¹¹ or N-R¹²,
where R¹² together with A³ is a trivalent hydrocarbon radical which has 3 to 20 carbon atoms and optionally contains at least one heteroatom.

8. A curable composition comprising at least one aldimine as claimed in any of claims 1 to 3 or an aldimino group-containing compound **AV** as claimed in claim 6, and at least one polyisocyanate.

9. The curable composition as claimed in claim 8, **characterized in that** this composition is a one-component moisture-curing composition, and **in that** the polyisocyanate is a polyisocyanate **P1** having aliphatic isocyanate groups.

10. The curable composition as claimed in claim 8, **characterized in that** it is a two-component composition consisting of a component **K1** and a component **K2**, and **in that** the polyisocyanate is a polyisocyanate **P2**.

11. A cured composition obtained by the reaction of a curable composition as claimed in any of claims 8 to 10 and water.

12. The use of an aldimine as claimed in any of claims 1 to 4 or of an aldimino group-containing compound **AV** as claimed in claim 6 in adhesives, sealants, potting compositions, coatings, floor coverings, paints, coating materials, primers or foams.

13. The use of a curable composition as claimed in claim 8 to 10 as a one- or two-component adhesive, sealant, potting composition, coating, floor covering, paint, coating material, primer or foam.

14. An article obtained utilizing a use as claimed in claim 13.

## Revendications

1. Aldimine de formule (I) 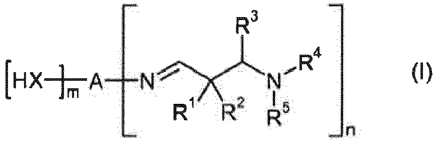 dans laquelle
A soit
représente le radical d'une amine après élimination de n groupes amino aliphatiques primaires et m groupes HX,
soit
représente conjointement avec R⁷ un radical hydrocarboné (n+2)-valent de 3 à 20 atomes C, qui contient éventuellement au moins un hétéroatome ;
n représente 1 ou 2 ou 3 ou 4, et
m représente 0 ou 1 ou 2 ou 3 ou 4,
à condition que m+n représente 2 ou 3 ou 4 ou 5,
R¹ et R² soit
représentent chacun indépendamment l'un de l'autre un radical hydrocarboné monovalent de 1 à 12 atomes C, soit
représentent ensemble un radical hydrocarboné bivalent de 4 à 12 atomes C, qui fait partie d'un cycle carbocyclique de 5 à 8 atomes C, éventuellement substitué ;
R³ représente un atome d'hydrogène ou un groupe alkyle ou un groupe arylalkyle ou un groupe alcoxycarbonyle ;
R⁴ et R⁵ soit
représentent chacun indépendamment l'un de l'autre un radical aliphatique, cycloaliphatique ou arylaliphatique monovalent de 1 à 20 atomes C, qui est exempt de groupes hydroxyle, et qui contient éventuellement des hétéroatomes sous la forme d'un oxygène d'éther ou d'un azote d'amine tertiaire,
soit
représentent ensemble un radical aliphatique bivalent de 3 à 20 atomes C, qui fait partie d'un cycle hétérocyclique éventuellement substitué de 5 à 8 atomes de cycle, ce cycle étant exempt de groupes hydroxyle et contenant éventuellement, outre l'atome d'azote, des hétéroatomes supplémentaires sous la forme d'un oxygène d'éther ou d'un azote d'amine tertiaire ;
X représente O ou S ou N-R⁶ ou N-R⁷,
R6
soit représentant un radical hydrocarboné monovalent de 1 à 20 atomes C, qui comprend éventuellement au moins un groupe ester d'acide carboxylique, nitrile, nitro, ester d'acide phosphonique, acide sulfonique ou ester d'acide sulfonique,
soit représentant un substituant de formule (II) : 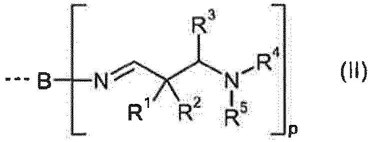
dans laquelle
p représente 0 ou un nombre entier de 1 à 10 000, et
B représente un radical hydrocarboné (p+1)-valent, qui contient éventuellement un oxygène d'éther, un azote d'amine tertiaire, des groupes hydroxyle, des groupes amino secondaires ou des groupes mercapto ; et
R⁷ représente conjointement avec A un radical hydrocarboné (n+2)-valent de 3 à 20 atomes C, qui contient éventuellement au moins un hétéroatome ;
l'aldimine de formule (I) n'étant pas un des composés suivants : 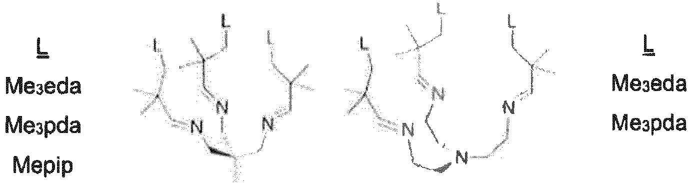 et L-H = 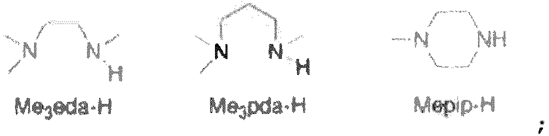 et

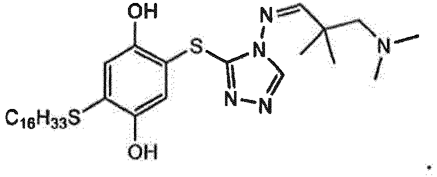

2. Aldimine de formule (I) selon la revendication 1, **caractérisée en ce que** celle-ci est une aldimine de formule (Ia) : 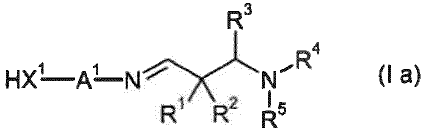 dans laquelle
A¹ ne comprend pas d'hydrogène actif et pas de groupes amino primaires, et
soit
représente un radical hydrocarboné bivalent de 2 à 20 atomes C, qui contient éventuellement au moins un hétéroatome,
soit
représente conjointement avec R⁹ un radical hydrocarboné trivalent de 3 à 20 atomes C, qui contient éventuellement au moins un hétéroatome ;
X¹ représente O ou S ou N-R⁸ ou N-R⁹,
R⁸
soit représentant un radical hydrocarboné monovalent de 1 à 20 atomes C, qui comprend éventuellement au moins un groupe ester d'acide carboxylique, nitrile, nitro, ester d'acide phosphonique, acide sulfonique ou ester d'acide sulfonique,
soit représentant un substituant de formule (IIa) : 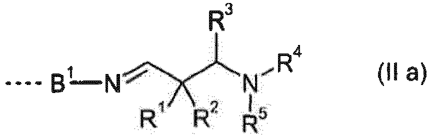
dans laquelle B¹ représente un radical hydrocarboné bivalent de 2 à 12 atomes C, comprenant éventuellement un oxygène d'éther ou un azote d'amine tertiaire ; et
R⁹ représente conjointement avec A¹ un radical hydrocarboné trivalent de 3 à 20 atomes C, qui contient éventuellement au moins un hétéroatome.

3. Aldimine de formule (I) selon la revendication 1, **caractérisée en ce que** celle-ci est une aldimine de formule (Ib) : 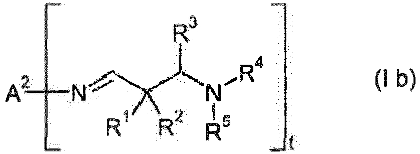 dans laquelle
m = 0 ;
t représente 2 ou 3 ;
A² représente le radical d'une polyamine contenant t groupes amino primaires après élimination de t groupes amino primaires, et ne contient pas d'hydrogène actif.

4. Aldimine de formule (IX) 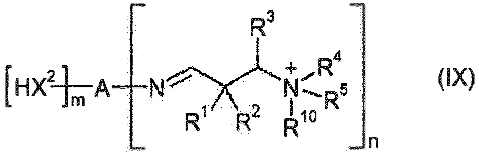
obtenue à partir d'une aldimine de formule (I) selon l'une quelconque des revendications 1 à 3 par protonation ou alkylation,
dans laquelle R¹⁰ représente un atome d'hydrogène ou un radical alkyle, cycloalkyle ou arylalkyle de 1 à 20 atomes C ;
X² représente O ou S ou N-R¹¹ ou N-R⁷ ; et
R¹¹
soit représente un radical hydrocarboné monovalent de 1 à 20 atomes C, qui comprend éventuellement au moins un groupe ester d'acide carboxylique, nitrile, nitro, ester d'acide phosphonique, acide sulfonique ou ester d'acide sulfonique,
soit représente un substituant de formule (IX')

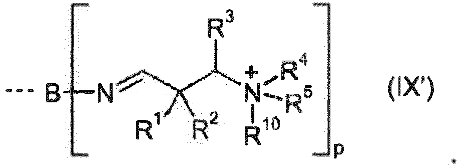

5. Procédé de fabrication d'une aldimine de formule (I), telle que décrite dans l'une quelconque des revendications 1 à 3, **caractérisé en ce qu'**un aldéhyde **ALD** de formule (IV) est mis en réaction avec une amine de formule (III) dans une réaction de condensation 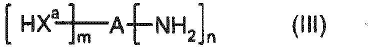 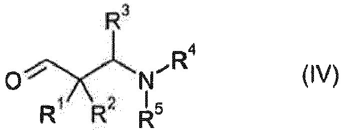 dans lesquelles X^{a} représente O ou S ou N-R^{6a} ou N-R⁷,
R^{6a} soit
représentant un radical hydrocarboné monovalent de 1 à 20 atomes C, qui comprend éventuellement au moins un groupe ester d'acide carboxylique, nitrile, nitro, ester d'acide phosphonique, acide sulfonique ou ester d'acide sulfonique,
soit
représentant un substituant de formule (III')
---B⁅NH₂]ₚ (III').

6. Composé contenant des groupes aldimino **AV** obtenu par la réaction d'au moins une aldimine de formule (I) selon l'une quelconque des revendications 1 à 3 avec m = 1 avec au moins un composé **D**, qui porte au moins un groupe réactif, qui peut réaliser des réactions d'addition avec le groupe HX de l'aldimine de formule (I), **caractérisé en ce que** celui-ci est un composé contenant des groupes aldimino **AV1** de formule (X) : 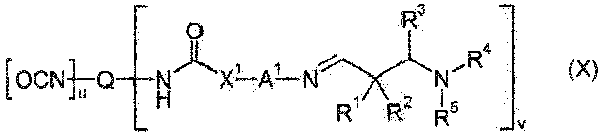 dans laquelle
u représente 0 ou 1 ou 2 ou 3 ou 4 ou 5,
v représente 1 ou 2 ou 3 ou 4 ou 5 ou 6, à condition que (u+v) représente 2 ou 3 ou 4 ou 5 ou 6 ; et
Q représente le radical d'un polyisocyanate comprenant (u+v) groupes isocyanate après élimination de tous les groupes isocyanate.

7. Composé contenant des groupes aldimino, obtenu à partir d'un composé contenant des groupes aldimino **AV** selon la revendication 6 par protonation ou alkylation, le composé contenant des groupes aldimino étant un composé contenant des groupes aldimino **AV2** de formule (XII) 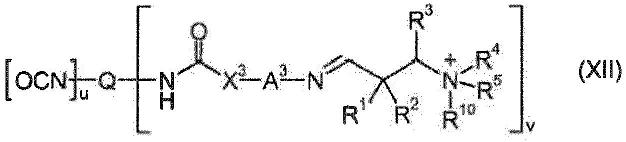 dans laquelle
u représente 0 ou 1 ou 2 ou 3 ou 4 ou 5,
v représente 1 ou 2 ou 3 ou 4 ou 5 ou 6,
à condition que (u+v) représente 2 ou 3 ou 4 ou 5 ou 6 ;
Q représente le radical d'un polyisocyanate comprenant (u+v) groupes isocyanate après élimination de tous les groupes isocyanate ;
A³ ne comprend pas d'hydrogène actif et pas de groupes amino primaires, et
soit
représente un radical hydrocarboné bivalent de 2 à 20 atomes C, qui contient éventuellement au moins un hétéroatome,
soit
représente conjointement avec R¹² un radical hydrocarboné trivalent de 3 à 20 atomes C, qui contient éventuellement au moins un hétéroatome ; et
X³ représente O ou S ou N-R¹¹ ou N-R¹²,
R¹² représentant conjointement avec A³ un radical hydrocarboné trivalent de 3 à 20 atomes C, qui contient éventuellement au moins un hétéroatome.

8. Composition durcissable contenant au moins une aldimine selon l'une quelconque des revendications 1 à 3 ou un composé contenant des groupes aldimino **AV** selon la revendication 6, ainsi qu'au moins un polyisocyanate.

9. Composition durcissable selon la revendication 8, **caractérisée en ce que** cette composition est une composition monocomposante durcissable à l'humidité, et **en ce que** le polyisocyanate est un polyisocyanate comprenant des groupes isocyanate aliphatiques **P1**.

10. Composition durcissable selon la revendication 8, **caractérisée en ce que** celle-ci est une composition bicomposante constituée par un composant **K1** et un composant **K2**, et **en ce que** le polyisocyanate est un polyisocyanate **P2**.

11. Composition durcie obtenue par la réaction d'une composition durcissable selon l'une quelconque des revendications 8 à 10 et d'eau.

12. Utilisation d'une aldimine selon l'une quelconque des revendications 1 à 4 ou d'un composé contenant des groupes aldimino **AV** selon la revendication 6 dans des adhésifs, des agents d'étanchéité, des masses de scellement, des revêtements, des revêtements de sol, des peintures, des vernis, des agents primaires ou des mousses.

13. Utilisation d'une composition durcissable selon les revendications 8 à 10 en tant qu'adhésif, agent d'étanchéité, masse de scellement, revêtement, revêtement de sol, peinture, vernis, agent primaire ou mousse mono- ou bicomposant.

14. Article, qui a été obtenu par une utilisation selon la revendication 13.
